# EUROPEAN PATENT SPECIFICATION

(11) **EP 2 691 109 B1**
(45) Date of publication and mention of the grant of the patent: **15.07.2015**
(21) Application number: 12712106.9
(22) Date of filing: 29.03.2012
(51) Int. Cl.: A61K 38/26, A61K 31/64, A61P 3/08

(54) **PREVENTION OF HYPOGLYCAEMIA IN DIABETES MELLITUS TYPE 2 PATIENTS**
VORBEUGUNG VON HYPOGLYKÄMIE BEI PATIENTEN MIT DIABETES MELLITUS TYP 2
PRÉVENTION DE L'HYPOGLYCÉMIE CHEZ DES PATIENTS ATTEINTS DU DIABÈTE SUCRÉ DE TYPE 2

(30) Priority: 29.03.2011 EP 11160270
(43) Date of publication of application: 05.02.2014
(73) Proprietor: Sanofi-Aventis Deutschland GmbH, 65929 Frankfurt am Main (DE)
(72) Inventor: BOKA, Gabor, F-75008 Paris (FR); SILVESTRE, Louise, F-75008 Paris (FR); MIOSSEC, Patrick, F-75008 Paris (FR)
(74) Representative: Weickmann & Weickmann
(86) International application number: PCT/EP2012/055660
(87) International publication number: WO 2012/130955

(56) References cited:
- CHRISTENSEN M ET AL: "Lixisenatide, a novel GLP-1 receptor agonist for the treatment of type 2 diabetes mellitus", I DRUGS: THE INVESTIGATIONAL DRUGS JOURNAL, CURRENT DRUGS LTD, GB, vol. 12, no. 8, 1 August 2009 (2009-08-01) , pages 503-513, XP002572656, ISSN: 2040-3410
- CAMPAS C ET AL: "AVE-0010 GLP-1 Receptor Agonist Treatment of Diabetes", DRUGS OF THE FUTURE, PROUS SCIENCE, ES, vol. 33, no. 10, 1 October 2008 (2008-10-01), pages 838-840, XP002572655, ISSN: 0377-8282, DOI: 10.1358/DOF.2008.33.10.1265206
- KENDALL DAVID M ET AL: "Effects of exenatide (exendin-4) on glycemic control over 30 weeks in patients with type 2 diabetes treated with metformin and a sulfonylurea.", DIABETES CARE MAY 2005 LNKD- PUBMED:15855571, vol. 28, no. 5, May 2005 (2005-05), pages 1083-1091, XP002658236, ISSN: 0149-5992
- RATNER R E ET AL: "Dose-dependent effects of the once-daily GLP-1 receptor agonist lixisenatide in patients with Type 2 diabetes inadequately controlled with metformin: A randomized, double-blind, placebo-controlled trial", DIABETIC MEDICINE, JOHN WILEY & SONS, LTD, GB, vol. 27, no. 9, 1 September 2010 (2010-09-01), pages 1024-1032, XP002626157, ISSN: 0742-3071, DOI: 10.1111/J.1464-5491.2010.03020.X [retrieved on 2010-04-30]
- Anonymous: "Lixisenatide Significantly Reduces HbA1c Without Increasing Hypoglycemia in Patients Uncontrolled on Sulfonylureas", , 12 April 2011 (2011-04-12), pages 1-2, XP055006128, Retrieved from the Internet: URL:http://en.sanofi.com/binaries/20110412 _LIXI_GETGOAL_S_EN_tcm28-31652.pdf [retrieved on 2011-09-02]
- Anonymous: "Additional positive results from global Phase III program with lixisenatide for Type 2 Diabetes", , 12 April 2011 (2011-04-12), pages 1-2, XP055006131, Retrieved from the Internet: URL:http://files.shareholder.com/downloads /ABEA-58QR0J/0x0x458202/3ccd84a6-5f99-451a -ada0-0a8282da3dad/ZEAL_News_2011_4_12_Com pany_Releases.pdf [retrieved on 2011-09-02]

## Description

Subject of the present invention is a pharmaceutical combination for the use in the prevention of hypoglycaemia in diabetes mellitus type 2, said combination comprising (a) lixisenatide (desPro³⁶Exendin-4(1-39)-Lys₆-NH₂, AVE0010) and (b) a sulfonyl urea.

Metformin is a biguanide hypoglycemic agent used in the treatment of Type 2 diabetes mellitus not responding to dietary modification. Metformin improves glycemic control by improving insulin sensitivity. Metformin is usually administered orally. However, control diabetes mellitus type 2 in obese patients by metformin may be insufficient. Thus, in these patients, additional measures for controlling diabetes mellitus type 2 may be required.

Hypoglycaemia is the critical limiting factor in the glycaemic management of diabetes in both the short and long term. Despite steady improvements in the glycaemic management of diabetes, population-based data indicate that hypoglycaemia continues to be a major problem for people with both type 1 and type 2 diabetes (American diabetes association, workgroup on hypoglycemia: Defining and Reporting Hypoglycemia in Diabetes. Diabetes Care 28(5), 2005, 1245-1249).

Kendall (Diabetes Care, 2005, 28(5):1083-1091) describes in a 30 week, double-blind, placebo-controlled study the effects of exendin-4 on glycemic control in patients with type 2 diabetes treated with metformin and a sulfonylurea. Exendin-4 significantly reduced HbA1c in patients with type 2 diabetes unable to achieve adequate glycemic control with maximally effective doses of combined metformin-sulfonylurea therapy.

Campas (Drugs of the Future, 2008, 33(10):838-840) and Christensen (Idrugs 2009, 12(8):503-513) review preclinical and clinical studies of desPro³⁶Exendin-4(1-39)-Lys₆-NH₂, AVE0010, lixisenatide).

Ratner (Diabet. Med. 2010, 27:1024-1032) discloses dose-dependent effects of once-daily and twice daily lixisenatide in patients with type 2 diabetes inadequately controlled with metformin in a randomized, double-blind, placebo-controlled, parallel-group, 13 weeks study.

A first aspect of the present invention is a pharmaceutical combination for use in the prevention of hypoglycaemia in diabetes mellitus type 2 patients, comprising
(a) desPro³⁶Exendin-4(1-39)-Lys₆-NH₂ or/and a pharmaceutically acceptable salt thereof, and
(b) a sulfonyl urea or/and a pharmaceutically acceptable salt thereof.

The skilled person knows suitable pharmaceutically acceptable salts of sulfonyl ureas.

In particular, the pharmaceutical combination is for use in the prevention of symptomatic hypoglycaemia or severe symptomatic hypoglycaemia in a diabetes mellitus type 2 patient.

More particular, the pharmaceutical combination of the present invention is for use in the prevention of hypoglycaemia in a diabetes type 2 patient having an increased risk of hypoglycaemia, in particular a diabetes type 2 patient having experienced at least one hypoglycaemic event. The hypoglycaemic event can be a symptomatic hypoglycaemic event or a severe symptomatic hypoglycaemic event.

In the present invention, hypoglycaemia is a condition wherein a diabetes mellitus type 2 patient experiences a plasma glucose concentration of below 60 mg/dL (or below 3.3 mmol/L), below 50 mg/dL, below 40 mg/dL, or below 36 mg/dL.

In the present invention, "symptomatic hypoglycaemia" or "symptomatic hypoglycaemic event" is a condition associated with a clinical symptom that results from the hypoglycaemia, wherein the plasma glucose concentration is below 60 mg/dL (or below 3.3 mmol/L), below 50 mg/dL, or below 40 mg/dL.

A clinical symptoms can be, for example, sweating, palpitations, hunger, restlessness, anxiety, fatigue, irritability, headache, loss of concentration, somnolence, psychiatric disorders, visual disorders, transient sensory defects, transient motor defects, confusion, convulsions, and coma. In the medical uses of the present invention, one or more clinical symptoms of symptomatic hypoglycaemia, as indicated herein, can be selected. Symptomatic hypoglycaemia may be associated with prompt recovery after oral carbohydrate administration.

In the present invention, "severe symptomatic hypoglycaemia" or "severe symptomatic hypoglycaemic event" is a condition with a clinical symptom, as indicated herein, that results from hypoglycaemia, wherein the plasma glucose concentration is below 36 mg/dL (or below 2.0 mmol/L). Severe symptomatic hypoglycaemia can be associated with acute neurological impairment resulting from the hypoglycaemic event. In a severe symptomatic hypoglycaemia, the patient may require the assistance of another person, if, for example, the patient could not treat or help him/herself due to the acute neurological impairment. The definition of severe symptomatic hypoglycaemia may include all episodes in which neurological impairment is severe enough to prevent self-treatment and which were thus thought to place patients at risk for injury to themselves or others. The acute neurological impairment may be at least one selected from somnolence, psychiatric disorders, visual disorders, transient sensory defects, transient motor defects, confusion, convulsions, and coma.

Severe symptomatic hypoglycaemia may be associated with prompt recovery after oral carbohydrate, intravenous glucose, or/and glucagon administration.

Normoglycaemia may relate to a blood plasma concentration of glucose of from 60 mg/dL to 140 mg/dL (corresponding to 3.3 mmol/L to 7.8 mmol/L).

It has surprisingly been found in a clinical trial that during treatment of diabetes mellitus type 2 patients with lixisenatide combined with a sulfonyl urea with or without metformin, the number of hypoglycaemic events in individual patients could be reduced. One hundred twenty seven (22.1%) patients treated with lixisenatide in combination with a sulfonyl urea with or without metformin had 389 symptomatic hypoglycemia events per protocol definition during the on-treatment period for the whole study, whereas 51 (17.9%) placebo-treated patients (i.e. treated with a sulfonyl urea with or without metformin) reported 230 symptomatic hypoglycemia events during the same period (Table 24), indicating that the number of hypoglycemia events is reduced in the lixisenatide-treated patients (on average 3.06 events in those patients reporting hypoglycaemic events) compared with the placebo-treated patients (on average 4.51 events in those patients reporting hypoglycaemic events).

Two (0.3%) patients treated with lixisenatide in combination with a sulfonyl urea with or without metformin had severe symptomatic hypoglycemia events during the on-treatment period for the whole study, whereas 1 (0.4%) placebo-treated patient (i.e. treated with a sulfonyl urea with or without metformin) reported a severe symptomatic hypoglycemia during the same period (Table 25).

These results indicate that the combination of lixisenatide and a sulfonyl urea with or without metformin can be used for the prevention of hypoglycaemia.

The compounds of (a) and (b) may be administered to a subject in need thereof, in an amount sufficient to induce a therapeutic effect.

The compound desPro³⁶Exendin-4(1-39)-Lys₆-NH₂ (AVE0010, lixisenatide) is a derivative of Exendin-4. AVE0010 is disclosed as SEQ ID NO:93 in WO 01/04156:
SEQ ID NO: 1 AVE0010 (44 AS)
SEQ ID NO: 2 Exendin-4 (39 AS)

Exendins are a group of peptides which can lower blood glucose concentration. The Exendin analogue AVE0010 is characterised by C-terminal truncation of the native Exendin-4 sequence. AVE0010 comprises six C-terminal lysine residues not present in Exendin-4.

In the context of the present invention, AVE0010 includes pharmaceutically acceptable salts thereof. The person skilled in the art knows pharmaceutically acceptable salts of AVE0010. A preferred pharmaceutically acceptable salt of AVE0010 employed in the present invention is acetate.

AVE0010 (desPro³⁶Exendin-4(1-39)-Lys₆-NH₂) or/and a pharmaceutically acceptable salt thereof may be administered by subcutaneous injection. Suitable injection devices, for instance the so-called "pens" comprising a cartridge comprising the active ingredient, and an injection needle, are known. AVE0010 or/and a pharmaceutically acceptable salt thereof may be administered in a suitable amount, for instance in an amount in the range of 10 to 15 µg per dose or 15 to 20 µg per dose once a day (progressive titration from 10 to 15 and to 20 µg/day. 20 µg is the effective maintenance dose).

In the present invention, AVE0010 or/and a pharmaceutically acceptable salt thereof may be administered in a daily dose in the range of 10 to 15 µg or in the range of 15 to 20 µg once a day (progressive titration from 10 to 15 and to 20 µg/day. 20 µg is the effective maintenance dose). AVE0010 or/and a pharmaceutically acceptable salt thereof may be administered by one injection per day.

In the present invention, a liquid composition comprising desPro³⁶Exendin-4(1-39)-Lys₆-NH₂ or/and a pharmaceutically acceptable salt thereof may be employed. The skilled person knows liquid compositions of AVE0010 suitable for parenteral administration. A liquid composition of the present invention may have an acidic or a physiologic pH. An acidic pH preferably is in the range of pH 1 - 6.8, pH 3.5 - 6.8, or pH 3.5 - 5. A physiologic pH preferably is in the range of pH 2.5 -8.5, pH 4.0 to 8.5, or pH 6.0 to 8.5. The pH may be adjusted by a pharmaceutically acceptable diluted acid (typically HCl) or pharmaceutically acceptable diluted base (typically NaOH). The preferred pH is in the range of pH 3,5 to 5,0.

The liquid composition may contain a buffer, such as a phosphate, a citrate, an acetate. Preferably, it can contain an acetate buffer, in quantities up to 5 µg/mL, up to 4 µg/mL or up to 2 µg/mL.

The liquid composition of the present invention may comprise a suitable preservative. A suitable preservative may be selected from phenol, m-cresol, benzyl alcohol and p-hydroxybenzoic acid ester. A preferred preservative is m-cresol.

The liquid composition of the present invention may comprise a tonicity agent. A suitable tonicity agent may be selected from glycerol, lactose, sorbitol, mannitol, glucose, NaCl, calcium or magnesium containing compounds such as CaCl₂. The concentration of glycerol, lactose, sorbitol, mannitol and glucose may be in the range of 100 - 250 mM. The concentration of NaCl may be up to 150 mM. A preferred tonicity agent is glycerol.

In addition, the liquid composition may contain L-methionin from 0,5 µg/mL to 20 µg/mL, preferably from 1 µg/mL to 5 µg/mL. Preferably, it contains L-methionin.

In the present invention, the sulfonyl urea may be administered orally. The skilled person knows formulations of a sulfonyl urea suitable for treatment of diabetes type 2 by oral administration. For oral administration, the sulfonyl urea may be formulated in a solid dosage form, such as a tablet or pill.

In the present invention, desPro³⁶Exendin-4(1-39)-Lys₆-NH₂ or/and a pharmaceutically acceptable salt can be administered in an add-on therapy to administration of a sulfonyl urea.

In the present invention, the terms "add-on", "add-on treatment" and "add-on therapy" include a treatment of diabetes mellitus type 2 with a sulfonyl urea and AVE0010. The sulfonyl urea and AVE0010 may be administered within a time interval of 24 h. The sulfonyl urea and AVE0010 each may be administered in a once-a-day-dosage. The sulfonyl urea and AVE0010 may be administered by different administration routes. The sulfonyl urea may be administered orally, and AVE0010 may be administered subcutaneously.

In the present invention, the sulfonyl urea can be selected from Glibenclamide, Glibenclamide MR, Gliclazide, Gliclazide LM, Glimepiride, Glipizide, Glipizide XL, Gliquidone, and Tolbutamide.

In the present invention, the sulfonyl urea may be Glibenclamide, Glibenclamide MR, Gliclazide, Gliclazide LM, Glimepiride, Glipizide, Glipizide XL, Gliquidone, or Tolbutamide.

A preferred dose of Glibenclamide is ≤10 mg/day, 10 - 20 mg/day, or ≥20 mg/day.

A preferred dose of Glibenclamide MR is ≤6 mg/day, 6 - 12 mg/day, or ≥12 mg/day.

A preferred dose of Gliclazide is ≤160 mg/day, 160 - 320 mg/day, or ≥320 mg/day.

A preferred dose of Gliclazide LM is ≤60 mg/day, 60 - 120 mg/day, or ≥120 mg/day.

A preferred dose of Glimepiride is ≤4 mg/day, 4 - 8 mg/day, or ≥8 mg/day.

A preferred dose of Glipizide is ≤20 mg/day, 20 - 40 mg/day, or ≥40 mg/day.

A preferred dose of Glipizide XL is ≤10 mg/day, 10 - 20 mg/day, or ≥20 mg/day.

A preferred dose of Gliquidone is ≤ 60 mg/day, 60 - 90 mg/day, or ≥ 90 mg/day.

A preferred dose of Tolbutamide is ≤ 1500 mg/day, or 2t 1500 mg/day.

The pharmaceutical combination for use of the present invention preferably is for the treatment of a subject suffering from diabetes type 2, wherein diabetes type 2 is not adequately controlled by treatment with sulfonyl urea alone, for instance by treatment for at least 3 months.

For example, a treatment with Glibenclamide alone with a dose of ≤ 10 mg/day, 10 - 20 mg/day, or ≥ 20 mg/day may be insufficient for adequate control of diabetes type 2.

For example, a treatment with Glibenclamide MR alone with a dose of ≤ 6 mg/day, 6 - 12 mg/day, or ≥ 12 mg/day may be insufficient for adequate control of diabetes type 2.

For example, a treatment with Gliclazide alone with a dose of ≤ 160 mg/day, 160 - 320 mg/day, or ≥ 320 mg/day may be insufficient for adequate control of diabetes type 2.

For example, a treatment with Gliclazide LM alone with a dose of ≤ 60 mg/day, 60 - 120 mg/day, or ≥ 120 mg/day may be insufficient for adequate control of diabetes type 2.

For example, a treatment with Glimepiride alone with a dose of ≤ 4 mg/day, 4 - 8 mg/day, or ≥ 8 mg/day may be insufficient for adequate control of diabetes type 2.

For example, a treatment with Glipizide alone with a dose of ≤ 20 mg/day, 20 - 40 mg/day, or ≥ 40 mg/day may be insufficient for adequate control of diabetes type 2.

For example, a treatment with Glipizide XL alone with a dose of ≤ 10 mg/day, 10 - 20 mg/day, or ≥ 20 mg/day may be insufficient for adequate control of diabetes type 2.

For example, a treatment with Gliquidone alone with a dose of ≤ 60 mg/day, 60 - 90 mg/day, or ≥ 90 mg/day may be insufficient for adequate control of diabetes type 2.

For example, a treatment with Tolbutamide alone with a dose of ≤ 1500 mg/day or ≥ 1500 mg/day may be insufficient for adequate control of diabetes type 2.

The pharmaceutical combination for use of the present invention may further comprise (c) metformin or/and a pharmaceutically acceptable salt thereof.

Metformin is the international non proprietary name of 1,1-dimethylbiguanide (CAS Number 657-24-9). In the present invention, the term "metformin" includes any pharmaceutically acceptable salt thereof.

In the present invention, metformin or/and the pharmaceutically acceptable salt thereof may be administered orally. The skilled person knows formulations of metformin suitable for treatment of diabetes type 2 by oral administration. Metformin may be administered in a dose of at least 1.0 g/day or at least 1.5 g/day. For oral administration, metformin may be formulated in a solid dosage form, such as a tablet or pill.

In the present invention, the terms "add-on", "add-on treatment" and "add-on therapy" include treatment of diabetes mellitus type 2 with a sulfonyl urea, AVE0010 and metformin. The sulfonyl urea, metformin and AVE0010 may be administered within a time interval of 24 h. The sulfonyl urea, metformin and AVE0010 each may be administered in a once-a-day-dosage. The sulfonyl urea, metformin and AVE0010 may be administered by different administration routes. The sulfonyl urea, and metformin may be administered orally, and AVE0010 may be administered subcutaneously.

The pharmaceutical combination for use of the present invention preferably is for the treatment of a subject suffering from diabetes type 2, wherein diabetes type 2 is not adequately controlled by treatment with a combination of a sulfonyl urea and metformin alone, for instance with a dose of ≤ 1500 mg/day metformin, ≥ 1500 - ≤ 2500 mg/day metformin, 2500 - ≤ 3000 mg/day metformin, or ≥ 3000 mg/day metformin for at least 3 months. The sulfonyl urea may be selected from sulfonyl ureas described herein. The dose of the sulfonyl urea may be a dose as indicated herein.

The subject suffering from diabetes type 2 to be treated by the pharmaceutical combination of the present invention may be an obese subject. In the present invention, an obese subject may have a body mass index of at least 30.

The subject to be treated by the pharmaceutical combination of the present invention may have a HbA1 c value of at least 8%, In particular, the subject to be treated by the pharmaceutical combination of the present invention may have a HbA1c value in the range of 8% to 10%.

In the present invention, a subject the diabetes type 2 of which is not adequately controlled may have a HbA1c value in the range of 8 % to 10%.

After treatment with the combination of the present invention, the HbA1c value may reach a value below 8 %, below 7% or below 6.5%. These HbA1c values may be reached by treatment for at least 3 months.

The subject to be treated by the pharmaceutical combination of the present invention may be an adult subject. The subject may have an age in the range of 18 to 50 years.

Another aspect of the present invention is a pharmaceutical combination comprising
(a) desPro³⁶Exendin-4(1-39)-Lys₆-NH₂ or/and a pharmaceutically acceptable salt thereof, and
(b) a sulfonyl urea or/and a pharmaceutically acceptable salt thereof.

Preferably, the pharmaceutical combination of the present invention is for use in the treatment of diabetes mellitus type 2.

Preferably, the combination of the present invention is for use in the prevention of hypoglycaemia, as described herein, in diabetes mellitus type 2 patients.

More preferably the combination of the present invention is for use in the prevention of hypoglycaemia in a diabetes type 2 patient having an increased risk of hypoglycaemia, in particular a diabetes type 2 patient having experienced at least one hypoglycaemic event. The hypoglycaemic event can be a symptomatic hypoglycaemic event or a severe symptomatic hypoglycaemic event.

The pharmaceutical combination of the present invention may be administered as described herein in the context of the medical uses of the present invention. The compounds (a) and (b) of the combination of the present invention may be formulated as described herein in the context of the medical uses of the present invention.

In the pharmaceutical combination of the present invention desPro³⁶Exendin-4(1-39)-Lys₆-NH₂ or/and the pharmaceutically acceptable salt thereof can be prepared for subcutaneous administration.

In the pharmaceutical combination of the present invention the sulfonyl urea or/and the pharmaceutically acceptable salt thereof can be prepared for oral administration.

The pharmaceutical combination may further comprise (c) metformin or/and a pharmaceutically acceptable salt thereof. In the pharmaceutical combination, metformin may be prepared for oral administration, as described herein.

In the pharmaceutical combination, the sulfonyl urea can be selected from Glibenclamide, Glibenclamide MR, Gliclazide, Gliclazide LM, Glimepiride, Glipizide, Glipizide XL, Gliquidone, and Tolbutamide.

A specific combination of the present invention comprises AVE0010 and Glibenclamide.

Another specific combination of the present invention comprises AVE0010 and Glibenclamide MR.

Another specific combination of the present invention comprises AVE0010 and Gliclazide.

Another specific combination of the present invention comprises AVE0010 and Gliclazide LM.

Another specific combination of the present invention comprises AVE0010 and Glimepiride.

Another specific combination of the present invention comprises AVE0010 and Glipizide.

Another specific combination of the present invention comprises AVE0010 and Glipizide XL.

Another specific combination of the present invention comprises AVE0010 and Gliquidone.

Another specific combination of the present invention comprises AVE0010 and Tolbutamide.

A specific combination of the present invention comprises AVE0010, Glibenclamide and Metformin.

Another specific combination of the present invention comprises AVE0010 Glibenclamide MR and Metformin.

Another specific combination of the present invention comprises AVE0010, Gliclazide and Metformin.

Another specific combination of the present invention comprises AVE0010, Gliclazide LM and Metformin.

Another specific combination of the present invention comprises AVE0010, Glimepiride and Metformin.

Another specific combination of the present invention comprises AVE0010, Glipizide and Metformin.

Another specific combination of the present invention comprises AVE0010, Glipizide XL and Metformin.

Another specific combination of the present invention comprises AVE0010, Gliquidone and Metformin.

Another specific combination of the present invention comprises AVE0010, Tolbutamide and Metformin.

Dosages of the compounds in the specific combinations of the present invention may be selected as described herein.

The pharmaceutical combination of the present invention can be used in the glycemic control in diabetes mellitus type 2 patients. In the present Example, Table 11 summarizes the results of the primary efficacy parameter, change from baseline to week 24 (LOCF) in HbA_{1c} using an ANCOVA analysis. The pre-specified primary analysis showed that treatment with lixisenatide combined with a sulfonyl urea with or without metformin (also termed herein "lixisenatide-treated group", "lixisenatide group" or "lixisenatide-treated patients") resulted in a statistically significant decrease in HbA_{1c} from baseline to week 24, compared with the placebo group (LS mean difference versus the placebo group = -0.74%; pvalue <.0001). The placebo group received a sulfonyl urea with or without metformin. Table 12 summarizes the proportion of patients with treatment response (HbA_{1c} ≤6.5% or <7% at week 24, respectively). The analysis of HbA_{1c} responders using the CMH method showed a significant treatment difference versus placebo for the lixisenatide-treated group (pvalue <.0001). At week 24, 19.3% of lixisenatide-treated patients and 4.7% of placebo-treated patients had achieved HbA_{1c} values ≤ 6.5%; 36.4% of patients in the lixisenatide group and 13.5% of patients in the placebo group had achieved HbA_{1c} values <7%.

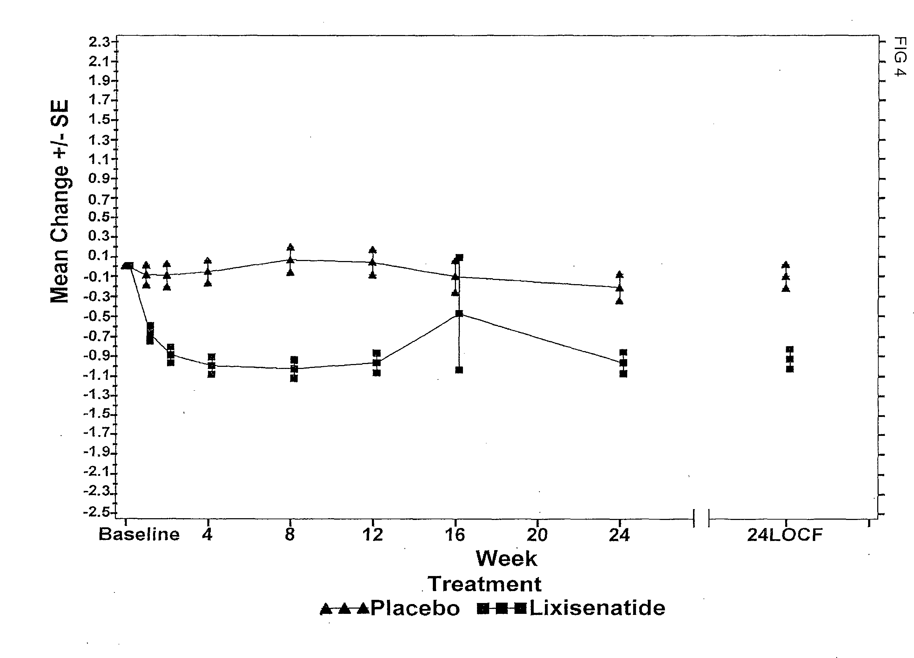

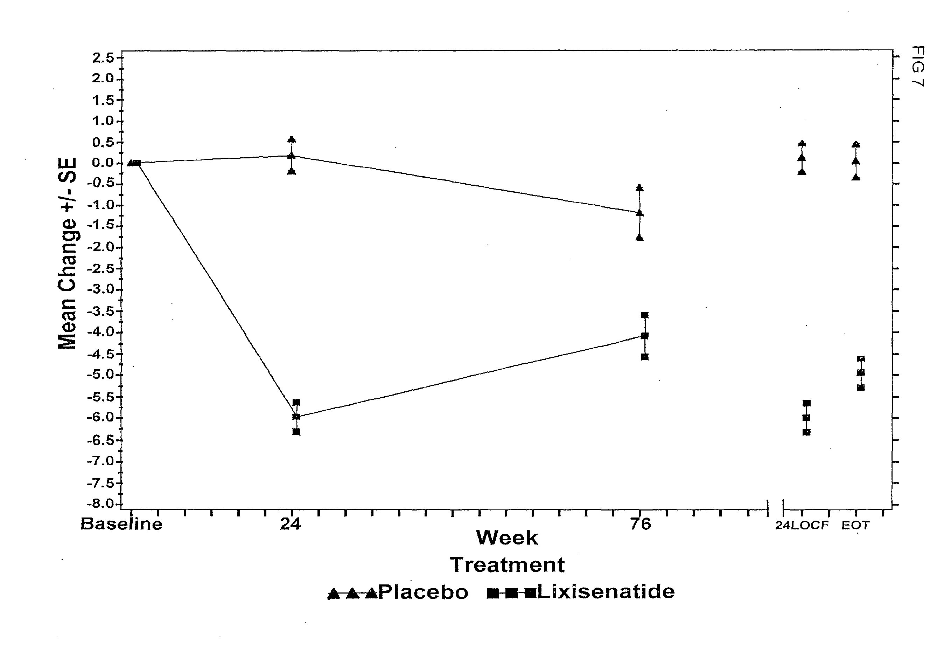

In particular, the pharmaceutical combination of the present invention can be used in the reduction of post-prandial plasma glucose concentration or/and in the reduction of fasting plasma glucose concentration. More particular, the pharmaceutical combination of the present invention can be use in the reduction of post-prandial plasma glucose concentration and in the reduction of fasting plasma glucose concentration. Tables 13, 14, and 17 summarize the ANCOVA analyses of 2-hour post-prandial plasma glucose concentration, fasting plasma glucose concentration (FPG), and HOMA-β, respectively. Figure 4 illustrates the Mean (±SE) change from baseline in FPG and body weight over time during the main 24-week double-blind treatment period. Figures 7, 8 and 10 in the appendix illustrate the mean (±SE) change from baseline over time in 2-hour post-prandial plasma glucose, FPG, and HOMA-β during the whole double-blind treatment period.

The results of the 2-hour post-prandial plasma glucose assessment showed a statistically significant improvement from baseline to week 24 in the lixisenatide group (lixisenatide combined with a sulfonyl urea with or without metformin) compared with the placebo group (sulfonyl urea with or without metformin) (LS mean difference versus placebo = 5.98 mmol/L; p-value <.0001, Table 13). For FPG, a statistically significant decrease from baseline to week 24 was observed in lixisenatide group compared with the placebo group (LS mean difference versus placebo = 0.63 mmol/L; p-value <.0001, Table 14). Treatment with lixisenatide substantially decreased glucose excursion from baseline to week 24 compared with the placebo group (LS mean difference = -5.57 mmol/L, 95% Cl = -6.397 to -4.744) as shown in Table 19.

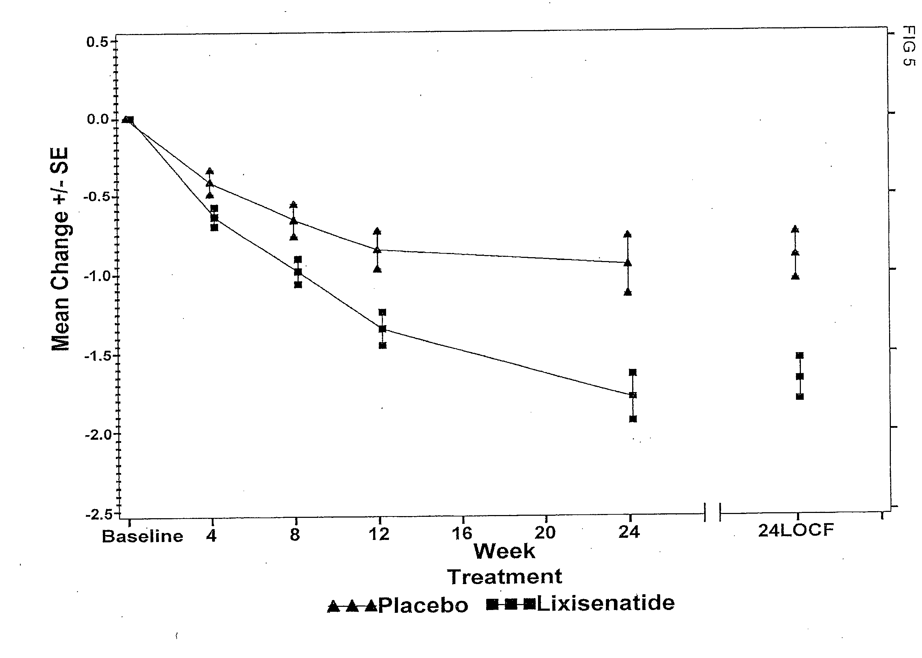

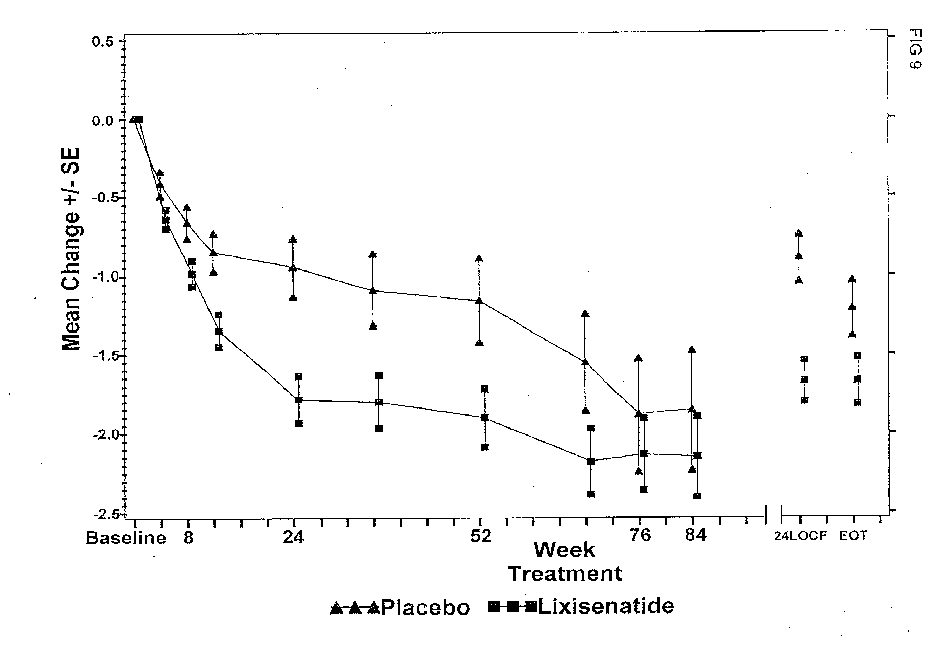

The pharmaceutical combination of the present invention can be used in the induction of weight loss in diabetes mellitus type 2 patients or/and in the prevention of weight gain in diabetes mellitus type 2 patients. Table 15 summarizes the ANCOVA analyses of body weight. Figure 5 illustrates the Mean (±SE) change from baseline in FPG and body weight over time during the main 24-week double-blind treatment period. Figure 9 illustrates body weight during the whole double-blind treatment period.

The LS mean body weight change from baseline at week 24 was -1.76 kg for the lixisenatide-treated patients (lixisenatide combined with a sulfonyl urea with or without metformin) and -0.93 kg for the placebo-treated patients (sulfonyl urea with or without metformin) (LS mean difference versus placebo = -0.84 kg) with statistically significant difference observed between treatment groups (pvalue <.0001). Body weight continued to decrease after the 24 week main treatment period in both treatments (Figure 9). About 14.4% lixisenatide-treated patients and 7.2% placebo-treated patients had ≥ 5% weight loss from baseline to week 24 (Table 16).

The invention is further illustrated by the following Figures and Example.

### Legends of the Figures

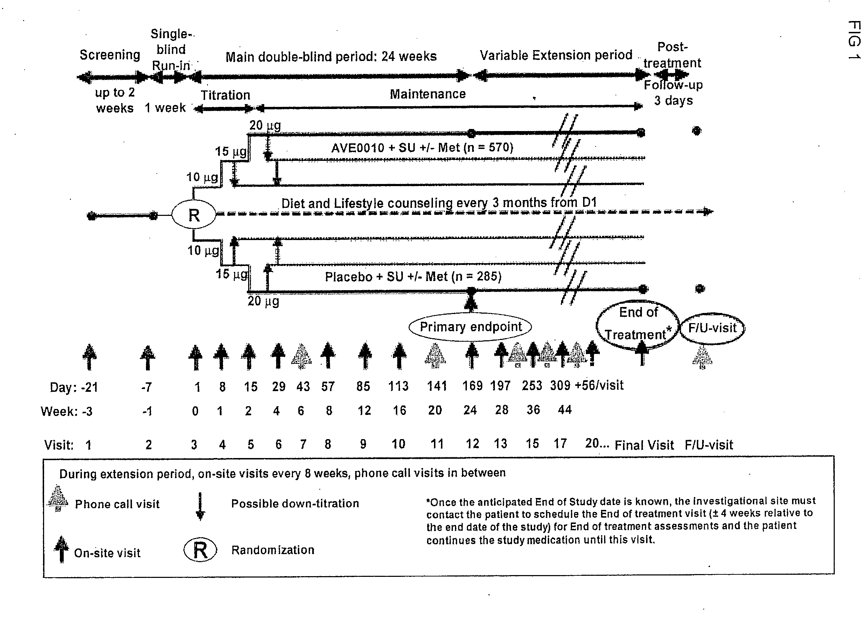

**Figure 1****:** Study design. SU: sulfonyl urea. Met: metformin.

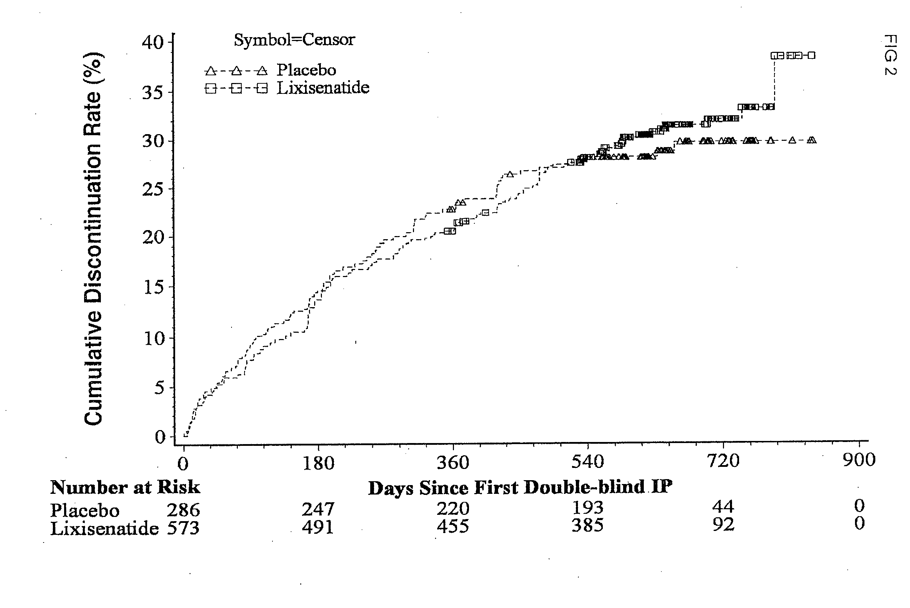

**Figure 2****:** Kaplan-Meier plot of time to treatment discontinuation due to any reason - Randomized population.

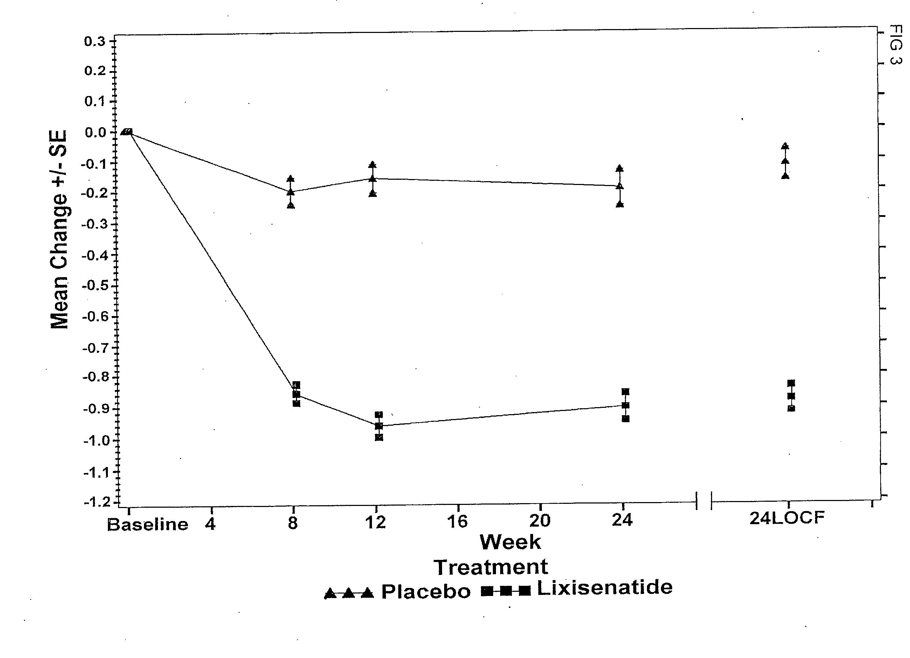

**Figure 3****:** Plot of mean change in HbA_{1c} (%) from baseline by visit up to week 24 and at endpoint- mITT population. LOCF = Last observation carry forward. Note: The plot included measurements obtained before the introduction of rescue medication and up to 3 days after the last dose of the double-blind investigational product injection on or before Visit 12 (Week 24), or Day 169 if Visit 12 (Week 24) is not available.
**Figure 4****:** Plot of mean change in fasting plasma glucose (mmol/L) from baseline by visit up to week 24 and at endpoint - mITT population. LOCF = Last observation carry forward. Note: The plot included measurements obtained before the introduction of rescue medication and up to 1 day after the last dose of the double-blind investigational product injection on or before Visit 12 (Week 24), or Day 169 if Visit 12 (Week 24) is not available.
**Figure 5****:** Plot of mean change in body weight (kg) from baseline by visit up to week 24 and at endpoint - mITT population. LOCF = Last observation carry forward. Note: The plot included measurements obtained before the introduction of rescue medication and up to 3 days after the last dose of the double-blind investigational product injection on or before Visit 12 (Week 24), or Day 169 if Visit 12 (Week 24) is not available.

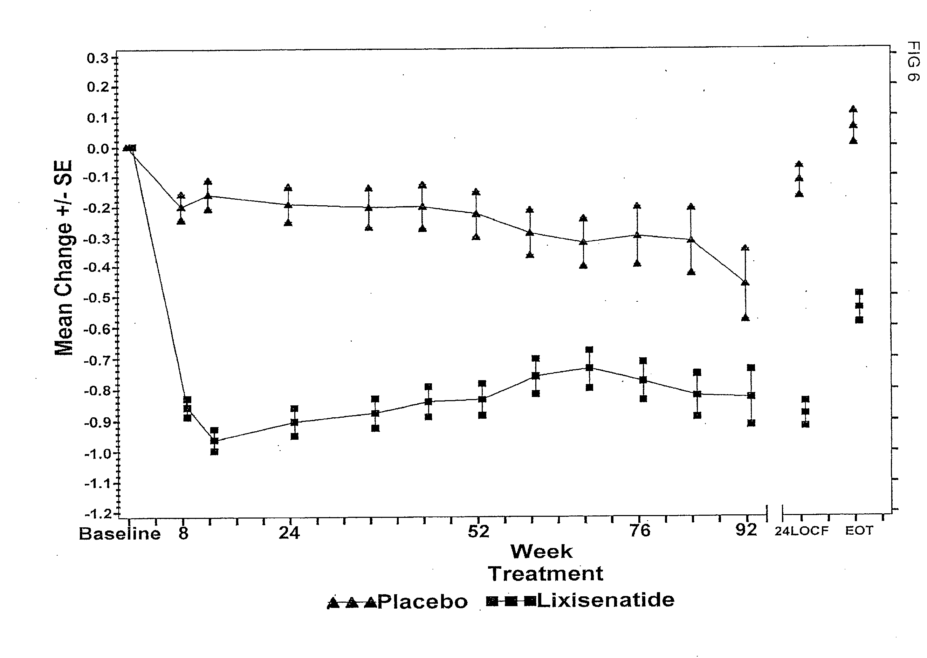

**Figure 6****:** Plot of mean change in HbA_{1c} (%) from baseline by visit and at endpoint- mITT population. LOCF = Last observation carry forward, EOT= Last on-treatment value. Note: The analysis excluded measurements obtained after the introduction of rescue medication and/or after the treatment cessation plus 3 days.
   For Week 24 (LOCF), the analysis included measurements obtained up to 3 days after the last dose of the double-blind investigational product injection on or before Visit 12 (Week 24), or Day 169 if Visit 12 (Week 24) is not available.
**Figure 7****:** Plot of mean change in 2-hour post-prandial plasma glucose (mmol/L) from baseline by visit and at endpoint in selected sites - mITT population. LOCF = Last observation carry forward, EOT= Last on-treatment value. Note: The analysis excluded measurements obtained after the introduction of rescue medication and/or after the treatment cessation. For Week 24 (LOCF), the analysis included measurements obtained up to the date of the last dose of the double-blind investigational product injection on or before Visit 12 (Week 24), or Day 169 if Visit 12 (Week 24) is not available.

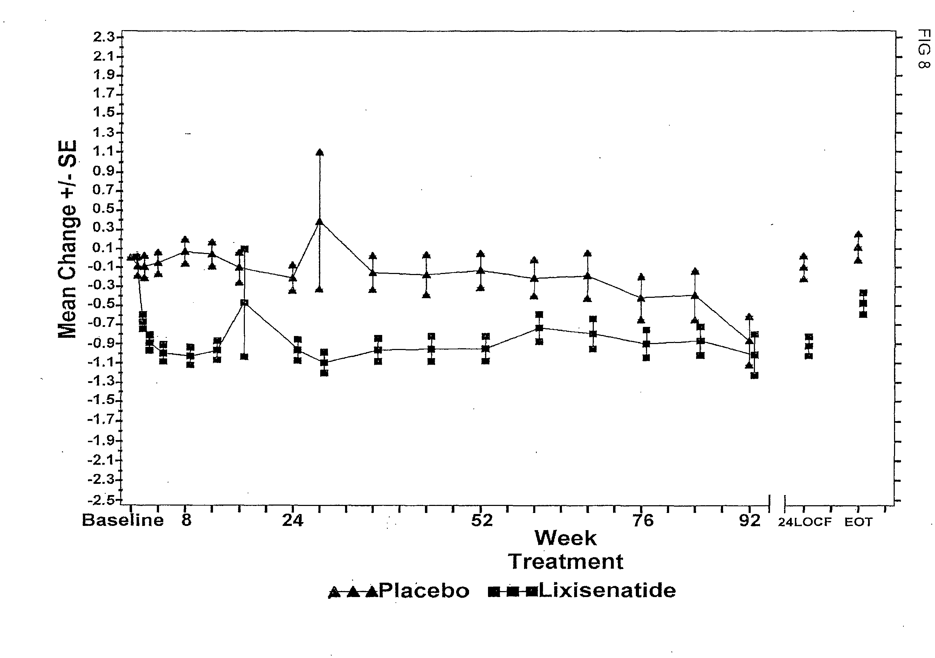

**Figure 8****:** Plot of mean change in fasting plasma glucose (mmol/L) from baseline by visit and at endpoint - mITT population. LOCF = Last observation carry forward, EOT= Last on-treatment value. Note: The analysis excluded measurements obtained after the introduction of rescue medication and/or after the treatment cessation plus 1 day. For Week 24 (LOCF), the analysis included measurements obtained up to 1 day after the last dose of the double-blind investigational product injection on or before Visit 12 (Week 24), or Day 169 if Visit 12 (Week 24) is not available.
**Figure 9****:** Plot of mean change in body weight (kg) from baseline by visit and at endpoint - mITT population. LOCF = Last observation carry forward, EOT= Last on-treatment value. Note: The analysis excluded measurements obtained after the introduction of rescue medication and/or after the treatment cessation plus 3 days. For Week 24 (LOCF), the analysis included measurements obtained up to 3 days after the last dose of the double-blind investigational product injection on or before Visit 12 (Week 24), or Day 169 if Visit 12 (Week 24) is not available.

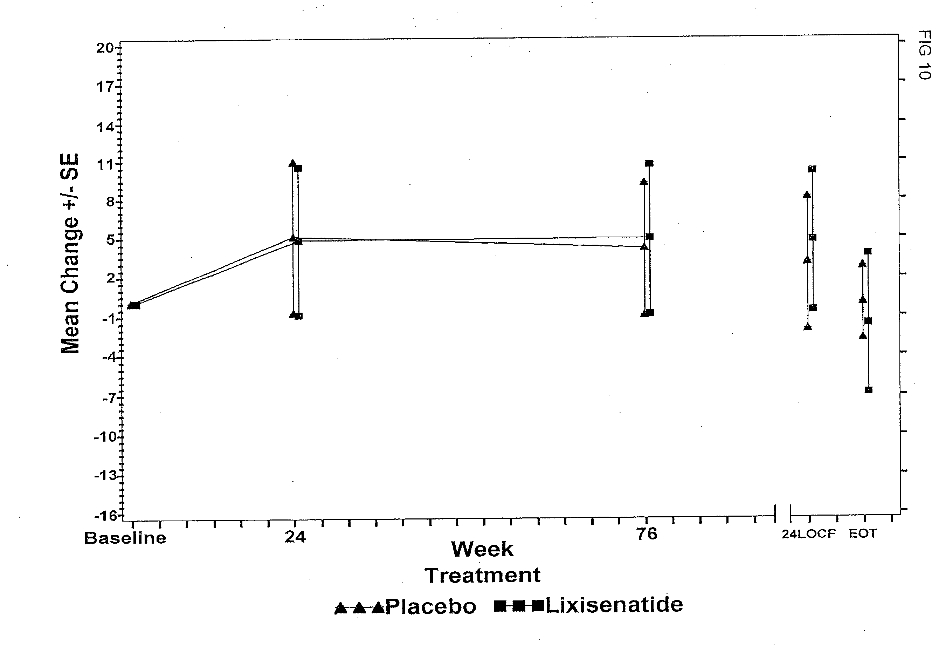

**Figure 10****:** Plot of mean change in HOMA-β from baseline by visit and at endpoint in selected sites- mITT population. LOCF = Last observation carry forward, EOT= Last on-treatment value. Note: The analysis excluded measurements obtained after the introduction of rescue medication and/or after the treatment cessation. For Week 24 (LOCF), the analysis included measurements obtained up to the date of the last dose of the double-blind investigational product injection on or before Visit 12 (Week 24), or Day 169 if Visit 12 (Week 24) is not available.

### Example

### A randomized, double-blind, placebo-controlled, 2-arm, parallel-group, multinational study assessing the efficacy and safety of lixisenatide in comparison to placebo as an add-on treatment to sulfonylurea in combination with or without metformin in patients with type 2 diabetes

### Summary

This example describes a randomized, double-blind, placebo-controlled, 2-arm, parallel-group, multinational study assessing the efficacy and safety of lixisenatide in comparison to placebo as an add-on treatment to sulfonylurea in combination with or without metformin in patients with type 2 diabetes. The approximate minimum study duration per patient was 79 weeks (up to 3 weeks screening + 24-week main treatment + variable extension + 3 days follow-up). The study was conducted in 136 centers in 16 countries. The primary objective of the study was to assess the efficacy of lixiseriatide on glycemic control in comparison to placebo in terms of HbA₁c reduction (absolute change) over a period of 24 weeks.

A total of 859 patients were randomized to one of the two treatment groups (573 in the lixisenatide group and 286 in the placebo group). All randomized patients were exposed to the study treatment. Demographics and baseline characteristics were generally similar across the treatment groups. Eleven patients (9 patients on lixisenatide and 2 patients on placebo) were excluded from the mITT population for efficacy analyses due to lack of post-baseline efficacy data. During the overall study treatment period, 259 (30.2%) patients prematurely discontinued the study treatment. The percentages of patients who discontinued the treatment were similar between treatment groups (30.9% for lixisenatide and 28.7% for placebo). For the lixisenatide group, the main reason for treatment discontinuation was "adverse events" (12.4% versus 8.0% for placebo) followed by "other reasons" (11.7% versus 9.1% for placebo).

The least squared (LS) mean changes from baseline to week 24 in HbA_{1c} were -0.85% for the lixisenatide group and -0.10% for the placebo group (LS mean difference vs. placebo = -0.74%; p-value <.0001). A total of 198 patients (36.4%) in the lixisenatide group had HbA_{1c}<7% at week 24 compared to 37 patients (13.5%) in the placebo group, and 105 (19.3%) of lixisenatide-treated patients had HBA_{1c} ≤6.5% compared to 13 (4.7%) of placebo-treated patients. The HbA_{1c} responder analysis (HbA_{1c} ≤6.5 or <7% at week 24) using Cochran-Mantel-Haenszel (CMH) method also showed a significant treatment difference versus placebo for lixisenatide group at week 24 (p-value <.0001).

Treatment with lixisenatide also improved post-prandial glycemic control as shown by the results for the 2-hour post-prandial plasma glucose (PPG) assessment and for glucose excursion. A statistically significant improvement in PPG was demonstrated in the lixisenatide group, compared with the placebo group with a LS mean difference of -5.98 mmol/L (p-value <.0001). Furthermore, treatment with lixisenatide demonstrated a statistically significant decrease in fasting plasma glucose (LS mean difference of -0.63 mmol/L; p-value < .0001) and body weight (LS mean difference of -0.84 kg; p-value <.0001) compared with the placebo group. For β-cell function assessed by HOMA-β, no significant difference was observed between treatment groups per a pre-specified parametric analysis. Since the normality assumption for the parametric model was violated, a sensitivity analysis using a nonparametric model was performed and it showed a statistically significant difference (p-value=0.0011) The percentage of patients requiring rescue therapy was statistically significantly lower for the lixisenatide group compared to placebo (20 patients [3.5%] in the lixisenatide group and 34 patients [12.0%] in the placebo group) without an adjustment for multiplicity.

Lixisenatide was well tolerated. The incidence of treatment emergent adverse events (TEAEs) was higher in the lixisenatide group compared to the placebo group (81.5% in the lixisenatide group compared with 75.8% in the placebo group), which was mainly attributable to a difference in TEAE coming from the Primary System Organ Class "Gastrointestinal Disorders", mainly nausea (28.0% in the lixisenatide group compared with 8.8% in the placebo group) and vomiting (10.6% in the lixisenatide group compared with 5.3% in the placebo group). Two patients in the lixisenatide group had TEAEs leading to death. Ninety three serious TEAEs occurred during the on-treatment period for the whole study with a slightly lower incidence rate in the lixisenatide group (10.1%) compared to the placebo group (12.3%). The most commonly reported TEAE for lixisenatide-treated patients was nausea (28.0% versus 8.8% for placebo-treated patients) followed by hypoglycemia (24.6% versus 19.3% for placebo-treated patients). One hundred twenty seven (22.1%) lixisenatide-treated patients had symptomatic hypoglycemia events as defined in the protocol during the on-treatment period whereas 51 (17.9%) patients in the placebo group reported symptomatic hypoglycemia during the same period. Three of the symptomatic hypoglycemia events were severe (2 patients [0.3%] in the lixisenatide group and 1 patient [0.4%] in the placebo group). A total of 12 patients (11 [1.9%] lixisenatide-treated patients and 1 [0.4%] placebo-treated patient) reported events adjudicated as an allergic reaction by the Allergic Reaction Assessment Committee (ARAC) but only one (lixisenatide-treated patient) of these (local reaction) was adjudicated as possibly related to the investigational product.

### 1. Objectives

### 1.1 Primary objective

The primary objective of this study was to assess the efficacy of lixisenatide on glycemic control in comparison to placebo as an add-on treatment to sulfonylurea, with or without metformin, in terms of absolute HbA_{1c} reduction over a period of 24 weeks in patients with type 2 diabetes.

### 1.2 Secondary objective(s)

The secondary objectives of this study were:
- To assess the effects of lixisenatide on:
   - Percentage of patients reaching HbA_{1c} <7% or HbA_{1c} ≤6.5%,
   - Body weight,
   - Fasting plasma glucose (FPG),
   - β-cell function assessed by HOMA-β,
   - 2-hour post-prandial plasma glucose (PPG), glucagon, insulin, proinsulin, and C-peptide after a standardized meal challenge test in a substudy in all the patients in selected centers (approximately 30% of all randomized patients),
- To assess the safety and tolerability of lixisenatide
- To assess lixisenatide pharmacokinectics and anti-lixisenatide antibody development

### 2. Trial design

This was a double-blind, randomized, placebo-controlled, 2-arm, parallel-group multinational study with an unbalanced 2:1 randomization ratio (i.e., 570 lixisenatide and 285 placebo treated patients). The study was double-blind with regard to active and placebo treatments. The study drug volume (i.e., dose of active drug or matching placebo) was not blinded.

The patients were stratified by screening values of glycosylated hemoglobin A_{1c} (HbA_{1c}) (<8%, ≥8%) and metformin use at screening (Yes, No). After a screening period, patients were centrally randomized via interactive voice response system (IVRS) in a 2:1 ratio to either lixisenatide or placebo.

Per the protocol amendment 4, the approximate minimum study duration per patient was 79 weeks (up to 3 weeks screening + 24 weeks main double-blind treatment + variable extension + 3 days follow-up). Patients who completed the 24-week main double-blind treatment period underwent a variable double-blind treatment extension period, which ended for all patients approximately at the scheduled date of week 76 visit (V25) for the last randomized patient.

Per the protocol amendment 3, patients who prematurely discontinued the study treatment were continued in the study up to the scheduled date of study completion. They were followed up according to the study procedures as specified in the protocol (except 3-day safety post-treatment follow-up, pharmacokinetics assessment, and meal challenge test).

The standardized meal challenge test was performed in all patients in selected centers (to obtain approximately 30% of all randomized patients).

### 3. Primary and key secondary endpoints

### 3.1 Primary endpoint

The primary efficacy variable was the absolute change in HbA_{1c} from baseline to week 24, which was defined as: HbA_{1c} at week 24 - HbA_{1c} at baseline.

If a patient discontinued the treatment prematurely or received rescue therapy during the main 24-week double-blind treatment period or did not have HbA_{1c} value at week 24 visit, the last post-baseline HbA_{1c} measurement during the main 24-week double-blind on-treatment period was used as HbA_{1c} value at week 24 (Last Observation Carry Forward [LOCF] procedure).

### 3.2 Secondary endpoints

### 3.2.1 Efficacy endpoints

For secondary efficacy variables, the same procedure for handling missing assessment/early discontinuation was applied as for the primary variable.

### Continuous variables

- Change in 2-hour post-prandial plasma glucose (mmol/L) after a standardized meal from baseline to week 24
- Change in FPG (mmol/L) from baseline to week 24
- Change in body weight (kg) from baseline to week 24
- Change in β-cell function assessed by HOMA-β from baseline to week 24
- Change in glucose excursion (2-hour post-prandial plasma glucose - plasma glucose 30 minutes prior to the meal test before study drug administration) (mmol/L) during a standardized meal challenge test from baseline to week 24
- Change in the following variables under fasting (30 minutes prior to the meal test before study drug administration) and 2-hour post-prandial conditions collected during a standardized meal test: glucagon (ng/L), plasma insulin (pmol/L), proinsulin (pmol/L), proinsulin-to-insulin ratio, and C-peptide (nmol/L) from baseline to week 24

### Categorical variables

- Percentage of patients with HbA_{1c} <7% at week 24
- Percentage of patients with HbA_{1c} ≤6.5% at week 24
- Percentage of patients requiring rescue therapy during the main 24-week double-blind treatment period
- Percentage of patients with ≥5% weight loss (kg) from baseline to week 24

### 3.2.2 Safety endpoints

The safety analysis was based on the reported TEAEs and other safety information including symptomatic hypoglycemia and severe symptomatic hypoglycemia, local tolerability at injection site, allergic events (as adjudicated by ARAC), suspected pancreatitis, increased calcitonin, vital signs, 12-lead ECG and laboratory tests.
Major cardiovascular events were also collected and adjudicated by a Cardiovascular Adjudication Committee (CAC). The adjudicated and confirmed events by CAC from this study and other lixisenatide phase 2-3 studies will be pooled for analyses and summarized in a separate report based on the statistical analysis plan for the overall cardiovascular assessment of lixisenatide.

### 4. Sample size calculation assumptions

The sample size calculation was based on the primary efficacy variable, change from baseline to week 24 in HbA_{1c}. This calculation assumed a common standard deviation of 1.3% with a 2-sided test at the 5% significance level and was based upon the 2-sample t-test, and was performed using nQuery Advisor® 5.0.

A sample size of 855 patients (570 for lixisenatide and 285 for placebo) was considered sufficient to detect a difference of 0.5% (or 0.4%) in the absolute change from baseline in HbA_{1c} to week 24 between lixisenatide and placebo, with a power of 99% (or 98%).

### 5. Statistical methods

### 5.1 Analysis populations

The modified intent-to-treat (mITT) population consisted of all randomized patients who received at least one dose of double-blind investigational product (IP), and had both a baseline assessment and at least one post-baseline assessment of efficacy variables.

The safety population was defined as all randomized patients who took at least one dose of the double-blind IP.

### 5.2 Primary efficacy analysis

The primary efficacy variable (change in HbA_{1c} from baseline to week 24) was analyzed using an analysis of covariance (ANCOVA) model with treatment, randomization strata of screening HbA_{1c} (<8.0, ≥8.0%), randomization strata of metformin use at screening (Yes, No) and country as fixed effects and using the baseline value as a covariate. Difference between lixisenatide and placebo and two-sided 95% confidence interval as wells as p-value were estimated within the framework of ANCOVA.

The primary analysis of the primary efficacy variable was performed based on the mITT population and the measurements obtained during the main 24-week double-blind on-treatment period for efficacy variables. The main 24-week double-blind on-treatment period for efficacy variables except those from the meal challenge test was defined as the time from the first dose of the double-blind IP up to 3 days (except for FPG by central laboratory, which was up to 1 day) after the last dose of the double-blind IP injection on or before V12/week 24 visit (or D169 if V12/week 24 visit was missing), or up to the introduction of the rescue therapy, whichever was the earliest. The main 24-week double-blind on-treatment period for efficacy variables from the meal challenge test including PPG, glucagon, plasma insulin, proinsulin, C-peptide, glucose excursion, HOMA-β and proinsulin-to-insulin ratio was defined as the time from the first dose of the double-blind IP up to the date of the last dose of the double-blind IP injection on or before V12/week 24 visit (or D169 if V12/week 24 visit was missing), or up to the introduction of the rescue therapy, whichever was the earliest. The LOCF procedure was used by taking this last available post-baseline on-treatment HbA_{1c} measurement (before the introduction of rescue therapy) as the HbA_{1c} value at week 24.

### 5.3 Secondary efficacy analysis

A stepwise testing procedure was applied in order to ensure control of type 1 error. Once the primary variable was statistically significant at α=0.05, the testing procedure was performed to test the following secondary efficacy variables by the following prioritized order. The tests stop as soon as an endpoint was found not statistically significant at α=0.05.
1. Change in 2-hour post-prandial plasma glucose (mmol/L) after a standardized meal test from baseline to week 24,
2. Change in FPG (mmol/L) from baseline to week 24,
3. Change in body weight (kg) from baseline to week 24,
4. Change in β-cell function assessed by HOMA-β from baseline to week 24,
5. Percentage of patients requiring rescue therapy during the main 24-week double-blind treatment period.

Similar to the analyses of HbA_{1c}, all continuous secondary efficacy variables as described in Section 3.2.1 were analyzed by ANCOVA as already described. The adjusted estimates of the treatment mean difference between lixisenatide and placebo and two-sided 95% confidence intervals were provided.

The following categorical secondary efficacy variables were analyzed using a Cochran-Mantel-Haenszel (CMH) method stratified on randomization strata (screening HbA_{1c} [<8.0, ≥8%] and metformin, use at screening [Yes, No]):
- Percentage of patients with HbA_{1c} <7.0% at week 24,
- Percentage of patients with HbA_{1c} ≤6.5% at week 24,
- Percentage of patients requiring rescue therapy during the main 24-week double-blind treatment period.
Number and percentage of patients with ≥5% weight loss from baseline at week 24 were presented by treatment groups.

All secondary endpoints at the end of treatment were only evaluated by descriptive statistics (mean, standard deviation, median and ranges).

### 5.4 Safety analysis

The safety analyses were primarily based on the on-treatment period for the whole study. The **on-treatment period for the whole study** was defined as the time from the first dose of double-blind IP up to 3 days after the last dose of IP administration during the whole study period regardless of rescue status. The 3-day interval was chosen based on the half-life of the IP (approximately 5 times the half-life).

The summary of safety results (descriptive statistics or frequency tables) is presented by treatment groups.

### 6. Results

### 6.1 Study patients

### 6.1.1 Patient accountability

The study was conducted in 136 centers in 16 countries (Bulgaria, Czech Republic, Egypt, Germany, India, Israel, Japan, Korea, Netherlands, Romania, Russian Federation, Taiwan, Thailand, Tunisia, Turkey and United States). A total of 1438 patients were screened and 859 were randomized to one of the two treatment groups. The main reason for screening failure was HbA_{1c} value at the screening visit out of the protocol defined ranges (306 [21.3%] out of 1438 screened patients).

All 859 randomized patients were exposed to the study treatment. Eleven patients (9 patients in the lixisenatide group and 2 patients in the placebo group) were excluded from mITT population for efficacy analyses due to lack of post-baseline efficacy data. Table 1 provides the number of patients included in each analysis population. One patient (#158503006) who was randomized to placebo by IVRS received the lixisenatide kits most of the time during the study (543 out of 561 days) due to a site dispensing error and consequently an error in IVRS. Therefore, she was considered a placebo patient in the mITT population (for efficacy analysis) but a lixisenatide-treated patient in the safety population (for safety analysis) per the data handling convention for mixed treatments.

**Table 1 Analysis populations - Randomized population**

| | **Placebo (N=286)** | **Lixisenatide (N=573)** | **All (N=859)** |
|---|---|---|---|
| Randomized population | 286 (100%) | 573 (100%) | 859 (100%) |
| Efficacy population Modified Intent-to-Treat (mITT) | 284 (99.3%) | 564 (98.4%) | 848 (98.7%) |
| Safety population | 285 | 574 | 859 |

| | | | |
|---|---|---|---|
| Note: The safety population patients are tabulated according to treatment actually received (as treated). For the efficacy population, patients are tabulated according to their randomized treatment (as randomized). | | | |

All 468 randomized patients (155 in placebo group and 313 in lixisenatide group) in Israel, Japan, Korea, Russian Federation and United States participated in meal challenge test. Out of 468 patients, 463 (154 in the placebo group and 309 in the lixisenatide group) are in the mITT population.

### 6.1.2 Study disposition

Table 2 provides the summary of patient disposition for each treatment group. During the overall treatment period, 259 (30.2%) patients prematurely discontinued the study treatment. The percentages of patients who discontinued the treatment were similar between treatment groups (30.9% for lixisenatide and 28.7% for placebo). For the lixisenatide group, the main reason for treatment discontinuation was "adverse events" (12.4% versus 8.0% for placebo) followed by "other reasons" (11.7% versus 9.1% for placebo). Similar results were observed for the 24-week main treatment period, where a total of 105 (12.2%) patients prematurely discontinued the study treatment with the main reason also being adverse events (8.4% for lixisenatide and 3.8% for placebo). The time-to-onset of treatment discontinuation due to any reason for the overall treatment period is depicted in Figure 2. A slightly higher discontinuation trend was observed for the placebo group between 6 months and 18 months, but after 18 months the lixisenatide group showed a slightly higher rate.

Out of 23 placebo-treated patients who discontinued the treatment due to an AE (Table 2), one discontinued the treatment due to an AE (injection site pain) that started prior to the first dosing (ie, pre-treatment AE), while 22 had TEAEs leading to treatment discontinuation (Table 20).

**Table 2 Patient disposition - Randomized population**

| | **Placebo (N=286)** | **Lixisenatide (N=573)** |
|---|---|---|
| Randomized and treated | 286 (100%) | 573 (100%) |
| Did not complete 24-week double-blind study treatment | 31 (10.8%) | 74 (12.9%) |
| | | |
| Subject's request for 24-week treatment discontinuation | 25 (8.7%) | 66 (11.5%) |
| | | |
| Reason for 24-week study treatment discontinuation | 31 (10.8%) | 74 (12.9%) |
| Adverse event | 11 (3.8%) | 48 (8.4%) |
| Lack of efficacy | 7 (2.4%) | 3 (0.5%) |
| Poor compliance to protocol | 1 (0.3%) | 5 (0.9%) |
| Lost to follow-up | 2 (0.7%) | 3 (0.5%) |
| Other reasons | 10 (3.5%) | 15 (2.6%) |
| | | |
| Did not complete double-blind study treatment | 82 (28.7%) | 177 (30.9%) |
| | | |
| Subject's request for treatment discontinuation | 64 (22.4%) | 148 (25.8%) |
| | | |
| Reason for study treatment discontinuation | 82 (28.7%) | 177 (30.9%) |
| Adverse event | 23 (8.0.%) | 71 (12.4%) |
| Lack of efficacy | 24 (8.4%) | 16 (2.8%) |
| Poor compliance to protocol | 7 (2.4%) | 14 (2.4%) |
| Lost to follow-up | 2 (0.7%) | 9 (1.6%) |
| Other reasons | 26 (9.1%) | 67 (11.7%) |
| | | |
| Status at last study contact | 286 (100%) | 573 (100%) |
| Alive | 283 (99.0%) | 559 (97.6%) |
| Dead | 1 (0.3%) | 3 (0.5%) |
| Lost to follow-up | 2 (0.7%) | 11 (1.9%) |

| | | |
|---|---|---|
| Note: Percentages are calculated using the number of randomized patients as denominator. | | |

### 6.1.3 Demographics and baseline characteristics

The demographic and patient baseline characteristics were generally similar between the two treatment groups for the safety population (Table 3). The median age of the study population was 58.0 years. The majority of the patients were Caucasian (52.2%) and Asian (44.8%).

**Table 3 Demographics and patient characteristics at screening or baseline - Safety population**

| | **Placebo (N=285)** | **Lixisenatide (N=574)** | **All (N=859)** |
|---|---|---|---|
| Age (years) | | | |
| Number | 285 | 574 | 859 |
| Mean (SD) | 57.8 (10.1) | 57.0 (9.8) | 57.2 (9.9) |
| Median | 58.0 | 58.0 | 58.0 |
| Min : Max | 20 : 78 | 25: 79 | 20:79 |
| | | | |
| Age group (years) [n (%)] | | | |
| Number | 285 | 574 | 859 |
| < 50 | 60 (21.1%) | 129 (22.5%) | 189 (22.0%) |
| ≥ 50 to < 65 | 151 (53.0%) | 314 (54.7%) | 465 (54.1 %) |
| ≥ 65 to < 75 | 65 (22.8%) | 116 (20.2%) | 181 (21.1%) |
| ≥ 75 | 9 (3.2%) | 15 (2.6%) | 24 (2.8%) |
| | | | |
| Gender [n (%)] | | | |
| Number | 285 | 574 | 859 |
| Male | 150 (52.6%) | 284 (49.5%) | 434 (50.5%) |
| Female | 135 (47.4%) | 290 (50.5%) | 425 (49.5%) |
| | | | |
| Race [n (%)] | | | |
| Number | 285 | 574 | 859 |
| Caucasian/White | 151 (53.0%) | 297 (51.7%) | 448 (52.2%) |
| Black | 9 (3.2%) | 17 (3.0%) | 26 (3.0%) |
| Asian/Oriental | 125 (43.9%) | 260 (45.3%) | 385 (44.8%) |
| Other | 0 | 0 | 0 |
| | | | |
| Ethnicity [n (%)] | | | |
| Number | 285 | 574 | 859 |
| Hispanic | 5 (1.8%) | 18 (3.1%) | 23 (2.7%) |
| Not Hispanic | 280 (98.2%) | 556 (96.9%) | 836 (97.3%) |
| | | | |
| Screening HbA1c (%) | | | |
| Number | 285 | 574 | 859 |
| Mean (SD) | 8.32 (0.81) | 8.37 (0.82) | 8.36 (0.82) |
| Median | 8.30 | 8.30 | 8.30 |
| Min: Max | 7.0 : 10.0 | 7.0 : 10.0 | 7.0 : 10.0 |
| | | | |
| Randomization strata of screening HbA1c (%) [n (%)] | | | |
| Number | 285 | 574 | 859 |
| <8 | 101 (35.4%) | 202 (35.2%) | 303 (35.3%) |
| ≥8 | 184 (64.6%) | 372 (64.8%) | 556 (64.7%) |
| | | | |
| Randomization strata of metformin use at screening [n (%)] | | | |
| Number | 285 | 574 | 859 |
| Yes | 240 (84.2%) | 483 (84.1%) | 723 (84.2%) |
| No | 45 (15.8%) | 91 (15.9%) | 136 (15.8%) |
| | | | |
| Baseline BMI (kg/m²) | | | |
| Number | 285 | 574 | 859 |
| Mean (SD) | 30.42 (6.64) | 30.13 (6.62) | 30.22 (6.62) |
| Median | 29.29 | 28.66 | 28.93 |
| Min : Max | 19.5 : 59.3 | 19.0 : 60.8 | 19.0 : 60.8 |
| Baseline BMI Categories (kg/m²) [n(%)] | | | |
| Number | 285 | 574 | 859 |
| <30 | 152 (53.3%) | 325 (56.6%) | 477 (55.5%) |
| ≥30 | 133 (46.7%) | 249 (43.4%) | 382 (44.5%) |

| | | | |
|---|---|---|---|
| BMI = Body Mass Index. | | | |

Disease characteristics including diabetic history were generally comparable between the two treatment groups (Table 4). The median duration of sulfonylurea treatment for the study population was 4.23 years (Table 5). Patients were mainly on glimepiride (42.1 %), glibenclamide (24.9%) and gliclazide LM (12.7%). Of 859 patients, 134 (15.6%) was on the sulfonylurea only and 725 (84.4%) had both sulfonylurea and metformin at the screening visit (Table 6). There was a discrepancy in the number of patients between "randomization strata of metformin use at screening" and actual "metformin use at screening" due to randomization strata errors.

**Table 4 Disease characteristics at screening or randomization - Safety population**

| | **Placebo (N=285)** | **Lixisenatide (N=574)** | **All (N=859)** |
|---|---|---|---|
| Duration of diabetes (years) | | | |
| Number | 285 | 574 | 859 |
| Mean (SD) | 9.81 (6.20) | 9.14(5.96) | 9.36 (6.04) |
| Median | 8.53 | 7.99 | 8.13 |
| Min : Max | 1.1 : 40.0 | 1.0: 35.3 | 1.0 : 40.0 |
| | | | |
| Age at onset of type 2 diabetes (years) | | | |
| Number | 285 | 574 | 859 |
| Mean (SD) | 48.0 (9.6) | 47.8 (9.4) | 47.9 (9.5) |
| Median | 49.0 | 48.0 | 49.0 |
| Min : Max | 14:71 | 13 : 73 | 13:73 |
| | | | |
| History of gestational diabetes [n (%)] | | | |
| Number (Female) | 135 | 290 | 425 |
| Yes (Female) | 5 (3.7%) | 9 (3.1%) | 14 (3.3%) |
| No (Female) | 130 (96.3%) | 281 (96.9%) | 411 (96.7%) |
| | | | |
| Prior use of GLP-1 receptor agonist [n (%)] | | | |
| Number | 285 | 574 | 859 |
| Yes | 16 (5.6%) | 17 (3.0%) | 33 (3.8%) |
| No | 269 (94.4%) | 557 (97.0%) | 826 (96.2%) |
| Diabetic retinopathy [n (%)] | | | |
| Number | 285 | 573 | 858 |
| Yes | 42 (14.7%) | 90 (15.7%) | 132 (15.4%) |
| No | 236 (82.8%) | 474 (82.7%) | 710 (82.8%) |
| Unknown | 7 (2.5%) | 9 (1.6%) | 16 (1.9%) |
| | | | |
| Diabetic sensory or motor neuropathy [n (%)] | | | |
| Number | 285 | 573 | 858 |
| Yes | 82 (28.8%) | 168 (29.3%) | 250 (29.1%) |
| No | 201 (70.5%) | 399 (69.6%) | 600 (69.9%) |
| Unknown | 2 (0.7%) | 6 (1.0%) | 8 (0.9%) |
| | | | |
| Diabetic autonomic neuropathy [n (%)] | | | |
| Number | 285 | 573 | 858 |
| Yes | 10 (3.5%) | 15 (2.6%) | 25 (2.9%) |
| No | 269 (94.4%) | 546 (95.3%) | 815 (95.0%) |
| Unknown | 6(2.1%) | 12 (2.1%) | 18 (2.1%) |
| | | | |
| Diabetic nephropathy [n (%)] | | | |
| Number | 285 | 573 | 858 |
| Yes | 29 (10.2%) | 48 (8.4%) | 77 (9.0%) |
| Microalbuminuria | 17 (6.0%) | 31 (5.4%) | 48 (5.6%) |
| Overt proteinuria | 10 (3.5%) | 14 (2.4%) | 24 (2.8%) |
| Impaired renal function | 0 | 1 (0.2%) | 1 (0.1%) |
| Dialysis or transplantation | 0 | 0 | 0 |
| No | 253 (88.8%) | 519 (90.6%) | 772 (90.0%) |
| Unknown | 3 (1.1%) | 6 (1.0%) | 9(1.0%) |
| | | | |
| Categorized albuminuria at randomization [n (%)] | | | |
| Number | 17 | 39 | 56 |
| <3 mg/L (Not reportable) | 1 (5.9%) | 7 (17.9%) | 8 (14.3%) |
| ≥3 mg/L (Reportable) | 16 (94.1%) | 32 (82.1%) | 48 (85.7%) |
| <20 mg/L | 7 (41.2%) | 20 (51.3%) | 27 (48.2%) |
| ≥20 - <200 mg/L | 9 (52.9%) | 11 (28.2%) | 20 (35.7%) |
| ≥200 mg/L | 0 | 1 (2.6%) | 1 (1.8%) |
| | | | |
| Creatinine clearance at screening (ml/min) | | | |
| Number | 285 | 573 | 858 |
| Mean (SD) | 112.62 (41.63) | 113.80 (43.69) | 113.41 (43.00) |
| Median | 104.77 | 103.55 | 103.75 |
| Min : Max | 39.9 : 288.4 | 39.9 : 350.4 | 39.9 : 350.4 |
| | | | |
| Creatinine clearance categories at screening [n (%)] | | | |
| Number | 285 | 573 | 858 |
| <30 ml/min (severe renal impairment) | 0 | 0 | 0 |
| ≥30 - <50 ml/min (moderate renal impairment) | 4 (1.4%) | 7 (1.2%) | 11 (1.3%) |
| ≥50 - ≤80 ml/min (mild renal impairment) | 66 (23.2%) | 113 (19.7%) | 179 (20.9%) |
| >80 ml/min (no renal impairment) | 215 (75.4%) | 453 (79.1%) | 668 (77.9%) |

| | | | |
|---|---|---|---|
| GLP-1 = Glucagon like peptide-1. Creatinine clearance value is derived using the equation of Cockcroft and Gault. | | | |

**Table 5 Disease characteristics - Sulfonylurea at screening or randomization - Safety population**

| | **Placebo (N=285)** | **Lixisenatide (N=574)** | **ALL (N=859)** |
|---|---|---|---|
| Duration of sulfonylurea treatment (years) | | | |
| Number | 284 | 572 | 856 |
| Mean (SD) | 5.30(4.20) | 5.24 (4.38) | 5.26(4.32) |
| Median | 4.44 | 4.21 | 4.23 |
| Min : Max | 0.3: 21.0 | 0.2:27.7 | 0.2: 27.7 |
| | | | |
| Sulfonylurea at randomization by INN and formulation [n (%)] | | | |
| Number | 285 | 574 | 859 |
| Glibenclamide | 69 (24.2%) | 145 (25.3%) | 214 (24.9%) |
| Glibenclamide MR | 23 (8.1%) | 44 (7.7%) | 67 (7.8%) |
| Gliclazide | 10 (3.5%) | 24 (4.2%) | 34 (4.0%) |
| Gliclazide LM | 27 (9.5%) | 82 (14.3%) | 109 (12.7%) |
| Glimepiride | 129 (45.3%) | 233 (40.6%) | 362(42.1%) |
| Glipizide | 15 (5.3%) | 21 (3.7%) | 36 (4.2%) |
| Glipizide XL | 10 (3.5%) | 24 (4.2%) | 34 (4.0%) |
| Gliquidone | 0 | 1 (0.2%) | 1 (0.1%) |
| Tolbutamide | 2 (0.7%) | 1 (0.2%) | 3 (0.3%) |
| | | | |
| Daily dose of sulfonylurea at randomization by INN and formulation (mg/day) Glibenclamide | | | |
| Number | 69 | 145 | 214 |
| Mean (SD) | 12.7(4.4) | 13.0(4.4) | 12.9(4.4) |
| Median | 10.5 | 10.5 | 10.5 |
| Min : Max | 5 : 20 | 5:30 | 5 : 30 |
| | | | |
| Glibenclamide MR | | | |
| Number | 23 | 44 | 67 |
| Mean (SD) | 16.8(14.4) | 14.0 (4.7) | 15.0(9.2) |
| Median | 14.0 | 14.0 | 14.0 |
| Min : Max | 10 : 80 | 5:20 | 5 : 80 |
| | | | |
| Gliclazide | | | |
| Number | 10 | 24 | 34 |
| Mean (SD) | 220.0 (57.3) | 226.7 (59.8) | 224.7 (58.3) |
| Median | 240.0 | 240.0 | 240.0 |
| Min : Max | 120 : 320 | 120: 320 | 120: 320 |
| | | | |
| Gliclazide LM | | | |
| Number | 27 | 82 | 109 |
| Mean (SD) | 84.4 (22.1) | 88.9 (26.4) | 87.8 (25.4) |
| Median | 90.0 | 90.0 | 90.0 |
| Min : Max | 60: 120 | 30 : 120 | 30: 120 |
| | | | |
| Glimepiride | | | |
| Number | 129 | 233 | 362 |
| Mean (SD) | 4.9 (1.3) | 5.1 (1.6) | 5.1 (1.5) |
| Median | 4.0 | 4.0 | 4.0 |
| Min : Max | 2 : 8 | 2:12 | 2: 12 |
| Glipizide | | | |
| Number | 15 | 21 | 36 |
| Mean (SD) | 23.7 (7.7) | 22.6 (9.2) | 23.1 (8.5) |
| Median | 20.0 | 20.0 | 20.0 |
| Mix :Max | 15:40 | 10:40 | 10:40 |
| | | | |
| Glipizide XL | | | |
| Number | 10 | 24 | 34 |
| Mean (SD) | 13.5 (4.7) | 15.8 (5.0) | 15.1 (5.0) |
| Median | 10.0 | 20.0 | 17.5 |
| Min : Max | 10:20 | 10:20 | 10:20 |
| | | | |
| Gliquidone | | | |
| Number | 0 | 1 | 1 |
| Mean (SD) | | 90.0 (NC) | 90.0 (NC) |
| Median | | 90.0 | 90.0 |
| Min : Max | | 90 : 90 | 90 : 90 |
| | | | |
| Tolbutamide | | | |
| Number | 2 | 1 | 3 |
| Mean (SD) | 1500.0 (0.0) | 1500.0 (NC) | 1500.0 (0.0) |
| Median | 1500.0 | 1500.0 | 1500.0 |
| Min : Max | 1500 : 1500 | 1500:1500 | 1500: 1500 |
| | | | |
| Categorized daily dose of sulfonylurea at randomization by INN [n (%)] | | | |
| Glibenclamide (Gd) and glibenclamide MR (GdMR) | | | |
| Number | 92 | 189 | 281 |
| <10 mg/day (Gd) or <6 mg/day (GdMR) | 10 (10.9%) | 12 (6.3%) | 22 (7.8%) |
| ≥10 - <20 mg/day (Gd) or ≥6 - <12 mg/day (GdMR) | 57 (62.0%) | 128 (67.7%) | 185 (65.8%) |
| ≥20 mg/day (Gd) or ≥12 mg/day (GdMR) | 25 (27.2%) | 49 (25.9%) | 74 (26.3%) |
| | | | |
| Gliclazide (Gz) and gliclazide LM (GzLM) | | | |
| Number | 37 | 106 | 143 |
| <160 mg/day (Gz) or <60 mg/day (GzLM) | 1 (2.7%) | 3 (2.8%) | 4 (2.8%) |
| ≥160 - <320 mg/day (Gz) or ≥60 - <120 mg/day (GzLM) | 30 (81.1%) | 70 (66.0%) | 100 (69.9%) |
| ≥320 mg/day (Gz) or ≥120 mg/day (GzLM) | 6 (16.2%) | 33 (31.1%) | 39 (27.3%) |
| | | | |
| Glimepiride | | | |
| Number | 129 | 233 | 362 |
| <4 mg/day | 1 (0.8%) | 2 (0.9%) | 3 (0.8%) |
| ≥4 - <8 mg/day | 118 (91.5%) | 195 (83.7%) | 313 (86.5%) |
| ≥8 mg/day | 10 (7.8%) | 36 (15.5%) | 46 (12.7%) |
| Glipizide (Gp) and glipizide XL (GpXL) | | | |
| Number | 25 | 45 | 70 |
| <20 mg/day (Gp) or <10 mg/day (GpXL) | 1 (4.0%) | 3 (6.7%) | 4 (5.7%) |
| ≥20 - <40 mg/day (Gp) or ≥10 - <20 mg/day (GpXL) | 19 (76.0%) | 24 (53.3%) | 43 (61.4%) |
| ≥40 mg/day (Gp) or ≥20 mg/day (GpXL) | 5 (20.0%) | 18 (40.0%) | 23 (32.9%) |
| | | | |
| Gliquidone | | | |
| Number | 0 | 1 | 1 |
| <60 mg/day | 0 | 0 | 0 |
| ≥60 - <90 mg/day | 0 | 0 | 0 |
| ≥90 mg/day | 0 | 1 (100%) | 1 (100%) |
| | | | |
| Tolbutamide | | | |
| Number | 2 | 1 | 3 |
| <1500 mg/day | 0 | 0 | 0 |
| ≥1500 mg/day | 2 (100%) | 1 (100%) | 3 (100%) |

| | | | |
|---|---|---|---|
| NC = Not computable INN = International nonproprietary name | | | |

**Table 6 Disease characteristics - Metformin at screening or baseline - Safety population**

| | **Placebo (N=285)** | **Lixisenatide (N=574)** | **All (N=859)** |
|---|---|---|---|
| Metformin use at screening [n (%)] | | | |
| Number | 285 | 574 | 859 |
| Yes | 239 (83.9%) | 486 (84.7%) | 725 (84.4%) |
| No | 46(16.1%) | 88 (15.3%) | 134 (15.6%) |
| | | | |
| Duration of metformin treatment (years) | | | |
| Number | 238 | 484 | 722 |
| Mean (SD) | 5.60 (4.12) | 5.02 (4.25) | 5.21 (4.21) |
| Median | 5.16 | 4.10 | 4.36 |
| Min : Max | 0.3: 24.7 | 0.3: 35.1 | 0.3 : 35.1 |
| | | | |
| Daily dose of metformin at baseline (mg) | | | |
| Number | 239 | 486 | 725 |
| Mean (SD) | 1778.9 (566.8) | 1850.9 (541.8) | 1827.2 (550.8) |
| Median | 2000.0 | 2000.0 | 2000.0 |
| Min : Max | 750 : 3400 | 750 : 4050 | 750 : 4050 |
| | | | |
| Categorized daily dose of metformin at baseline (mg) [n (%)] | | | |
| Number | 239 | 486 | 725 |
| <1500 | 37 (15.5%) | 57 (11.7%) | 94 (13.0%) |
| ≥1500 - < 2500 | 167 (69.9%) | 345 (71.0%) | 512 (70.6%) |
| ≥2500 - < 3000 | 26 (10.9%) | 65 (13.4%) | 91 (12.6%) |
| ≥3000 | 9 (3.8%) | 19 (3.9%) | 28 (3.9%) |

HbA_{1c} and FPG at baseline were comparable between the two treatment groups for the safety population (Table 7). A higher mean body weight at baseline was observed in the placebo group (84.42 kg) compared with the lixisenatide group (82.30 kg), but mean baseline BMI was similar between the two treatment groups (30.13 kg/m² for lixisenatide versus 30.42 kg/m² for placebo) as shown in Table 3.

**Table 7 Baseline efficacy variables - Safety population**

| | **Placebo (N=285)** | **Lixisenatide (N=574)** | **All (N=859)** |
|---|---|---|---|
| HbA1c (%) | | | |
| Number | 285 | 574 | 859 |
| Mean (SD) | 8.21 (0.84) | 8.28 (0.86) | 8.25 (0.85) |
| Median | 8.20 | 8.20 | 8.20 |
| Min : Max | 6.4 : 10.8 | 6.5 : 12.5 | 6.4 : 12.5 |
| | | | |
| Weight (kg) | | | |
| Number | 285 | 574 | 859 |
| Mean (SD) | 84.42 (22.83) | 82.30 (21.76) | 83.00 (22.13) |
| Median | 80.80 | 78.00 | 79.00 |
| Min : Max | 45.3 : 166.8 | 45.7 : 200.4 | 45.3 : 200.4 |
| | | | |
| FPG (mmol/L) | | | |
| Number | 285 | 574 | 859 |
| Mean (SD) | 9.29 (2.37) | 9.67 (2.24) | 9.55 (2.29) |
| Median | 9.20 | 9.40 | 9.30 |
| Min : Max | 4.4 : 19.6 | 4.6:19.8 | 4.4 : 19.8 |
| | | | |
| 2-hour post-prandial plasma glucose (mmol/L) | | | |
| Number | 154 | 309 | 463 |
| Mean (SD) | 16.44 (3.74) | 16.69 (4.02) | 16.60 (3.92) |
| Median | 16.30 | 16.60 | 16.50 |
| Min : Max | 7.3 : 25.0 | 5.0: 31.0 | 5.0 : 31.0 |
| | | | |
| Glucose excursion* (mmol/L) | | | |
| Number | 154 | 309 | 463 |
| Mean (SD) | 6.84 (3.78) | 6.99 (3.71) | 6.94 (3.73) |
| Median | 6.70 | 6:70 | 6.70 |
| Min : Max | -12.2 : 15.2 | -10.7: 17.9 | -12.2 : 17.9 |
| | | | |
| HOMA-β* | | | |
| Number | 152 | 306 | 458 |
| Mean (SD) | 36.43 (39.43) | 34.28 (69.82) | 34.99 (61.39) |
| Median | 25.21 | 23.60 | 24.25 |
| Min : Max | 2.3 : 282.6 | 2.4: 1036.0 | 2.3 : 1036.0 |

| | | | |
|---|---|---|---|
| FPG = Fasting plasma glucose. * For patients in selected sites where the meal challenge test was performed. Glucose excursion = 2-hour post-prandial plasma glucose - plasma glucose 30 minutes prior to the meal test before study drug administration. | | | |

### 6.1.4 Dosage and duration

The average treatment exposure was similar between the two treatment groups: 531.7 days (76.0 weeks) for the lixisenatide group and 528.4 days (75.5 weeks) for the placebo group [Table 8]. Out of 859 patients, 739 (85.2% in the lixisenatide group and 87.7% in the placebo group) had at least 169 days (24 weeks) of treatment and 571 (66.0% in the lixisenatide group and 67.4% in the placebo group) had at least 547 days (18 months} of treatment. Sixteen patients (13 for lixisenatide and 3 for placebo) had the last administration date on the "End of treatment" CRF missing mainly due to lost to follow-up (9 for lixisenatide and 2 for placebo) and hence their treatment durations were set to missing following the SAP data handling convention.

For the lixisenatide group, 509 (88.7%) patients and 515 (89.7 %) patients were at the target total daily dose of 20 µg at the end of the 24-week double-blind treatment period and at the end of double-blind treatment, respectively (Tables 9 and 10). For the placebo group, 277 (97.2%) patients and 276 (96.8%) patients were at the target total daily dose of 20 µg at the end of 24-week double-blind treatment period and at the end of double-blind treatment, respectively (Tables 9 and 10).

**Table 8 Exposure-Safety population**

| | **Placebo (N=285)** | **Lixisenatide (N=574)** |
|---|---|---|
| Cumulative duration of treatment exposure (patient years) | 407.9 | 816.7 |
| | | |
| Duration of study treatment (days) | | |
| Number | 282 | 561 |
| Mean (SD) | 528.4 (220.6) | 531.7 (219.5) |
| Median | 617.0 | 610.0 |
| Min : Max | 5 : 840 | 1 : 839 |
| Duration of study treatment by category [n (%)] | | |
| Missing duration | 3 (1.1%) | 13 (2.3%) |
| 1-14 days | 7 (2.5%) | 9 (1.6%) |
| 15-28 days | 3 (1.1%) | 10 (1.7%) |
| 29-56 days | 5 (1.8%) | 15 (2.6%) |
| 57-84 days | 3 (1.1%) | 12 (2.1%) |
| 85-168 days | 14 (4.9%) | 26 (4.5%) |
| 169-364 days | 32 (11.2%) | 38 (6.6%) |
| 365-546 days | 26 (9.1%) | 72 (12.5%) |
| 547-728 days | 150 (52.6%) | 293 (51.0%) |
| >728 days | 42 (14.7%) | 86 (15.0%) |
| | | |
| Cumulative duration of study treatment by category [n (%)] | | |
| Missing duration | 3 (1.1%) | 13 (2.3%) |
| ≥ 1 day | 282 (98.9%) | 561 (97.7%) |
| ≥ 15 days | 275 (96.5%) | 552 (96.2%) |
| ≥ 29 days | 272 (95.4%) | 542 (94.4%) |
| ≥ 57 days | 267 (93.7%) | 527 (91.8%) |
| ≥ 85 days | 264 (92.6%) | 515 (89.7%) |
| ≥ 169 days | 250 (87.7%) | 489 (85.2%) |
| ≥ 365 days | 218 (76.5%) | 451 (78.6%) |
| ≥ 547 days | 192 (67.4%) | 379 (66.0%) |
| ≥ 729 days | 42 (14.7%) | 86 (15.0%) |

| | | |
|---|---|---|
| Duration of exposure = (date of the last double-blind investigational product injection - date of the first double-blind investigational product injection) + 1. | | |

**Table 9 Number (%) of patients by final total daily dose at the end of the 24-week double-blind treatment - Safety population**

| **Dose at the end of the 24-week** | **Placebo (N=285)** | **Lixisenatide (N=574)** |
|---|---|---|
| 10 µg | 4 (1.4%) | 30 (5.2%) |
| 15 µg | 4 (1.4%) | 35 (6.1%) |
| 20 µg | 277 (97.2%) | 509 (88.7%) |

| | | |
|---|---|---|
| Dose = Dose of active drug or volume-matched placebo. Note: Percentages are calculated using the number of safety patients as the denominator. | | |

**Table 10 Number (%) of patients by final total daily dose at the end of the double-blind treatment - Safety population**

| **Final dose** | **Placebo (N=285)** | **Lixisenatide (N=574)** |
|---|---|---|
| 10 µg | 5 (1.8%) | 32 (5.6%) |
| 15 µg | 4 (1.4%) | 27 (4.7%) |
| 20 µg | 276 (96.8%) | 515 (89.7%) |

| | | |
|---|---|---|
| Dose = Dose of active drug or volume-matched placebo. Note: Percentages are calculated using the number of safety patients as the denominator. | | |

### 6.2 Efficacy

### 6.2.1 Primary efficacy endpoint

### Main analysis

Table 11 summarizes the results of the primary efficacy parameter, change from baseline to week 24 (LOCF) in HbA_{1c} using an ANCOVA analysis.

The pre-specified primary analysis showed that treatment with lixisenatide resulted in a statistically significant decrease in HbA_{1c} from baseline to week 24, compared with the placebo group (LS mean difference versus the placebo group = -0.74%; pvalue <.0001)

**Table 11 Mean change in HbA_{1c} (%) from baseline to week 24 - mlTT population**

| **HbA1c (%)** | **Placebo (N=284)** | **Lixisenatide (N=564)** |
|---|---|---|
| Baseline | | |
| Number | 274 | 544 |
| Mean (SD) | 8.22 (0.83) | 8.28 (0.86) |
| Median | 8.20 | 8.20 |
| Min : Max | 6.4 : 10.8 | 6.5 : 12.5 |
| | | |
| Week 24 (LOCF) | | |
| Number | 274 | 544 |
| Mean (SD) | 8.10 (1.11) | 7.40 (1.00) |
| Median | 7.90 | 7.30 |
| Min : Max | 6.0 : 12.2 | 5.1 : 12.4 |
| | | |
| Change from baseline to week 24 (LOCF) | | |
| Number | 274 | 544 |
| Mean (SD) | -0.12 (0.82) | -0.88 (0.93) |
| Median | -0.10 | -0.90 |
| Min : Max | -2.4 : 3.1 | -3.3 : 3.4 |
| LS Mean (SE) ^{a} | -0.10 (0.071) | -0.85 (0.061) |
| | | |
| LS Mean difference (SE) vs. Placebo ^{a} | - | -0.74 (0.063) |
| 95% Cl | - | (-0.867 to -0.621) |
| p-value | | <.0001 |

| | | |
|---|---|---|
| LOCF = Last observation carry forward. ^{a} Analysis of covariance (ANCOVA) model with treatment groups (lixisenatide and placebo), randomization strata of screening HbA1c (<8.0, ≥8.0%), randomization strata of metformin use at screening, and country as fixed effects and baseline HbA1 c value as a covariate. Note: The analysis included measurements obtained before the introduction of rescue medication and up to 3 days after the last dose of the double-blind investigational product injection on or before Visit 12 (Week 24), or Day 169 if Visit 12 (Week 24) is not available. Patients with both baseline and Week 24 (LOCF) measurements are included. | | |

Figure 3 illustrates the mean (±SE) change from baseline in HbA₁c over time during the main 24-week double-blind treatment period. Figure 6 in the appendix illustrates the Mean (±SE) change from baseline in HbA₁c over time up to week 92. The HbA_{1c} reduction was relatively maintained over time beyond 24 weeks.

### Secondary analysis

Table 12 summarizes the proportion of patients with treatment response (HbA_{1c} ≤6.5% or <7% at week 24, respectively). The analysis of HbA_{1c} responders using the CMH method showed a significant treatment difference versus placebo for the lixisenatide-treated group (p-value <.0001). At week 24, 19.3% of lixisenatide-treated patients and 4.7% of placebo-treated patients had achieved HbA_{1c} values ≤6.5%; 36.4% of patients in the lixisenatide group and 13.5% of patients in the placebo group had achieved HbA_{1c} values <7%.

**Table 12 Number (%) of patients with HbA_{1c} value≤6.5% or <7% respectively at week 24 - mlTT population**

| **HbA1c (%)** | **Placebo (N=284)** | **Lixisenatide (N=564)** |
|---|---|---|
| Number | 274 | 544 |
| ≤6.5% | 13 (4.7%) | 105 (19.3%) |
| >6.5% | 261 (95.3%) | 439 (80.7%) |
| p-value vs. placebo^{a} | - | <0.0001 |
| | | |
| Number | 274 | 544 |
| <7.0% | 37 (13.5%) | 198 (36.4%) |
| ≥7.0% | 237 (86.5%) | 346 (63.6%) |
| p-value vs. placebo^{a} | - | <0.0001 |

| | | |
|---|---|---|
| ^{a} Cochran-Mantel-Haenszel (CMH) method stratified by randomization strata of screening HbA1c (<8.0 or ≥8.0 %) and randomization strata of metformin use at screening (Yes or No). Note: The analysis included measurements obtained before the introduction of rescue medication and up to 3 days after the last dose of the double-blind investigational product injection on or before Visit 12 (week 24), or Day 169 if Visit 12 (week 24) is not available.) | | |

### 6.2.2 Secondary efficacy endpoints

Tables 13, 14, 15 and 17 summarize the ANCOVA analyses of 2-hour post-prandial plasma glucose, FPG, body weight and HOMA-β, respectively. Figure 4 and Figure 5 illustrate the Mean (±SE) change from baseline in FPG and body weight over time during the main 24-week double-blind treatment period. Figures 7, 8, 9 and 10 in the appendix illustrate the mean (±SE) change from baseline over time in 2-hour post-prandial plasma glucose, FPG (up to week 92), body weight (up to week 84) and HOMA-β during the whole double-blind treatment period.

The results of the 2-hour post-prandial plasma glucose assessment showed a statistically significant improvement from baseline to week 24 in lixisenatide group compared with the placebo group (LS mean difference versus placebo = 5.98 mmol/L; p-value <.0001)

For FPG, a statistically significant decrease from baseline to week 24 was observed in lixisenatide group compared with the placebo group (LS mean difference versus placebo = 0.63 mmol/L; p-value <.0001)

The LS mean body weight change from baseline at week 24 was -1.76 kg for the lixisenatide-treated patients and -0.93 kg for the placebo-treated patients (LS mean difference versus placebo = -0.84 kg) with statistically significant difference observed between treatment groups (p-value <0001). Body weight continued to decrease after the 24 week main treatment period in both treatments (Figure 9). About 14.4% lixisenatide-treated patients and 7.2% placebo-treated patients had ≥ 5% weight loss from baseline to week 24 (Table 16).

For β-cell function assessed by HOMA-β, a median increase of 4.37 for the lixisenatide group compared with -0.33 for the placebo group was observed, with no significant difference between treatment groups (LS mean difference versus placebo = 1.80; p-value = 0.7387) per a pre-specified parametric analysis (Table 17). The mean change in HOMA-β from baseline to week 24 in the lixisenatide group (4.76) was higher compared with the placebo group (3.05), but the change in LS mean for the lixisenatide group (4.83) was lower compared with the placebo group (6.63) due to an outlier in the lixisenatide group. This outlier did not affect the conclusion from the parametric model. Since the normality assumption for the parametric model was violated, a sensitivity analysis using a nonparametric model was performed and it showed a statistically significant difference (p-value=0.0011).

As per the testing strategy adjusting for multiplicity, inferential testing for the percentages of patients requiring rescue therapy at week 24 was exploratory since the preceding test (HOMA-β) failed to show a statistically significant difference between groups.

The percentages of patients requiring rescue therapy at week 24 was significantly lower for the lixisenatide group compared to placebo (20 patients [3.5%] in the lixisenatide group and 34 patients [12.0%] in the placebo group) without an adjustment for multiplicity (Table 18).

Treatment with lixisenatide substantially decreased glucose excursion from baseline to week 24 compared with the placebo group (LS mean difference = -5.57 mmol/L, 95% Cl = -6.397 to -4.744) as shown in Table 19.

**Table 13 Mean change in 2-hour post-prandial plasma glucose (mmol/L) from baseline to week 24 in selected sites - miTT population**

| **2-hour post-prandial plasma glucose (mmol/L)** | **Placebo (N=284)** | **Lixisenatide (N=564)** |
|---|---|---|
| Baseline | | |
| Number | 120 | 249 |
| Mean (SD) | 16.55 (3.74) | 16.61 (4.09) |
| Median | 16.70 | 16.50 |
| Min : Max | 7.3 : 25.0 | 5.0 : 29.3 |
| | | |
| Week 24 (LOCF) | | |
| Number | 120 | 249 |
| Mean (SD) | 16.67 (3.89) | 10.61 (4.73) |
| Median | 16.85 | 9.80 |
| Min : Max | 7.6 : 27.1 | 3.2 : 24.5 |
| Change from baseline to week 24 (LOCF) | | |
| Number | 120 | 249 |
| Mean (SD) | 0.12 (3.71) | -6.00 (5.39) |
| Median | 0.20 | -5.90 |
| Min : Max | -10.8: 17.7 | -21.6 : 11.9 |
| LS Mean (SE)^{a} | -0.21 (0.489) | -6.19 (0.408) |
| | | |
| LS Mean difference (SE) vs. Placebo ^{a} | - | -5.98 (0.475) |
| 95% CI | - | (-6.912 to -5.043) |
| p-value | | <.0001 |

| | | |
|---|---|---|
| LOCF = Last observation carry forward. ^{a} Analysis of covariance (ANCOVA) model with treatment groups (lixisenatide and placebo), randomization strata of screening HbA1c (<8.0, ≥8.0%), randomization strata of metformin use at screening (Yes, No), and country as fixed effects and baseline 2-hour post-prandial plasma glucose value as a covariate. Note: The analysis included measurements obtained before the introduction of rescue medication and up to the date of the last dose of the double-blind investigational product injection on or before Visit 12 (Week 24), or Day 169 if Visit 12 (Week 24) is not available. Patients with both baseline and Week 24 (LOCF) measurements are included. | | |

**Table 14 Mean change in fasting plasma glucose (mmol/L) from baseline to week 24 - mITT population**

| **Fasting plasma glucose (mmol/L)** | **Placebo (N=284)** | **Lixisenatide (N=564)** |
|---|---|---|
| Baseline | | |
| Number | 283 | 564 |
| Mean (SD) | 9.29 (2.37) | 9.67 (2.24) |
| Median | 9.20 | 9.40 |
| Min : Max | 4.4 : 19.6 | 4.6 : 19.8 |
| | | |
| Week 24 (LOCF) | | |
| Number | 283 | 564 |
| Mean (SD) | 9.19 (2.33) | 8.74 (2.32) |
| Median | 8.90 | 8.60 |
| Min : Max | 3.6: 19.4 | 2.4: 21.5 |
| | | |
| Change from baseline to week 24 (LOCF) | | |
| Number | 283 | 564 |
| Mean (SD) | -0.10 (2.00) | -0.93 (2.37) |
| Median | -0.20 | -0.83 |
| Min : Max | -6.8 : 7.0 | -15.7 : 6.3 |
| LS Mean (SE) ^{a} | -0.36 (0.161) | -0.99 (0.139) |
| | | |
| LS Mean difference (SE) vs. Placebo ^{a} | - | -0.63 (0.146) |
| 95% Cl | - | (-0.919 to -0.346) |
| p-value | | <.0001 |

| | | |
|---|---|---|
| LOCF = Last observation carry forward. ^{a} Analysis of covariance (ANCOVA) model with treatment groups (lixisenatide and placebo), randomization strata of screening HbA1c (<8.0, ≥8.0%), metformin use at screening (Yes, No), and country as fixed effects and baseline fasting plasma glucose as a covariate. Note: The analysis included measurements obtained before the introduction of rescue medication and up to 1 day after the last dose of the double-blind investigational product injection on or before Visit 12 (Week 24), or Day 169 if Visit 12 (Week 24) is not available. Patients with both baseline and Week 24 (LOCF) measurements are included. | | |

**Table 15 Mean change in body weight (kg) from baseline to week 24 - mITT population**

| **Body weight (kg)** | **Placebo (N=284)** | **Lixisenatide (N=564)** |
|---|---|---|
| Baseline | | |
| Number | 278 | 554 |
| Mean (SD) | 84.52 (22.81) | 82.58 (21.88) |
| Median | 80.85 | 78.00 |
| Min : Max | 45.3 : 166.8 | 45.7: 200.4 |
| | | |
| Week 24 (LOCF) | | |
| Number | 278 | 554 |
| Mean (SD) | 83.63 (22.98) | 80.91 (21.37) |
| Median | 80.20 | 76.75 |
| Min : Max | 45.5 : 168.6 | 44.6: 191.4 |
| | | |
| Change from baseline to week 24 (LOCF) | | |
| Number | 278 | 554 |
| Mean (SD) | -0.89 (2.48) | -1.67 (3.08) |
| Median | -1.00 | -1.30 |
| Min : Max | -12.4 : 14.1 | -16.9 : 16.3 |
| LS Mean (SE) ^{a} | -0.93 (0.234) | -1.76 (0.202) |
| | | |
| LS Mean difference (SE) vs. Placebo ^{a} | - | -0.84 (0.211) |
| 95% Cl | - | (-1.250 to -0.421) |
| p-value | | <.0001 |

| | | |
|---|---|---|
| LOCF = Last observation carry forward. ^{a} Analysis of covariance (ANCOVA) model with treatment groups (lixisenatide and placebo), randomization strata of screening HbA1c (<8.0, ≥8.0%), metformin use at screening (Yes, No), and country as fixed effects and baseline body weight as a covariate. Note: The analysis included measurements obtained before the introduction of rescue medication and up to 3 days after the last dose of the double-blind investigational product injection on or before Visit 12 (Week 24), or Day 169 if Visit 12 (Week 24) is not available. Patients with both baseline and Week 24 (LOCF) measurements are included. | | |

**Table 16 Number (%) of patients with >=5 % weight loss from baseline to week 24 - mITT population**

| **Weight loss** | **Placebo (N=284)** | **Lixisenatide (N=564)** |
|---|---|---|
| Number | 278 | 554 |
| ≥5% | 20 (7.2%) | 80 (14.4%) |
| <5% | 258 (92.8%) | 474 (85.6%) |

| | | |
|---|---|---|
| Note: The analysis included measurements obtained before the introduction of rescue medication and up to 3 days after the last dose of the double-blind investigational product injection on or before Visit 12 (Week 24), or Day 169 if Visit 12 (Week 24) is not available. Patients with both baseline and Week 24 (LOCF) measurements are included. | | |

**Table 17 Mean change in HOMA-β from baseline to week 24 in selected sites - mITT population**

| **HOMA-β** | **Placebo (N=284)** | **Lixisenatide (N=564)** |
|---|---|---|
| Baseline | | |
| Number | 116 | 241 |
| Mean (SD) | 35.07 (40.46) | 34.57 (77.41) |
| Median | 23.17 | 22.00 |
| Min : Max | 2.3 : 282.6 | 3.2: 1036.0 |
| | | |
| Week 24 (LOCF) | | |
| Number | 116 | 241 |
| Mean (SD) | 38.12 (60.72) | 39.34 (42.88) |
| Median | 22.41 | 27.50 |
| Min : Max | 4.2 : 585.6 | 2.4 : 468.2 |
| | | |
| Change from baseline to week 24 (LOCF) | | |
| Number | 116 | 241 |
| Mean (SD) | 3.05 (54.57) | 4.76 (83.61) |
| Median | -0.33 | 4.37 |
| Min : Max | -112.1 : 528.2 | -1023.0 : 434.6 |
| LS Mean (SE) ^{a} | 6.63 (5.663) | 4.83 (4.686) |
| | | |
| LS Mean difference (SE) vs. Placebo ^{a} | - | -1.80 (5.400) |
| 95% CI | - | (-12.424 to 8.819) |
| p-value | | 0.7387 |

| | | |
|---|---|---|
| LOCF = Last observation carry forward. ^{a} Analysis of covariance (ANCOVA) model with treatment groups (lixisenatide and placebo), randomization strata of screening HbA1c (<8.0, ≥8.0%), metformin use at screening (Yes, No), and country as fixed effects and baseline HOMA-β value as a covariate. Note: The analysis included measurements obtained before the introduction of rescue medication and up to the date of the last dose of the double-blind investigational product injection on or before Visit 12 (Week 24), or Day 169 if Visit 12 (Week 24) is not available. Patients with both baseline and Week 24 (LOCF) measurements are included. | | |

**Table 18 Number (%) of patients requiring rescue therapy during the 24-week treatment period - miTT population**

| **Requiring rescue therapy** | **Placebo (N=284)** | **Lixisenatide (N=564)** |
|---|---|---|
| Number | 284 | 564 |
| Yes | 34 (12.0%) | 20 (3.5%) |
| No | 250 (88.0%) | 544 (96.5%) |
| p-value vs. placebo^{a} | - | <0.0001 |

| | | |
|---|---|---|
| ^{a} Cochran-Mantel-Haenszel (CMH) method stratified by randomization strata of screening HbA1c (<8.0 or ≥8.0 %) and metformin use at screening (Yes, No). | | |

**Table 19 Mean change in glucose excursion (mmol/L) from baseline to week 24 in selected sites - mITT population**

| **Glucose excursion (mmol/L)** | **Placebo (N=284)** | **Lixisenatide (N=564)** |
|---|---|---|
| Baseline | | |
| Number | 120 | 249 |
| Mean (SD) | 7.04 (4.01) | 6.93(3.79) |
| Median | 7.10 | 6.70 |
| Min : Max | -12.2: 15.2 | -10.7: 17.9 |
| | | |
| Week 24 (LOCF) | | |
| Number | 120 | 249 |
| Mean (SD) | 7.61 (3.30) | 1.94(4.18) |
| Median | 7.43 | 1.60 |
| Min : Max | -0.5: 16.6 | -6.7: 14.9 |
| | | |
| Change from baseline to week 24 (LOCF) | | |
| Number | 120 | 249 |
| Mean (SD) | 0.57 (3.77) | -4.99 (5.04) |
| Median | 0.78 | -4.90 |
| Min : Max | -9.8: 19.7 | -19.1 : 12.0 |
| LS Mean (SE) ^{a} | 0.35 (0.432) | -5.22 (0.360) |
| | | |
| LS Mean difference (SE) vs. Placebo ^{a} | - | -5.57 (0.420) |
| 95% CI | - | (-6.397 to -4.744) |

| | | |
|---|---|---|
| LOCF = Last observation carry forward. Glucose excursion = 2-hour post-prandial plasma glucose - plasma glucose 30 minutes prior to the meal test before study drug administration. ^{a} Analysis of covariance (ANCOVA) model with treatment groups (lixisenatide and placebo), randomization strata of screening HbA1c (<8.0, ≥8.0%), randomization strata of metformin use at screening (Yes, No), and country as fixed effects and baseline glucose excursion value as a covariate. The analysis included measurements obtained before the introduction of rescue medication and up to the date of the last dose of the double-blind investigational product injection on or before Visit 12 (week 24), or Day 169 if Visit 12 (Week 24) is not available. Patients with both baseline and Week 24 (LOCF) measurements are included.) | | |

### 6.3 Safety

An overview of the adverse events observed during the on-treatment period for the whole study is provided in Table 20. The percentage of patients with treatment emergent adverse events (TEAEs) was 81.5% in the lixisenatide group compared with 75.8% in the placebo group. Two patients (in the lixisenatide group) had TEAEs leading to death. Ninety three serious TEAEs occurred during the on-treatment period for the whole study with a slightly lower incidence rate in the lixisenatide group (10.1%) compared to the placebo group (12.3%). The percentage of patients with TEAEs leading to treatment discontinuation was higher in the lixisenatide group compared to the placebo group (12.4% in the lixisenatide group compared with 7.7% in the placebo group). Tables 21, 22, and 23 summarize TEAEs leading to death, serious TEAEs, and TEAEs leading to treatment discontinuation by primary SOC, HLGT, HLT and PT, respectively. The most common TEAE leading to treatment discontinuation was nausea in the lixisenatide group (24 patients [4.2%]). The corresponding number of patients (%) in the placebo group was 1 (0.4%).

Table 33 in the appendix presents the incidences of TEAEs during the on-treatment period for the whole study occurring in at least 1% of patients in any treatment group. Nausea was the most frequently reported TEAE in the lixisenatide group (161 patients [28.0%]) versus 25 placebo-treated patients (8.8%). The second most frequently reported TEAE in the lixisenatide group was hypoglycemia (141 patients [24.6%] for lixisenatide versus 55 patients [19.3%] for placebo) followed by nasopharyngitis (91 patients [15.9%] for lixisenatide versus 58 [20.4%] for placebo), diarrhoea (71 patients [12.4%] for lixisenatide versus 27 [9.5%] for placebo), vomiting (61 patients [10.6%] for lixisenatide versus 15 [5.3%] for placebo), and dizziness (60 patients [10.5%] for lixisenatide versus 18 [6.3%] for placebo).

**Table 20 - Overview of adverse event profile: treatment emergent adverse events during the overall treatment period - Safety population**

| | **Placebo (N=285)** | **Lixisenatide (N=574)** |
|---|---|---|
| Patients with any TEAE | 216 (75.8%) | 468 (81.5%) |
| Patients with any serious TEAE | 35 (12.3%) | 58 (10.1%) |
| Patients with any TEAE leading to death | 0 | 2 (0.3%) |
| Patients with any TEAE leading to permanent treatment discontinuation | 22 (7.7%) | 71 (12.4%) |

| | | |
|---|---|---|
| TEAE: Treatment Emergent Adverse Event n (%) = number and percentage of patients with at least one adverse event | | |

**Table 21 - Number (%) of patients experiencing TEAE(s) leading to death during the overall treatment period by primary SOC, HLGT, HLT, and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=285)** | **(N=574)** |
| Any class | 0 | 2 (0.3%) |
| | | |
| CARDIAC DISORDERS | 0 | 1 (0.2%) |
| HLGT: Coronary artery disorders | 0 | 1 (0.2%) |
| HLT: Ischaemic coronary artery disorders | 0 | 1 (0.2%) |
| Myocardial infarction | 0 | 1 (0.2%) |
| | | |
| GENERAL DISORDERS AND ADMINISTRATION SITE CONDITIONS | 0 | 1 (0.2%) |
| HLGT: Fatal outcomes | 0 | 1 (0.2%) |
| HLT: Death and sudden death | 0 | 1 (0.2%) |
| Sudden cardiac death | 0 | 1 (0.2%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 13.1. n (%) = number and percentage of patients with at least one TEAE leading to death. Table sorted by SOC internationally agreed order and HLGT, HLT, PT alphabetic order. | | |

**Table 22 - Number (%) of patients experiencing serious TEAE(s) during the overall treatment period presented by primary SOC, HLGT, HLT, and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=285)** | **(N=574)** |
| Any class | 35 (12.3%) | 58(10.1%) |
| | | |
| INFECTIONS AND INFESTATIONS | 6 (2.1%) | 3 (0.5%) |
| HLGT: Bacterial infectious disorders | 3 (1.1%) | 1 (0.2%) |
| HLT: Bacterial infections NEC | 3 (1.1%) | 0 |
| Arthritis bacterial | 1 (0.4%) | 0 |
| Cellulitis | 2 (0.7%) | 0 |
| HLT: Leptospira infections | 0 | 1 (0.2%) |
| Leptospirosis | 0 | 1 (0.2%) |
| HLGT: Infections - pathogen unspecified | 2 (0.7%) | 2 (0.3%) |
| HLT: Lower respiratory tract and lung infections | 1 (0.4%) | 0 |
| Pneumonia | 1 (0.4%) | 0 |
| HLT: Urinary tract infections | 1 (0.4%) | 2 (0.3%) |
| Pyelonephritis acute | 0 | 1 (0.2%) |
| Urinary tract infection | 1 (0.4%) | 2 (0.3%) |
| HLGT: Viral infectious disorders | 1 (0.4%) | 0 |
| HLT: Herpes viral infections | 1 (0.4%) | 0 |
| Colitis herpes | 1 (0.4%) | 0 |
| NEOPLASMS BENIGN, MALIGNANT AND UNSPECIFIED (INCL CYSTS AND POLYPS) | 4 (1.4%) | 6 (1.0%) |
| HLGT: Breast neoplasms malignant and unspecified (incl nipple) | 2 (0.7%) | 0 |
| HLT: Breast and nipple neoplasms malignant | 2 (0.7%) | 0 |
| Breast cancer | 2 (0.7%) | 0 |
| HLGT: Gastrointestinal neoplasms malignant and unspecified | 0 | 3 (0.5%) |
| HLT: Gastric neoplasms malignant | 0 | 1 (0.2%) |
| Gastric cancer | 0 | 1 (0.2%) |
| HLT: Rectal neoplasms malignant | 0 | 2 (0.3%) |
| Rectal cancer | 0 | 1 (0.2%) |
| Rectosigmoid cancer | 0 | 1 (0.2%) |
| HLGT: Hepatobiliary neoplasms malignant and unspecified | 1 (0.4%) | 0 |
| HLT: Hepatic neoplasms malignant | 1 (0.4%) | 0 |
| Hepatic neoplasm malignant | 1 (0.4%) | 0 |
| HLGT: Nervous system neoplasms benign | 0 | 1 (0.2%) |
| HLT: Nervous system neoplasms benign NEC | 0 | 1 (0.2%) |
| Brain neoplasm benign | 0 | 1 (0.2%) |
| HLGT: Reproductive neoplasms female benign | 1 (0.4%) | 0 |
| HLT: Uterine neoplasms benign | 1 (0.4%) | 0 |
| Uterine leiomyoma | 1 (0.4%) | 0 |
| HLGT: Reproductive neoplasms male malignant and unspecified | 0 | 1 (0.2%) |
| HLT: Prostatic neoplasms malignant | 0 | 1 (0.2%) |
| Prostate cancer | 0 | 1 (0.2%) |
| HLGT: Respiratory and mediastinal neoplasms malignant and unspecified | 0 | 1 (0.2%) |
| HLT: Laryngeal neoplasms malignant | 0 | 1 (0.2%) |
| Glottis carcinoma | 0 | 1 (0.2%) |
| | | |
| IMMUNE SYSTEM DISORDERS | 0 | 2 (0.3%) |
| HLGT: Allergic conditions | 0 | 2 (0.3%) |
| HLT: Allergic conditions NEC | 0 | 1 (0.2%) |
| Allergy to arthropod sting | 0 | 1 (0.2%) |
| HLT: Anaphylactic responses | 0 | 1 (0.2%) |
| Anaphylactic shock | 0 | 1 (0.2%) |
| | | |
| METABOLISM AND NUTRITION DISORDERS | 2 (0.7%) | 1 (0.2%) |
| HLGT: Glucose metabolism disorders (incl diabetes mellitus) | 2 (0.7%) | 1 (0.2%) |
| HLT: Hyperglycaemic conditions NEC | 1 (0.4%) | 0 |
| Hyperglycaemia | 1 (0.4%) | 0 |
| HLT: Hypoglycaemic conditions NEC | 1 (0.4%) | 1 (0.2%) |
| Hypoglycaemia | 1 (0.4%) | 1 (0.2%) |
| | | |
| PSYCHIATRIC DISORDERS | 0 | 2 (0.3%) |
| HLGT: Depressed mood disorders and disturbances | 0 | 1 (0.2%) |
| HLT: Depressive disorders | 0 | 1 (0.2%) |
| Depression | 0 | 1 (0.2%) |
| HLGT: Schizophrenia and other psychotic disorders | 0 | 2 (0.3%) |
| HLT: Schizophrenia NEC | 0 | 2 (0.3%) |
| Schizophrenia simple | 0 | 1 (0.2%) |
| Schizophrenia, paranoid type | 0 | 1 (0.2%) |
| | | |
| NERVOUS SYSTEM DISORDERS | 6 (2.1%) | 6 (1.0%) |
| HLGT: Central nervous system vascular disorders | 3 (1.1%) | 5 (0.9%) |
| HLT: Central nervous system haemorrhages and cerebrovascular accidents | 2 (0.7%) | 3 (0.5%) |
| Cerebral infarction | 0 | 2 (0.3%) |
| Ischaemic stroke | 1 (0.4%) | 1 (0.2%) |
| Lacunar infarction | 1 (0.4%) | 0 |
| HLT: Transient cerebrovascular events | 1 (0.4%) | 2 (0.3%) |
| Transient ischaemic attack | 1 (0.4%) | 2 (0.3%) |
| HLGT: Cranial nerve disorders (excl neoplasms) | 1 (0.4%) | 1 (0.2%) |
| HLT: Facial cranial nerve disorders | 1 (0.4%) | 1 (0.2%) |
| Vilth nerve paralysis | 1 (0.4%) | 1 (0.2%) |
| HLGT: Movement disorders (incl parkinsonism) | 1 (0.4%) | 0 |
| HLT: Tremor (excl congenital) | 1 (0.4%) | 0 |
| Essential tremor | 1 (0.4%) | 0 |
| HLGT: Neurological disorders NEC | 1 (0.4%) | 0 |
| HLT: Neurological signs and symptoms NEC | 1 (0.4%) | 0 |
| Dizziness | 1 (0.4%) | 0 |
| | | |
| EYE DISORDERS | 0 | 3 (0.5%) |
| HLGT: Anterior eye structural change, deposit and degeneration | 0 | 2 (0.3%) |
| HLT: Cataract conditions | 0 | 2 (0.3%) |
| Cataract | 0 | 1 (0.2%) |
| Cataract nuclear | 0 | 1 (0.2%) |
| HLGT: Retina, choroid and vitreous haemorrhages and vascular disorders | 0 | 1 (0.2%) |
| HLT: Choroid and vitreous haemorrhages and vascular disorders | 0 | 1 (0.2%) |
| Vitreous haemorrhage | 0 | 1 (0.2%) |
| | | |
| EAR AND LABYRINTH DISORDERS | 0 | 2 (0.3%) |
| HLGT: Hearing disorders | 0 | 2 (0.3%) |
| HLT: Hearing losses | 0 | 2 (0.3%) |
| Sudden hearing loss | 0 | 2 (0.3%) |
| | | |
| CARDIAC DISORDERS | 5 (1.8%) | 12 (2.1%) |
| HLGT: Cardiac arrhythmias | 0 | 2 (0.3%) |
| HLT: Cardiac conduction disorders | 0 | 2 (0.3%) |
| Atrioventricular block complete | 0 | 2 (0.3%) |
| HLGT: Cardiac valve disorders | 0 | 1 (0.2%) |
| HLT: Mitral valvular disorders | 0 | 1 (0.2%) |
| Mitral valve incompetence | 0 | 1 (0.2%) |
| HLGT: Coronary artery disorders | 5 (1.8%) | 11 (1.9%) |
| HLT: Coronary artery disorders NEC | 1 (0.4%) | 3 (0.5%) |
| Coronary artery disease | 1 (0.4%) | 2 (0.3%) |
| Coronary artery stenosis | 0 | 1 (0.2%) |
| HLT: Ischaemic coronary artery disorders | 5 (1.8%) | 8 (1.4%) |
| Acute coronary syndrome | 1 (0.4%) | 1 (0.2%) |
| Acute myocardial infarction | 1 (0.4%) | 2 (0.3%) |
| Angina pectoris | 2 (0.7%) | 1 (0.2%) |
| Angina unstable | 1 (0.4%) | 2 (0.3%) |
| Myocardial infarction | 1 (0.4%) | 2 (0.3%) |
| HLGT: Heart failures | 0 | 1 (0.2%) |
| HLT: Heart failures NEC | 0 | 1 (0.2%) |
| Cardiac failure congestive | 0 | 1 (0.2%) |
| | | |
| VASCULAR DISORDERS | 2 (0.7%) | 2 (0.3%) |
| HLGT: Arteriosclerosis, stenosis, vascular insufficiency and necrosis | 0 | 2 (0.3%) |
| HLT: Aortic necrosis and vascular insufficiency | 0 | 1 (0.2%) |
| Aortic stenosis | 0 | 1 (0.2%) |
| HLT: Peripheral vasoconstriction, necrosis and vascular insufficiency | 0 | 1 (0.2%) |
| Peripheral arterial occlusive disease | 0 | 1 (0.2%) |
| HLGT: Embolism and thrombosis | 1 (0.4%) | 0 |
| HLT: Peripheral embolism and thrombosis | 1 (0.4%) | 0 |
| Thrombophlebitis | 1 (0.4%) | 0 |
| HLGT: Vascular hypertensive disorders | 1 (0.4%) | 0 |
| HLT: Vascular hypertensive disorders NEC | 1 (0.4%) | 0 |
| Hypertension | 1 (0.4%) | 0 |
| RESPIRATORY, THORACIC AND MEDIASTINAL DISORDERS | 1 (0.4%) | 1 (0.2%) |
| HLGT: Lower respiratory tract disorders (excl obstruction and infection) | 1 (0.4%) | 0 |
| HLT: Parenchymal lung disorders NEC | 1 (0.4%) | 0 |
| Interstitial lung disease | 1 (0.4%) | 0 |
| HLGT: Respiratory disorders NEC | 0 | 1 (0.2%) |
| HLT: Lower respiratory tract signs and symptoms | 0 | 1 (0.2%) |
| Hiccups | 0 | 1 (0.2%) |
| GASTROINTESTINAL DISORDERS | 2 (0.7%) | 9 (1.6%) |
| HLGT: Abdominal hernias and other abdominal wall conditions | 0 | 1 (0.2%) |
| HLT: Diaphragmatic hernias | 0 | 1 (0.2%) |
| Hiatus hernia | 0 | 1 (0.2%) |
| HLGT: Benign neoplasms gastrointestinal | 0 | 1 (0.2%) |
| HLT: Benign neoplasms gastrointestinal (excl oral cavity) | 0 | 1 (0.2%) |
| Colonic polyp | 0 | 1 (0.2%) |
| HLGT: Exocrine pancreas conditions | 1 (0.4%) | 2 (0.3%) |
| HLT: Acute and chronic pancreatitis | 1 (0.4%) | 2 (0.3%) |
| Pancreatitis acute | 1 (0.4%) | 1 (0.2%) |
| Pancreatitis chronic | 0 | 1 (0.2%) |
| HLGT: Gastrointestinal inflammatory conditions | 0 | 3 (0.5%) |
| HLT: Gastritis (excl infective) | 0 | 1 (0.2%) |
| Gastritis | 0 | 1 (0.2%) |
| HLT: Oesophagitis (excl infective) | 0 | 2 (0.3%) |
| Reflux oesophagitis | 0 | 2 (0.3%) |
| HLGT: Gastrointestinal motility and defaecation conditions | 1 (0.4%) | 2 (0.3%) |
| HLT: Diarrhoea (excl infective) | 1 (0.4%) | 1 (0.2%) |
| Diarrhoea | 1 (0.4%) | 1 (0.2%) |
| HLT: Gastrointestinal atonic and hypomotility disorders NEC | 0 | 1 (0.2%) |
| Gastrooesophageal reflux disease | 0 | 1 (0.2%) |
| HLGT: Gastrointestinal vascular conditions | 0 | 1 (0.2%) |
| HLT: Haemorrhoids and gastrointestinal varices (excl oesophageal) | 0 | 1 (0.2%) |
| Haemorrhoids | 0 | 1 (0.2%) |
| | | |
| HEPATOBILIARY DISORDERS | 1 (0.4%) | 4 (0.7%) |
| HLGT: Gallbladder disorders | 0 | 3 (0.5%) |
| HLT: Cholecystitis and cholelithiasis | 0 | 3 (0.5%) |
| Cholecystitis | 0 | 2 (0.3%) |
| Cholelithiasis | 0 | 1 (0.2%) |
| HLGT: Hepatic and hepatobiliary disorders | 1 (0.4%) | 1 (0.2%) |
| HLT: Hepatocellular damage and hepatitis NEC | 1 (0.4%) | 1 (0.2%) |
| Hepatitis | 0 | 1 (0.2%) |
| Hepatitis acute | 1 (0.4%) | 0 |
| | | |
| MUSCULOSKELETAL AND CONNECTIVE TISSUE DISORDERS | 3 (1.1%) | 1 (0.2%) |
| HLGT: Joint disorders | 0 | 1 (0.2%) |
| HLT: Joint related disorders NEC | 0 | 1 (0.2%) |
| Rotator cuff syndrome | 0 | 1 (0.2%) |
| HLGT: Musculoskeletal and connective tissue deformities (incl intervertebral disc disorders) | 2 (0.7%) | 0 |
| HLT: Intervertebral disc disorders NEC | 2 (0.7%) | 0 |
| Intervertebral disc protrusion | 2 (0.7%) | 0 |
| HLGT: Musculoskeletal and connective tissue disorders NEC | 1 (0.4%) | 0 |
| HLT: Musculoskeletal and connective tissue pain and discomfort | 1 (0.4%) | 0 |
| Back pain | 1 (0.4%) | 0 |
| | | |
| RENAL AND URINARY DISORDERS | 2 (0.7%) | 2 (0.3%) |
| HLGT: Renal disorders (excl nephropathies) | 0 | 1 (0.2%) |
| HLT: Renal failure and impairment | 0 | 1 (0.2%) |
| Renal failure acute | 0 | 1 (0.2%) |
| HLGT: Urinary tract signs and symptoms | 1 (0.4%) | 0 |
| HLT: Bladder and urethral symptoms | 1 (0.4%) | 0 |
| Urinary retention | 1 (0.4%) | 0 |
| HLGT: Urolithiases | 1 (0.4%) | 1 (0.2%) |
| HLT: Urinary tract lithiasis (excl renal) | 1 (0.4%) | 1 (0.2%) |
| Calculus ureteric | 0 | 1 (0.2%) |
| Calculus urinary | 1 (0.4%) | 0 |
| | | |
| CONGENITAL, FAMILIAL AND GENETIC DISORDERS | 1 (0.4%) | 0 |
| HLGT: Gastrointestinal tract disorders congenital | 1 (0.4%) | 0 |
| HLT: Intestinal disorders congenital | 1 (0.4%) | 0 |
| Adenomatous polyposis coli | 1 (0.4%) | 0 |
| GENERAL DISORDERS AND ADMINISTRATION SITE CONDITIONS | 2 (0.7%) | 2 (0.3%) |
| HLGT: Body temperature conditions | 1 (0.4%) | 0 |
| HLT: Febrile disorders | 1 (0.4%) | 0 |
| Pyrexia | 1 (0.4%) | 0 |
| HLGT: Fatal outcomes | 0 | 1 (0.2%) |
| HLT: Death and sudden death | 0 | 1 (0.2%) |
| Sudden cardiac death | 0 | 1 (0.2%) |
| HLGT: General system disorders NEC | 1 (0.4%) | 1 (0.2%) |
| HLT: Pain and discomfort NEC | 1 (0.4%) | 1 (0.2%) |
| Chest pain | 1 (0.4%) | 1 (0.2%) |
| INVESTIGATIONS | 0 | 1 (0.2%) |
| HLGT: Endocrine investigations (incl sex hormones) | 0 | 1 (0.2%) |
| HLT: Gastrointestinal, pancreatic and APUD hormone analyses | 0 | 1 (0.2%) |
| Blood calcitonin increased | 0 | 1 (0.2%) |
| INJURY, POISONING AND PROCEDURAL COMPLICATIONS | 3 (1.1%) | 9 (1.6%) |
| HLGT: Bone and joint injuries | 1 (0.4%) | 4 (0.7%) |
| HLT: Lower limb fractures and dislocations | 1 (0.4%) | 3 (0.5%) |
| Ankle fracture | 1 (0.4%) | 1 (0.2%) |
| Lower limb fracture | 0 | 1 (0.2%) |
| Patella fracture | 0 | 1 (0.2%) |
| HLT: Upper limb fractures and dislocations | 0 | 1 (0.2%) |
| Hand fracture | 0 | 1 (0.2%) |
| HLGT: Injuries NEC | 1 (0.4%) | 5 (0.9%) |
| HLT: Chest and lung injuries NEC | 0 | 1 (0.2%) |
| Pneumothorax traumatic | 0 | 1 (0.2%) |
| HLT: Eye injuries NEC | 0 | 1 (0.2%) |
| Periorbital haematoma | 0 | 1 (0.2%) |
| HLT: Muscle, tendon and ligament injuries | 1 (0.4%) | 0 |
| Muscle injury | 1 (0.4%) | 0 |
| HLT: Non-site specific injuries NEC | 0 | 3 (0.5%) |
| Multiple injuries | 0 | 1 (0.2%) |
| Road traffic accident | 0 | 3 (0.5%) |
| HLT: Skin injuries NEC | 0 | 1 (0.2%) |
| Contusion | 0 | 1 (0.2%) |
| HLGT: Procedural related injuries and complications NEC | 1 (0.4%) | 0 |
| HLT: Cardiac and vascular procedural complications | 1 (0.4%) | 0 |
| In-stent coronary artery restenosis | 1 (0.4%) | 0 |
| SURGICAL AND MEDICAL PROCEDURES | 3 (1.1%) | 5 (0.9%) |
| HLGT: Vascular therapeutic procedures | 3 (1.1%) | 5 (0.9%) |
| HLT: Arterial therapeutic procedures (excl aortic) | 3 (1.1%) | 5 (0.9%) |
| Coronary angioplasty | 0 | 2 (0.3%) |
| Coronary arterial stent insertion | 1 (0.4%) | 0 |
| Percutaneous coronary intervention | 2 (0.7%) | 3 (0.5%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 13.1. n (%) = number and percentage of patients with at least one serious TEAE. Table sorted by SOC internationally agreed order and HLGT, HLT, PT alphabetic order. | | |

**Table 23 - Number (%) of patients experiencing TEAE(s) leading to permanent treatment discontinuation during the overall treatment period by primary SOC, HLGT, HLT, and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=285)** | **(N=574)** |
| Any class | 22 (7.7%) | 71(12.4%) |
| | | |
| INFECTIONS AND INFESTATIONS | 2 (0.7%) | 0 |
| HLGT: Bacterial infectious disorders | 1 (0.4%) | 0 |
| HLT: Bacterial infections NEC | 1 (0.4%) | 0 |
| Arthritis bacterial | 1 (0.4%) | 0 |
| HLGT: Infections - pathogen unspecified | 1 (0.4%) | 0 |
| HLT: Lower respiratory tract and lung infections | 1 (0.4%) | 0 |
| Pneumonia | 1 (0.4%) | 0 |
| | | |
| NEOPLASMS BENIGN, MALIGNANT AND UNSPECIFIED (INCL CYSTS AND POLYPS) | 2 (0.7%) | 1 (0.2%) |
| HLGT: Breast neoplasms malignant and unspecified (incl nipple) | 1 (0.4%) | 0 |
| HLT: Breast and nipple neoplasms malignant | 1 (0.4%) | 0 |
| Breast cancer | 1 (0.4%) | 0 |
| HLGT: Gastrointestinal neoplasms malignant and unspecified | 0 | 1 (0.2%) |
| HLT: Rectal neoplasms malignant | 0 | 1 (0.2%) |
| Rectal cancer | 0 | 1 (0.2%) |
| HLGT: Hepatobiliary neoplasms malignant and unspecified | 1 (0.4%) | 0 |
| HLT: Hepatic neoplasms malignant | 1 (0.4%) | 0 |
| Hepatic neoplasm malignant | 1 (0.4%) | 0 |
| | | |
| BLOOD AND LYMPHATIC SYSTEM DISORDERS | 0 | 1 (0.2%) |
| HLGT: White blood cell disorders | 0 | 1 (0.2%) |
| HLT: Leukopenias NEC | 0 | 1 (0.2%) |
| Leukopenia | 0 | 1 (0.2%) |
| HLT: Neutropenias | 0 | 1 (0.2%) |
| Neutropenia | 0 | 1 (0.2%) |
| | | |
| ENDOCRINE DISORDERS | 0 | 1 (0.2%) |
| HLGT: Thyroid gland disorders | 0 | 1 (0.2%) |
| HLT: Thyroid disorders NEC | 0 | 1 (0.2%) |
| Thyroid C-cell hyperplasia | 0 | 1 (0.2%) |
| METABOLISM AND NUTRITION DISORDERS | 1 (0.4%) | 5 (0.9%) |
| HLGT: Appetite and general nutritional disorders | 1 (0.4%) | 4 (0.7%) |
| HLT: Appetite disorders | 1 (0.4%) | 4 (0.7%) |
| Decreased appetite | 1 (0.4%) | 4 (0.7%) |
| HLGT: Glucose metabolism disorders (incl diabetes mellitus) | 0 | 1 (0.2%) |
| HLT: Hypoglycaemic conditions NEC | 0 | 1 (0.2%) |
| Hypoglycaemia | 0 | 1 (0.2%) |
| | | |
| PSYCHIATRIC DISORDERS | 0 | 3 (0.5%) |
| HLGT: Anxiety disorders and symptoms | 0 | 1 (0.2%) |
| HLT: Anxiety symptoms | 0 | 1 (0.2%) |
| Nervousness | 0 | 1 (0.2%) |
| HLGT: Depressed mood disorders and disturbances | 0 | 1 (0.2%) |
| HLT: Depressive disorders | 0 | 1 (0.2%) |
| Depression | 0 | 1 (0.2%) |
| HLGT: Schizophrenia and other psychotic disorders | 0 | 1 (0.2%) |
| HLT: Schizophrenia NEC | 0 | 1 (0.2%) |
| Schizophrenia, paranoid type | 0 | 1 (0.2%) |
| | | |
| NERVOUS SYSTEM DISORDERS | 2 (0.7%) | 7 (1.2%) |
| HLGT: Central nervous system vascular disorders | 1 (0.4%) | 0 |
| HLT: Central nervous system haemorrhages and cerebrovascular accidents | 1 (0.4%) | 0 |
| Ischaemic stroke | 1 (0.4%) | 0 |
| HLGT: Neurological disorders NEC | 0 | 7 (1.2%) |
| HLT: Neurological signs and symptoms NEC | 0 | 6 (1.0%) |
| Dizziness | 0 | 6 (1.0%) |
| HLT: Sensory abnormalities NEC | 0 | 1 (0.2%) |
| Dysgeusia | 0 | 1 (0.2%) |
| HLGT: Peripheral neuropathies | 1 (0.4%) | 0 |
| HLT: Peripheral neuropathies NEC | 1 (0.4%) | 0 |
| Neuropathy peripheral | 1 (0.4%) | 0 |
| | | |
| EYE DISORDERS | 0 | 3 (0.5%) |
| HLGT: Anterior eye structural change, deposit and degeneration | 0 | 1 (0.2%) |
| HLT: Cataract conditions | 0 | 1 (0.2%) |
| Cataract | 0 | 1 (0.2%) |
| HLGT: Retina, choroid and vitreous haemorrhages and vascular disorders | 0 | 2 (0.3%) |
| HLT: Retinopathies NEC | 0 | 2 (0.3%) |
| Diabetic retinopathy | 0 | 2 (0.3%) |
| | | |
| EAR AND LABYRINTH DISORDERS | 0 | 2 (0.3%) |
| HLGT: Hearing disorders | 0 | 1 (0.2%) |
| HLT: Hearing losses | 0 | 1 (0.2%) |
| Sudden hearing loss | 0 | 1 (0.2%) |
| HLGT: Inner ear and Vlllth cranial nerve disorders | 0 | 1 (0.2%) |
| HLT: Inner ear signs and symptoms | 0 | 1 (0.2%) |
| Vertigo | 0 | 1 (0.2%) |
| CARDIAC DISORDERS | 1 (0.4%) | 5 (0.9%) |
| HLGT: Cardiac disorder signs and symptoms | 0 | 1 (0.2%) |
| HLT: Cardiac signs and symptoms NEC | 0 | 1 (0.2%) |
| Palpitations | 0 | 1 (0.2%) |
| HLGT: Coronary artery disorders | 1 (0.4%) | 3 (0.5%) |
| HLT: Ischaemic coronary artery disorders | 1 (0.4%) | 3 (0.5%) |
| Acute myocardial infarction | 0 | 1 (0.2%) |
| Angina pectoris | 0 | 1 (0.2%) |
| Angina unstable | 1 (0.4%) | 0 |
| Myocardial infarction | 0 | 1 (0.2%) |
| HLGT: Heart failures | 0 | 1 (0.2%) |
| HLT: Heart failures NEC | 0 | 1 (0.2%) |
| Cardiac failure congestive | 0 | 1 (0.2%) |
| | | |
| VASCULAR DISORDERS | 0 | 1 (0.2%) |
| HLGT: Arteriosclerosis, stenosis, vascular insufficiency and necrosis | 0 | 1 (0.2%) |
| HLT: Aortic necrosis and vascular insufficiency | 0 | 1 (0.2%) |
| Aortic stenosis | 0 | 1 (0.2%) |
| RESPIRATORY, THORACIC AND MEDIASTINAL DISORDERS | 1 (0.4%) | 0 |
| HLGT: Lower respiratory tract disorders (excl obstruction and infection) | 1 (0.4%) | 0 |
| HLT: Parenchymal lung disorders NEC | 1 (0.4%) | 0 |
| Interstitial lung disease | 1 (0.4%) | 0 |
| GASTROINTESTINAL DISORDERS | 6 (2.1%) | 37 (6.4%) |
| HLGT: Exocrine pancreas conditions | 2 (0.7%) | 4 (0.7%) |
| HLT: Acute and chronic pancreatitis | 2 (0.7%) | 4 (0.7%) |
| Pancreatitis | 1 (0.4%) | 1 (0.2%) |
| Pancreatitis acute | 1 (0.4%) | 2 (0.3%) |
| Pancreatitis chronic | 0 | 1 (0.2%) |
| HLGT: Gastrointestinal inflammatory conditions | 0 | 1 (0.2%) |
| HLT: Gastritis (excl infective) | 0 | 1 (0.2%) |
| Gastritis | 0 | 1 (0.2%) |
| HLGT: Gastrointestinal motility and defaecation conditions | 1 (0.4%) | 5 (0.9%) |
| HLT: Diarrhoea (excl infective) | 1 (0.4%) | 4 (0.7%) |
| Diarrhoea | 1 (0.4%) | 4 (0.7%) |
| HLT: Gastrointestinal spastic and hypermotility disorders | 0 | 1 (0.2%) |
| Frequent bowel movements | 0 | 1 (0.2%) |
| HLGT: Gastrointestinal signs and symptoms | 3 (1.1%) | 31 (5.4%) |
| HLT: Dyspeptic signs and symptoms | 0 | 3 (0.5%) |
| Dyspepsia | 0 | 3 (0.5%) |
| HLT: Gastrointestinal and abdominal pains (excl oral and throat) | 2 (0.7%) | 1 (0.2%) |
| Abdominal pain | 1 (0.4%) | 1 (0.2%) |
| Abdominal pain lower | 1 (0.4%) | 0 |
| HLT: Nausea and vomiting symptoms | 1 (0.4%) | 28 (4.9%) |
| Nausea | 1 (0.4%) | 24 (4.2%) |
| Vomiting | 0 | 8 (1.4%) |
| HEPATOBILIARY DISORDERS | 0 | 1 (0.2%) |
| HLGT: Hepatic and hepatobiliary disorders | 0 | 1 (0.2%) |
| HLT: Hepatocellular damage and hepatitis NEC | 0 | 1 (0.2%) |
| Hepatitis | 0 | 1 (0.2%) |
| | | |
| SKIN AND SUBCUTANEOUS TISSUE DISORDERS | 1 (0.4%) | 1 (0.2%) |
| HLGT: Epidermal and dermal conditions | 1 (0.4%) | 0 |
| HLT: Bullous conditions | 1 (0.4%) | 0 |
| Blister | 1 (0.4%) | 0 |
| HLGT: Skin appendage conditions | 0 | 1 (0.2%) |
| HLT: Alopecias | 0 | 1 (0.2%) |
| Alopecia | 0 | 1 (0.2%) |
| | | |
| MUSCULOSKELETAL AND CONNECTIVE TISSUE DISORDERS | 3 (1.1%) | 1 (0.2%) |
| HLGT: Joint disorders | 0 | 1 (0.2%) |
| HLT: Arthropathies NEC | 0 | 1 (0.2%) |
| Arthritis | 0 | 1 (0.2%) |
| HLGT: Musculoskeletal and connective tissue disorders NEC | 3 (1.1%) | 0 |
| HLT: Musculoskeletal and connective tissue pain and discomfort | 3 (1.1%) | 0 |
| Back pain | 2 (0.7%) | 0 |
| Musculoskeletal chest pain | 1 (0.4%) | 0 |
| | | |
| RENAL AND URINARY DISORDERS | 1 (0.4%) | |
| HLGT: Renal disorders (excl nephropathies) | 1 (0.4%) | 0 |
| HLT: Renal failure and impairment | 1 (0.4%) | 0 |
| Renal failure acute | 1 (0.4%) | 0 |
| | | |
| PREGNANCY, PUERPERIUM AND PERINATAL CONDITIONS | 0 | 1 (0.2%) |
| HLGT: Pregnancy, labour, delivery and postpartum conditions | 0 | 1 (0.2%) |
| HLT: Normal pregnancy, labour and delivery | 0 | 1 (0.2%) |
| Pregnancy | 0 | 1 (0.2%) |
| | | |
| GENERAL DISORDERS AND ADMINISTRATION SITE CONDITIONS | 0 | 8 (1.4%) |
| HLGT: Administration site reactions | 0 | 3 (0.5%) |
| HLT: Injection site reactions | 0 | 3 (0.5%) |
| Injection site pain | 0 | 1 (0.2%) |
| Injection site pruritus | 0 | 1 (0.2%) |
| Injection site urticaria | 0 | 1 (0.2%) |
| HLGT: Fatal outcomes | 0 | 1 (0.2%) |
| HLT: Death and sudden death | 0 | 1 (0.2%) |
| Sudden cardiac death | 0 | 1 (0.2%) |
| HLGT: General system disorders NEC | 0 | 4 (0.7%) |
| HLT: Asthenic conditions | 0 | 4 (0.7%) |
| Asthenia | 0 | 2 (0.3%) |
| Fatigue | 0 | 2 (0.3%) |
| INVESTIGATIONS | 4 (1.4%) | 6 (1.0%) |
| HLGT: Endocrine investigations (incl sex hormones) | 0 | 2 (0.3%) |
| HLT: Gastrointestinal, pancreatic and APUD hormone analyses | 0 | 2 (0.3%) |
| Blood calcitonin increased | 0 | 2 (0.3%) |
| HLGT: Gastrointestinal investigations | 3 (1.1%) | 2 (0.3%) |
| HLT: Digestive enzymes | 3 (1.1%) | 2 (0.3%) |
| Lipase increased | 0 | 1 (0.2%) |
| Pancreatic enzymes increased | 3 (1.1%) | 1 (0.2%) |
| HLGT: Hepatobiliary investigations | 1 (0.4%) | 0 |
| HLT: Liver function analyses | 1 (0.4%) | 0 |
| Transaminases increased | 1 (0.4%) | 0 |
| HLGT: Metabolic, nutritional and blood gas investigations | 0 | 1 (0.2%) |
| HLT: Carbohydrate tolerance analyses (incl diabetes) | 0 | 1 (0.2%) |
| Blood glucose decreased | 0 | 1 (0.2%) |
| HLGT: Physical examination topics | 0 | 1 (0.2%) |
| HLT: Physical examination procedures | 0 | 1 (0.2%) |
| Weight decreased | 0 | 1 (0.2%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 13.1. n (%) = number and percentage of patients with at least one TEAE leading to permanent treatment discontinuation. Table sorted by SOC internationally agreed order and HLGT, HLT, PT alphabetic order. | | |

One hundred twenty seven (22.1%) lixisenatide-treated patients had 389 symptomatic hypoglycemia events per protocol definition during the on-treatment period for the whole study, whereas 51 (17.9%) placebo-treated patients reported 230 symptomatic hypoglycemia events during the same period (Table 24). Of these patients who had symptomatic hypoglycemia events per protocol definition, 2 lixisenatide-treated patients and 1 placebo-treated patient had the investigator reported terms other than hypoglycemia (hunger and restlessness for the lixisenatide-treated patients and dizziness for the placebo-treated patient) on the AE form for symptomatic hypoglycemia with complementary page. As a result, these 3 patients were included in the respective PT (other than hypoglycemia PT) summary for TEAE summary tables. In addition, 21 additional patients (16 for lixisenatide and 5 for placebo) reported hypoglycemia on the AE form for symptomatic hypoglycemia but their events did not meet the protocol-specified definition: 16 patients (13 for lixisenatide and 3 for placebo) with their associated glucose values ≥60 mg/dL, 2 lixisenatide-treated patients without prompt recovery after oral carbohydrate intake, 2 patients (1 in each group) without their associated glucose values and without counter measurements, and 1 placebo-treated patient without the associated glucose value and without the information on prompt recovery.

Two (0.3%) lixisenatide-treated patients had severe symptomatic hypoglycemia events per protocol definition during the on-treatment period for the whole study, whereas 1 (0.4%) placebo-treated patient reported a severe symptomatic hypoglycemia during the same period (Table 25).

### Symptomatic hypoglycaemia

Symptomatic hypoglycemia is defined as an event with clinical symptoms that are considered to result from a hypoglycemic episode (e.g., sweating, palpitations, hunger, restlessness, anxiety, fatigue, irritability, headache, loss of concentration, somnolence, psychiatric or visual disorders, transient sensory or motor defects, confusion, convulsions, or coma) with an accompanying plasma glucose <60 mg/dL (3.3 mmol/L) or associated with prompt recovery after oral carbohydrate administration if no plasma glucose value is available. Symptoms with an associated plasma glucose ≥60 mg/dL (3.3 mmol/L) should not be reported as a hypoglycemia.

Symptomatic hypoglycemia is to be reported as an adverse event. Additional information should be collected on a specific symptomatic hypoglycemic event complementary form.

### Severe symptomatic hypoglycemia

Severe symptomatic hypoglycemia is defined as an event with clinical symptoms that are considered to result from hypoglycemia in which the patient required the assistance of another person, because the patient could not treat him/herself due to acute neurological impairment directly resulting from the hypoglycemic event, and one of the following:
- The event was associated with a plasma glucose level below 36 mg/dL (2.0 mmol/L).
- If no plasma glucose value is available, then the event was associated with prompt recovery after oral carbohydrate, intravenous glucose, or glucagon administration.

The definition of severe symptomatic hypoglycemia includes all episodes in which neurological impairment was severe enough to prevent self-treatment and which were thus thought to place patients at risk for injury to themselves or others. Note that "requires assistance" means that the patient could not help himself or herself. Someone being kind that assists spontaneously the patient when not necessary does not qualify as "requires assistance."

Severe symptomatic hypoglycemia will be qualified as an SAE only if it fulfills SAE criteria.

**Table 24 - Summary of symptomatic hypoglycemia during the on-treatment period for the whole study - Safety population**

| **Type** | **Placebo (N=285)** | **Lixisenatide (N=574)** |
|---|---|---|
| Total patient years | 410.6 | 829.5 |
| | | |
| Any symptomatic hypoglycemia | | |
| Number of patients with events, n (%) | 51 (17.9%) | 127 (22.1%) |
| Number of patients with events per 100 patient years^{a} | 12.4 | 15.3 |
| | | |
| Blood glucose <60 mg/dL | | |
| Number of patients with events, n (%) | 43 (15.1%) | 111 (19.3%) |
| Number of patients with events per 100 patient years^{a} | 10.5 | 13.4 |
| | | |
| No blood glucose reported | | |
| Number of patients with events, n (%) | 15 (5.3%) | 36 (6.3%) |
| Number of patients with events per 100 patient years^{a} | 3.7 | 4.3 |

| | | |
|---|---|---|
| ^{a} Calculated as (number of patients with events*100 divided by total exposure + 3 days in patient years). Symptomatic hypoglycemia = Symptomatic hypoglycemia as defined per protocol. Note: On-treatment period for the whole study = the time from the first dose of double-blind study medication up to 3 days after the last dose administration. | | |

**Table 25 - Summary of severe symptomatic hypoglycemia during the on-treatment period for the whole study - Safety population**

| **Type** | **Placebo (N=285)** | **Lixisenatide (N=574)** |
|---|---|---|
| Total patient years | 410.6 | 829.5 |
| | | |
| Any severe symptomatic hypoglycemia | | |
| Number of patients with events, n (%) | 1 (0.4%) | 2 (0.3%) |
| Number of patients with events per 100 patient years^{a} | 0.2 | 0.2 |
| | | |
| Blood glucose <36 mg/dL | | |
| Number of patients with events, n (%) | 1 (0.4%) | 0 |
| Number of patients with events per 100 patient years^{a} | 0.2 | 0.0 |
| | | |
| No blood glucose reported | | |
| Number of patients with events, n (%) | 0 | (0.3%) |
| Number of patients with events per 100 patient years^{a} | 0.0 | 0.2 |

| | | |
|---|---|---|
| ^{a} Calculated as (number of patients with events*100 divided by total exposure + 3 days in patient years). Severe symptomatic hypoglycemia = Severe symptomatic hypoglycemia as defined per protocol. Note: On-treatment period for the whole study = the time from the first dose of double-blind study medication up to 3 days after the last dose administration. | | |

Thirty six patients (4.9% for lixisenatide and 2.8% for placebo) experienced injection site reaction AEs (Table 26). The injection site reaction AEs were identified by searching the term "injection site" in either the investigator reported AE PTs or PTs from the ARAC diagnosis during the allergic reaction adjudication. None of the reactions was serious or severe in intensity.

**Table 26 - Number (%) of patients experiencing injection site reactions during the on-treatment period for the whole study- Safety population**

| **Event source** | **Placebo** | **Lixisenatide** |
|---|---|---|
| **Preferred Term** | **(N=285)** | **(N=574)** |
| Any injection site reactions | 8 (2.8%) | 28 (4.9%) |
| | | |
| Investigator reported PTs | 7 (2.5%) | 27 (4.7%) |
| Injection site pain | 5 (1.8%) | 6 (1.0%) |
| Injection site mass | 1 (0.4%) | 0 |
| Injection site reaction | 1 (0.4%) | 2 (0.3%) |
| Injection site erythema | 0 | 5 (0.9%) |
| Injection site haemorrhage | 0 | 2 (0.3%) |
| Injection site hypersensitivity | 0 | 1 (0.2%) |
| Injection site induration | 0 | 2 (0.3%) |
| Injection site nodule | 0 | 1 (0.2%) |
| Injection site pruritus | 0 | 9 (1.6%) |
| Injection site rash | 0 | 1 (0.2%) |
| Injection site swelling | 0 | 1 (0.2%) |
| Injection site urticaria | 0 | 1 (0.2%) |
| PTs by ARAC diagnosis | 1 (0.4%) | 10 (1.7%) |
| Injection site reaction | 1 (0.4%) | 10 (1.7%) |

| | | |
|---|---|---|
| ARAC = Allergic Reaction Assessment Committee. Note: On-treatment period for the whole study = the time from the first dose of double-blind study medication up to 3 days after the last dose administration. | | |

A total of 44 events were reported as a suspected allergic event by investigators during the on-treatment period for the whole study and sent to ARAC for adjudication. Of these, 13 events from 12 patients (11 [1.9%] lixisenatide-treated patients and 1[0.4%] placebo-treated patient) were adjudicated as an allergic reaction by the ARAC, but only one event (local reaction) from one lixisenatide-treated patient (#764501007) was adjudicated as possibly related to IP (Table 27).

In the lixisenatide treated patients, there are 2 patients with anaphylactic shocks: one anaphylactic shock was due to a bee sting and the other one occurred during a surgery (after intravenous application of an antibiotic drug).

**Table 27 - Number (%) of patients with events adjudicated as allergic reaction by ARAC during the on-treatment period of the whole study - Safety population**

| **Relationship** to **study treatment (by ARAC)** | **MedDRA coded term (PT) for ARAC diagnosis** | **ARAC diagnosis** | **Placebo (N=285)** | **Lixisenatide (N=574)** |
|---|---|---|---|---|
| All | Events adjudicated as an allergic reaction by ARAC | | 1 (0.4%) | 11 (1.9%) |
| | | ANAPHYLACTIC | | |
| | Anaphylactic shock | SHOCK | 0 | 2 (0.3%) |
| | Angioedema | ANGIOEDEMA | 0 | 1 (0.2%) |
| | | ALLERGIC | | |
| | Conjunctivitis allergic | CONJUNCTIVITIS | 0 | 2 (0.3%) |
| | | CONTACT | | |
| | Dermatitis contact | DERMATITIS | 0 | 1 (0.2%) |
| | | INCREASING | | |
| | | LARGE LOCAL | | |
| | Local reaction | REACTION | 0 | 1 (0.2%) |
| | | GENERALIZED | | |
| | Pruritus generalised | PRURITUS | 0 | 1 (0.2%) |
| | | ALLERGIC | | |
| | Rhinitis allergic | RHINITIS | 1 (0.4%) | 2 (0.3%) |
| | | URTICARIA | | |
| | Urticaria | (HIVES) | 0 | 2 (0.3%) |
| Possibly related to IP | Events adjudicated as an allergic reaction by ARAC | | 0 | 1 (0.2%) |
| | Local reaction | INCREASING LARGE LOCAL REACTION | 0 | 1 (0.2%) |
| Not related to IP | Events adjudicated as an allergic reaction by ARAC | | 1 (0.4%) | 10 (1.7%) |
| | Anaphylactic shock | ANAPHYLACTIC SHOCK | 0 | 2 (0.3%) |
| | Angioedema | ANGIOEDEMA | 0 | 1 (0.2%) |
| | Conjunctivitis allergic | ALLERGIC CONJUNCTIVITIS | 0 | 2 (0.3%) |
| | Dermatitis contact. | CONTACT DERMATITIS | 0 | 1 (0.2%) |
| | Pruritus generalised | GENERALIZED PRURITUS | 0 | 1 (0.2%) |
| | Rhinitis allergic | ALLERGIC RHINITIS | 1 (0.4%) | 2 (0.3%) |
| | Urticaria | URTICARIA (HIVES) | 0 | 2 (0.3%) |

| | | | | |
|---|---|---|---|---|
| ARAC = Allergic Reaction Assessment Committee. IP=Investigational Product. Note: On-treatment period for the whole study = the time from the first dose of double-blind study medication up to 3 days after the last dose administration. | | | | |

Per protocol, any confirmed increase in amylase and/or lipase above twice the upper limit of normal range (ULN) was to be monitored and documented on a specific form: "adverse event form for suspected pancreatitis". During the on-treatment period for the whole study, this form was completed for 9 (3.2%) placebo-treated patients and 15 (2.6%) lixisenatide-treated patients (Table 28). Among these 15 patients, the PT was acute pancreatitis for two patients and pancreatitis for two other patients:

Of the two acute pancreatitis events in the lixisenatide group, one was serious with a lipase value >3 ULN as well as amylase value >3 ULN during the on-treatment period (patient #100505015, amylase 12.5 ULN and lipase 67.1 ULN on Day 364) and the other (patient #840527002) was not serious with a lipase value >3 ULN and an amylase value >2 ULN.
- Patient #100505015 developed epigastric pain and constipation 51 weeks after the first dose of IP without a history of alcohol abuse, hyperlipidemia, blunt abdominal trauma or gallbladder diseases. A pylorospasm was diagnosed and treated with isosorbide dinitrate and omeprazole. One week later, a toxic acute pancreatitis of moderate intensity was detected. The IP was permanently discontinued the day after, as the event was considered as related to IP. The patient fully recovered 8 days later with normal lipase and amylase values.
- Patient #840527002 without a history of alcohol abuse or gallbladder diseases developed abdominal pain 428 days after first dose of IP. The event was assessed as related to IP and IP was discontinued. An analgetic treatment with Acetaminophen and Oxycodone succeeded in a complete pain relief on the same day. The event was resolved 1,5 months later.

None of the two pancreatits events in the lixisenatide group was serious. One of the patients had a lipase value >3 ULN and the other had a lipase value >2 ULN and an amylase value >2 ULN.
- For Patient #840506006 without a history of alcohol abuse or gallbladder diseases a "suspected pancreatitis due to increased lipase from gallstones" reported on day 15 after first dose of IP. Small scattered gall stones have been found. The investigator assessed the event as not related to IP, but 2 days later, IP was permanently discontinued. Without corrective treatment, the event resolved 2 months later.
- For Patient #356509010 without a history of alcohol abuse or gallbladder diseases had repeated elevations of Amylase and Lipase.1,5 years after first dose of IP an event "elevated Lipase and amylase - suspected pancreatitis" was reported. Magnetic resonance cholangiopancreatography revealed a normal result. IP was continued per protocol until end of the study. One month after end of treatment with IP, the event was resolved without corrective treatment and Lipase returned to normal, whereas Amylase was still slightly elevated (148 IU/L (28-120)).

Patients who had at least one value of lipase or amylase ≥ 3 ULN during the on-treatment period are summarized in Table 29. A total of 26 patients (17 [3.0%] patients in the lixisenatide group and 9 [3.2%] in the placebo group) with elevated lipase (≥ 3ULN) was observed. Three (0.5%) patients in the lixisenatide group had elevated amylase (≥ 3ULN), and none in the placebo group.

**Table 28 - Number (%) of patients with a specific adverse event form for suspected pancreatitis completed during the on-treatment period for the whole study - Safety population**

| **Preferred Term** | **Placebo (N=285)** | **Lixisenatide (N=574)** |
|---|---|---|
| Any | 9 (3.2%) | 15 (2.6%) |
| | | |
| Blood amylase increased | 1 (0.4%) | 1 (0.2%) |
| Lipase increased | 4 (1.4%) | 8 (1.4%) |
| Pancreatic enzymes increased | 3 (1.1%) | 1 (0.2%) |
| Pancreatitis | 1 (0.4%) | 2 (0.3%) |
| Pancreatitis acute | 1 (0.4%) | 2 (0.3%) |
| Pancreatitis chronic | 0 | 2 (0.3%) |

| | | |
|---|---|---|
| n (%) = number and percentage of patients with any cases reported on the AE form for suspected pancreatitis along with complementary form. Note: On-treatment period for the whole study = the time from the first dose of double-blind study medication up to 3 days after the last dose administration. | | |

**Table 29 - Pancreatic enzymes: Number (%) of patients with at least one post-baseline PCSA during the on-treatment period for the whole study according to baseline status - Safety population**

| **Laboratory parameter** | | |
|---|---|---|
| **Baseline** | **Placebo** | **Lixisenatide** |
| **By PCSA criteria n/N1 (%)** | **(N=285)** | **(N=574)** |
| Lipase (IU/L) | | |
| Total* | | |
| ≥ 3 ULN | 9/284 (3.2%) | 17/566 (3.0%) |
| Normal/Missing | | |
| ≥ 3 ULN | 8/282 (2.8%) | 17/565 (3.0%) |
| | | |
| Amylase (IU/L) | | |
| Total* | | |
| ≥ 3 ULN | 0/284 | 3/566 (0.5%) |
| Normal/Missing | | |
| ≥ 3 ULN | 0/284 | 3/566 (0.5%) |

| | | |
|---|---|---|
| PCSA: Potentially Clinically Significant Abnormalities, ULN= Upper limit of normal. *Regardless of baseline. Note: On-treatment period for the whole study = the time from the first dose of double-blind study medication up to 3 days after the last dose administration. The number (n) represents the subset of the total number of patients who met the criterion in question at least once. The denominator (/N1) for each parameter within a treatment. group is the number of patients for the treatment group who had that parameter assessed post-baseline by baseline PCSA status. Only the worsening of the worst case for each patient is presented by baseline status. | | |

Per protocol, any calcitonin value confirmed as being ≥ 20 pg/mL, was to be monitored and reported on a specific adverse event form for "increased calcitonin ≥ 20 pg/mL". During the on-treatment period for whole study, this form was completed for 5 (1.8%) placebo-treated patients and 8 (1.4%) lixisenatide-treated patients (Table 30). For 7 of these 8 patients, the PT was blood calcitonin increased: 2 patients had a calcitonin value ≥50 ng/L, 4 had a calcitonin value ≥20 ng/L but < 50 ng/L and one had a value <20 ng/L (the specific form was unnecessarily completed for this patient). For the eighth patient, the event with PT thyroid C-cell hyperplasia was reported:
- This patient was found to have moderately elevated calcitonin values (23.89 ng/L / 2.8 ULN) on the day of the first dose IP. IP was permanently discontinued 2 months after first dose of IP. Further investigation revealed a pathological pentagatsrin test, followed by a total thyreoidectomy (histology: multifocal hyperplasia of C-cells on both sides without signs for malignancy). Afterwards, calcitonin value returned to normal (<0.59 ng/L) and did not change any more during the one year follow-up after surgery.

**Table 30 Number (%) of patients with increased calcitonin during the on-treatment period for the whole study - Safety population**

| **Preferred Term** | **Placebo (N=285)** | **Lixisenatide (N=574)** |
|---|---|---|
| Any | 5 (1.8%) | 8 (1.4%) |
| | | |
| Blood calcitonin increased | 5 (1.8%) | 7 (1.2%) |
| Thyroid C-cell hyperplasia | 0 | 1 (0.2%) |

| | | |
|---|---|---|
| n (%) = number and percentage of patients with any cases reported on the AE form for increased calcitonin ≥ 20ng/L. Note: On-treatment period for the whole study = the time from the first dose of double-blind study medication up to 3 days after the last dose administration. | | |

Ten (2.0%) patients in the lixisenatide group and 7 (2.8%) patients in the placebo group had at least one value of calcitonin ≥20 ng/L during the on-treatment period (Table 31). It should be pointed out that calcitonin measurements were added in a protocol amendment after most patients were already randomized in this study. Therefore, baseline calcitonin values are missing for most patients.

**Table 31 - Serum calcitonin - Number (%) of patients by pre-defined categories during the on-treatment period of the whole study according to baseline category - Safety population**

| **Laboratory criteria** | **Placebo (N=285)** | **Lixisenatide (N=574)** |
|---|---|---|
| **Baseline status** | | |
| **Post-baseline** | | |
| Calcitonin (ng/L) | | |
| Total* | | |
| ≥ULN | 221/253 (87.4%) | 438/507 (86.4%) |
| >ULN - <20 ng/L | 25/253 (9.9%) | 59/507 (11.6%) |
| ≥20 ng/L - <50 ng/L | 7/253 (2.8%) | 8/507 (1.6%) |
| ≥50 ng/L | 0/253 | 2/507 (0.4%) |
| | | |
| Missing | | |
| ≤ULN | 208/238 (87.4%) | 406/469 (86.6%) |
| >ULN - <20 ng/L | 24/238 (10.1%) | 55/469 (11.7%) |
| ≥20 ng/L - <50 ng/L | 6/238 (2.5%) | 6/469 (1.3%) |
| ≥50 ng/L | 0/238 | 2/469 (0.4%) |
| | | |
| ≤ULN | | |
| ≤ULN | 11/11 (100%) | 32/34 (94.1%) |
| >ULN - <20 ng/L | 0/11 | 2/34 (5.9%) |
| ≥20 ng/L - <50 ng/L | 0/11 | 0/34 |
| ≥50 ng/L | 0/11 | 0/34 |
| | | |
| >ULN - <20 ng/L | | |
| ≤ULN | 1/3 (33.3%) | 0/3 |
| >ULN - <20 ng/L | 1/3 (33.3%) | 2/3 (66.7%) |
| ≥20 ng/L - <50 ng/L | 1/3 (33.3%) | 1/3 (33.3%) |
| ≥50 ng/L | 0/3 | 0/3 |
| ≥20 ng/L - <50 ng/L | | |
| ≤ULN | 1/1 (100%) | 0/1 |
| >ULN - <20 ng/L | 0/1 | 0/1 |
| ≥20 ng/L - <50 ng/L | 0/1 | 1/1 (100%) |
| ≥50 ng/L | 0/1 | 0/1 |
| | | |
| ≥50 ng/L | | |
| ≤ULN | 0/0 | 0/0 |
| >ULN - <20 ng/L | 0/0 | 0/0 |
| ≥20 ng/L - <50 ng/L | 0/0 | 0/0 |
| ≥50 ng/L | 0/0 | 0/0 |

| | | |
|---|---|---|
| ULN= Upper limit of normal *Regardless of baseline. Note: On-treatment period for the whole study = the time from the first dose of double-blind study medication up to 3 days after the last dose administration. The numerator represents the number of patients who were in the pre-specified categories at post-baseline in each baseline category. The denominator for each parameter within a treatment group is the number of patients for the treatment group who had that parameter assessed post-baseline by baseline status. A patient is counted only in the worst category. | | |

### 7 APPENDIX

**Table 32 - Mean change in HbA1c (%) from baseline by visit - mITT population**

| | **Observed data** | | | | | | | **Change from baseline** | | | | | | |
|---|---|---|---|---|---|---|---|---|---|---|---|---|---|---|
| **Treatment** | | | | | | | | | | | | | | |

| **Time point** | **N** | **Mean** | **SD** | **SE** | **Median** | **Min** | **Max** | **N** | **Mean** | **SD** | **SE** | **Median** | **Min** | **Max** |
|---|---|---|---|---|---|---|---|---|---|---|---|---|---|---|
| Placebo (N=284) | | | | | | | | | | | | | | |
| Screening | 284 | 8.33 | 0.81 | 0.048 | 8.30 | 7.0 | 10.0 | | | | | | | |
| Baseline | 284 | 8.22 | 0.84 | 0.050 | 8.20 | 6.4 | 10.8 | | | | | | | |
| Week 8 | 259 | 8.01 | 1.01 | 0.063 | 7.90 | 5.2 | 10.9 | 259 | -0.20 | 0.69 | 0.043 | -0.10 | -2.8 | 1.8 |
| Week 12 | 257 | 8.02 | 1.02 | 0.063 | 7.90 | 5.7 | 11.5 | 257 | -0.16 | 0.76 | 0.047 | -0.10 | -2.3 | 1.8 |
| Week 24 | 212 | 7.93 | 1.05 | 0.072 | 7.80 | 6.0 | 10.9 | 212 | -0.19 | 0.84 | 0.058 | -0.20 | -2.4 | 3.1 |
| Week 24 (LOCF) | 274 | 8.10 | 1.11 | 0.067 | 7.90 | 6.0 | 12.2 | 274 | -0.12 | 0.82 | 0.049 | -0.10 | -2.4 | 3..1 |
| Week 36 | 182 | 7.85 | 0.96 | 0.071 | 7.80 | 5.8 | 10.8 | 182 . | -0.20 | 0.88 | 0.065 | -0.30 | -2.2 | 4.1 |
| Week 44 | 167 | 7.84 | 0.98 | 0.076 | 7.70 | 5.1 | 11.5 | 167 | -0.20 | 0.93 | 0.072 | -0.20 | -2.7 | 4.8 |
| Week 52 | 147 | 7.76 | 0.94 | 0.078 | 7.60 | 6.0 | 11.0 | 147 | -0.23 | 0.91 | 0.075 | -0.30 | -2.4 | 4.0 |
| Week 60 | 128 | 7.72 | 0.91 | 0.081 | 7.60 | 5.7 | 10.7 | 128 | -0.29 | 0.85 | 0.075 | -0.25 | -2.4 | 2.7 |
| Week 68 | 120 | 7.67 | 0.92 | 0.084 | 7.60 | 5.5 | 11.1 | 120 | -0.32 | 0.87 | 0.080 | -0.40 | -2.1 | 3.0 |
| Week 76 | 118 | 7.67 | 1.03 | 0.095 | | 5.8 | 11.7 | 118 | -0.30 | 1.05 | 0.097 | -0.40 | -2.4 | 5.0 |
| Week 84 | 95 | 7.67 | 0.99 | 0.102 | 7.60 | 5.8 | 11.6 | 95 | -0.32 | 1.07 | 0.110 | -0.40 | -1.9 | 4.9 |
| Week 92 | 66 | 7.62 | 0.85 | 0.104 | 7.50 | 5.9 | 10.2 | 66 | -0.46 | 0.95 | 0.117 | -0.65 | -1.9 | 3.1 |
| Week 100 | 27 | 7.70 | 0.75 | 0.145 | 7.80 | 6.1 | 9.5 | 27 | -0.50 | 0.80 | 0.155 | -0.40 | -1.8 | 1.2 |
| Week 108 | 11 | 8.02 | 1.03 | 0.310 | 7.50 | 7.2 | 10.3 | 11 | -0.14 | 0.86 | 0.259 | 0.00 | -1.3 | 1.3 |
| Week 116 | 1 | 8.30 | NC | NC | 8.30 | 8.3 | 8.3 | 1 | 1.60 | NC | NC | 1.60. | 1.6 | 1.6 |
| Last on-treatment value | 274 | 8.28 | 1.08 | 0.065 | 8.20 | 5.9 | 12.2 | 274 | 0.06 | 0.86 | 0.052 | 0.10 | -1.9 | 3.3 |
| | | | | | | | | | | | | | | |
| Lixisenatide (N=564) | | | | | | | | | | | | | | |
| Screening | 564 | 8.38 | 0.82 | 0.034 | 8.30 | 7.0 | 10.0 | | | | | | | |
| Baseline | 564 | 8.28 | 0.86 | 0.036 | 8.20 | 6.5 | 12.5 | | | | | | | |
| Week 8 | 525 | 7.41 | 0.83 | 0.036 | 7.30 | 5.3 | 10.1 | 525 | -0.86 | 0.67 | 0.029 | -0.80 | -3.3 | 1.5 |
| Week 12 | 514 | 7.31 | 0.88 | 0.039 | 7.20 | 5.0 | 10.4 | 514 | -0.96 | 0.80 | 0.035 | -0.90 | -3.8 | 2.9 |
| Week 24 | 465 | 7.33 | 0.96 | 0.044 | 7.20 | 5.1 | 11.0 | 465 | -0.90 | 0.95 | 0.044 | -0.90 | -3.3 | 3.4 |
| Week 24 (LOCF) | 544 | 7.40 | 1.00 | 0.043 | 7.30 | 5.1 | 12.4 | 544 | -0.88 | 0.93 | 0.040 | -0.90 | -3.3 | 3.4 |
| Week 36 | 408 | 7.32 | 0.92 | 0.045 | 7.30 | 5.4 | 10.6 | 408 | -0.88 | 0.93 | 0.046 | -0.90 | -3.5 | 2.2 |
| Week 44 | 374 | 7.33 | 0.86 | 0.044 | 7.30 | 5.3 | 10.3 | 374 | -0.84 | 0.93 | 0.048 | -0.80 | -3.2 | 1.5 |

| | **Observed data** | | | | | | | **Change from baseline** | | | | | | |
|---|---|---|---|---|---|---|---|---|---|---|---|---|---|---|
| **Treatment** | | | | | | | | | | | | | | |
| **Time point** | **N** | **Mean** | **SD** | **SE** | **Median** | **Min** | **Max** | **N** | **Mean** | **SD** | **SE** | **Median** | **Min** | |
| Week 52 | 350 | 7.32 | 0.91 | 0.049 | 7.20 | 5.3 | 10.7 | 350 | -0.84 | 0.97 | 0.052 | -0.80 | -3.6 | **Max** 2.9 |
| Week 60 | 327 | 7.37 | 0.97 | 0.054 | 7.30 | 5.3 | 12.3 | 327 | -0.76 | 1.00 | 0.056 | -0.80 | -3.3 | 5.3 |
| Week 68 | 308 | 7.41 | 1.02 | 0.058 | 7.30 | 5.2 | 12.0 | 308 | -0.74 | 1.07 | 0.061 | -0.80 | -3.3 | 3.5 |
| Week 76 | 287 | 7.38 | 1.03 | 0.061 | 7.30 | 5.1 | 11.6 | 287 | -0.78 | 1.02 | 0.060 | -0.80 | -3.4 | 3.1 |
| Week 84 | 239 | 7.35 | 1.04 | 0.067 | 7.20 | 5.0 | 11.7 | 239 | -0.82 | 1.05 | 0.068 | -0.90 | -3.6 | 2.8 |
| Week 92 | 139 | 7.37 | 0.97 | 0.082 | 7.20 | 4.6 | 10.2 | 139 | -0.83 | 1.03 | 0.087 | -0.90 | -3.2 | 2.8 |
| Week 100 | 71 | 7.62 | 1.03 | 0.122 | 7.40 | 5.6 | 9.7 | 71 | -0.62 | 1.07 | 0.127 | -0.60 | -2.8 | 2.5 |
| Week 108 | 30 | 7.46 | 1.29 | 0.235 | 7.20 | 5.5 | 10.1 | 30 | -0.61 | 1.18 | 0.215 | -0.85 | -2.5 | 3.1 |
| Week 116 | 7 | 6.99 | 0.77 | 0.290 | 6.90 | 5.9 | 8.1 | 7 | -1.09 | 0.90 | 0.342 | -1.30 | -2.2 | 0.3 |
| Last on-treatment value | 544 | 7.73 | 1.14 | 0.049 | 7.70 | 4.6 | 12.4 | 544 | -0.54 | 1.07 | 0.046 | *-0.60* | -3.6 | 3.2 |

| | | | | | | | | | | | | | | |
|---|---|---|---|---|---|---|---|---|---|---|---|---|---|---|
| LOCF = Last observation carry forward. NC = Not computable. Note: The analysis excluded measurements obtained after the introduction of rescue medication and/or after the treatment cessation plus 3 days. For Week 24 (LOCF), the analysis included measurement obtained up to 3 days after the last dose of the double-blind investigational product injection on or before Visit 12 (Week 24), or Day 169 if Visit 12 (Week 24) is not available. | | | | | | | | | | | | | | |

**Table 33 Number (%) of patients experiencing common TEAE(s) (PT ≥1% in any treatment group) during the overall treatment period presented by primary SOC, HLGT, HLT and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=285)** | **(N=574)** |
| Any class | 216 (75.8%) | 468 (81.5%) |
| | | |
| INFECTIONS AND INFESTATIONS | 121 (42.5%) | 238 (41.5%) |
| HLGT: Bacterial infectious disorders | 7 (2.5%) | 8 (1.4%) |
| HLT: Bacterial infections NEC | 7 (2.5%) | 1 (0.2%) |
| Cellulitis | 4 (1.4%) | 0 |
| HLGT: Fungal infectious disorders | 10 (3.5%) | 12(2.1%) |
| HLT: Fungal infections NEC | 7 (2.5%) | 5 (0.9%) |
| Onychomycosis | 3 (1.1%) | 2 (0.3%) |
| HLGT: Infections - pathogen unspecified | 102 (35.8%) | 198 (34.5%) |
| HLT: Abdominal and gastrointestinal infections | 7 (2.5%) | 17 (3.0%) |
| Gastroenteritis | 6 (2.1%) | 13 (2.3%) |
| HLT: Lower respiratory tract and lung infections | 10 (3.5%) | 29 (5.1%) |
| Bronchitis | 7 (2.5%) | 25 (4.4%) |
| Pneumonia | 3 (1.1%) | 2 (0.3%) |
| HLT: Skin structures and soft tissue infections | 6 (2.1%) | 7 (1.2%) |
| Furuncle | 4 (1.4%) | 3 (0.5%) |
| HLT: Upper respiratory tract infections | 78 (27.4%) | 153 (26.7%) |
| Nasopharyngitis | 58 (20.4%) | 91 (15.9%) |
| Rhinitis | 0 | 6 (1.0%) |
| Sinusitis | 2 (0.7%) | 15 (2.6%) |
| Upper respiratory tract infection | 21 (7.4%) | 43 (7.5%) |
| HLT: Urinary tract infections | 10 (3.5%) | 24 (4.2%) |
| Cystitis | 2 (0.7%) | 10 (1.7%) |
| Urinary tract infection | 8 (2.8%) | 14 (2.4%) |
| HLGT: Viral infectious disorders | 27 (9.5%) | 62 (10.8%) |
| HLT: Influenza viral infections | 12 (4.2%) | 30 (5.2%) |
| Influenza | 11 (3.9%) | 30 (5.2%) |
| HLT: Viral infections NEC | 8 (2.8%) | 28 (4.9%) |
| Gastroenteritis viral | 2 (0.7%) | 6 (1.0%) |
| Viral infection | 3 (1.1%) | 13 (2.3%) |
| | | |
| BLOOD AND LYMPHATIC SYSTEM DISORDERS | 8 (2.8%) | 17 (3.0%) |
| HLGT: Anaemias nonhaemolytic and marrow depression | 7 (2.5%) | 15 (2.6%) |
| HLT: Anaemia deficiencies | 3 (1.1%) | 2 (0.3%) |
| Iron deficiency anaemia | 3 (1.1%) | 2 (0.3%) |
| HLT: Anaemias NEC | 4 (1.4%) | 13 (2.3%) |
| Anaemia | 4 (1.4%) | 11 (1.9%) |
| | | |
| IMMUNE SYSTEM DISORDERS | 3 (1.1%) | 7 (1.2%) |
| HLGT: Allergic conditions | 3 (1.1%) | 7 (1.2%) |
| HLT: Atopic disorders | 3 (1.1%) | 4 (0.7%) |
| Seasonal allergy | 3 (1.1%) | 4 (0.7%) |
| | | |
| METABOLISM AND NUTRITION DISORDERS | 74 (26.0%) | 176 (30.7%) |
| HLGT: Appetite and general nutritional disorders | 11 (3.9%) | 27 (4.7%) |
| HLT: Appetite disorders | 11 (3.9%) | 26 (4.5%) |
| Decreased appetite | 8 (2.8%) | 26 (4.5%) |
| HLGT: Electrolyte and fluid balance conditions | 4 (1.4%) | 3 (0.5%) |
| HLT: Potassium imbalance | 3 (1.1%) | 2 (0.3%) |
| Hyperkalaemia | 3 (1.1%) | 2 (0.3%) |
| HLGT: Glucose metabolism disorders (incl diabetes mellitus) | 59 (20.7%) | 144 (25.1%) |
| HLT: Hyperglycaemic conditions NEC | 3 (1.1%) | 3 (0.5%) |
| Hyperglycaemia | 3 (1.1%) | 3 (0.5%) |
| HLT: Hypoglycaemic conditions NEC | 56 (19.6%) | 141 (24.6%) |
| Hypoglycaemia | 55 (19.3%) | 141 (24.6%) |
| Hypoglycaemia unawareness | 3 (1.1%) | 0 |
| HLGT: Lipid metabolism disorders | 9 (3.2%) | 11 (1.9%) |
| HLT: Elevated triglycerides | 2 (0.7%) | 6 (1.0%) |
| Hypertriglyceridaemia | 2 (0.7%) | 6 (1.0%) |
| HLT: Lipid metabolism and deposit disorders NEC | 3 (1.1%) | 3 (0.5%) |
| Dyslipidaemia | 3 (1.1%) | 3 (0.5%) |
| | | |
| PSYCHIATRIC DISORDERS | 15 (5.3%) | 37 (6.4%) |
| HLGT: Depressed mood disorders and disturbances | 6 (2.1%) | 12 (2.1%) |
| HLT: Depressive disorders | 6 (2.1%) | 11 (1.9%) |
| Depression | 6 (2.1%) | 10 (1.7%) |
| HLGT: Sleep disorders and disturbances | 5 (1.8%) | 16 (2.8%) |
| HLT: Disturbances in initiating and maintaining sleep | 5 (1.8%) | 14 (2.4%) |
| Insomnia | 5 (1.8%) | 14 (2.4%) |
| | | |
| NERVOUS SYSTEM DISORDERS | 60 (21.1%) | 151 (26.3%) |
| HLGT: Headaches | 21 (7.4%) | 48 (8.4%) |
| HLT: Headaches NEC | 21 (7.4%) | 45 (7.8%) |
| Headache | 20 (7.0%) | 44 (7.7%) |
| HLGT: Movement disorders (incl parkinsonism) | 5 (1.8%) | 18 (3.1%) |
| HLT: Tremor (excl congenital) | 4 (1.4%) | 18 (3.1%) |
| Tremor | 3 (1.1%) | 18 (3.1%) |
| HLGT: Neurological disorders NEC | 28 (9.8%) | 82 (14.3%) |
| HLT: Disturbances in consciousness NEC | 1 (0.4%) | 10 (1.7%) |
| Somnolence | 0 | 6 (1.0%) |
| HLT: Neurological signs and symptoms NEC | 19 (6.7%) | 61 (10.6%) |
| Dizziness | 18 (6.3%) | 60 (10.5%) |
| HLT: Sensory abnormalities NEC | 6 (2.1 %) | 16 (2.8%) |
| Hypoaesthesia | 5 (1.8%) | 8 (1.4%) |
| HLGT: Peripheral neuropathies | 11 (3.9%) | 11 (1.9%) |
| HLT: Chronic polyneuropathies | 4 (1.4%) | 6 (1.0%) |
| Diabetic neuropathy | 4 (1.4%) | 6 (1.0%) |
| HLT: Peripheral neuropathies NEC | 6 (2.1 %) | 5 (0.9%) |
| Neuropathy peripheral | 6(2.1%) | 4 (0.7%) |
| | | |
| EYE DISORDERS | 20 (7.0%) | 46 (8.0%) |
| HLGT: Ocular infections, irritations and inflammations | 5 (1.8%) | 11 (1.9%) |
| HLT: Conjunctival infections, irritations and inflammations | 4 (1.4%) | 7 (1.2%) |
| Conjunctivitis | 3 (1.1%) | 6 (1.0%) |
| HLGT: Retina, choroid and vitreous haemorrhages and vascular disorders | 6 (2.1%) | 13 (2.3%) |
| HLT: Retinopathies NEC | 6 (2.1%) | 10 (1.7%) |
| Diabetic retinopathy | 6 (2.1%) | 9 (1.6%) |
| HLGT: Vision disorders | 0 | 10 (1.7%) |
| HLT: Visual disorders NEC | 0 | 9 (1.6%) |
| Vision blurred | 0 | 8 (1.4%) |
| | | |
| EAR AND LABYRINTH DISORDERS | 3 (1.1%) | 24 (4.2%) |
| HLGT: Inner ear and VIIIth cranial nerve disorders | 3 (1.1%) | 18 (3.1%) |
| HLT: Inner ear signs and symptoms | 3 (1.1%) | 17 (3.0%) |
| Vertigo | 1 (0.4%) | 14 (2.4%) |
| | | |
| CARDIAC DISORDERS | 12 (4.2%) | 34 (5.9%) |
| HLGT: Cardiac disorder signs and symptoms | 4 (1.4%) | 10 (1.7%) |
| HLT: Cardiac signs and symptoms NEC | 4 (1.4%) | 10 (1.7%) |
| Palpitations | 4 (1.4%) | 10 (1.7%) |
| HLGT: Coronary artery disorders | 8 (2.8%) | 15 (2.6%) |
| HLT: Ischaemic coronary artery disorders | 8 (2.8%) | 10 (1.7%) |
| Angina pectoris | 5 (1.8%) | 3 (0.5%) |
| | | |
| VASCULAR DISORDERS | 17 (6.0%) | 41 (7.1%) |
| HLGT: Vascular hypertensive disorders | 11 (3.9%) | 26 (4.5%) |
| HLT: Vascular hypertensive disorders NEC | 11 (3.9%) | 25 (4.4%) |
| Hypertension | 11 (3.9%) | 25 (4.4%) |
| | | |
| RESPIRATORY, THORACIC AND MEDIASTINAL DISORDERS | 29 (10.2%) | 56 (9.8%) |
| HLGT: Respiratory disorders NEC | 19 (6.7%) | 37 (6.4%) |
| HLT: Coughing and associated symptoms | 12 (4.2%) | 17 (3.0%) |
| Cough | 12 (4.2%) | 14 (2.4%) |
| HLT: Upper respiratory tract signs and symptoms | 7 (2.5%) | 17 (3.0%) |
| Oropharyngeal pain | 4 (1.4%) | 11 (1.9%) |
| HLGT: Upper respiratory tract disorders (excl infections) | 8 (2.8%) | 27 (4.7%) |
| HLT: Nasal congestion and inflammations | 3 (1.1%) | 14 (2.4%) |
| Nasal congestion | 2 (0.7%) | 7 (1.2%) |
| HLT: Nasal disorders NEC | 1 (0.4%) | 6 (1.0%) |
| Epistaxis | 1 (0.4%) | 6 (1.0%) |
| HLT: Paranasal sinus disorders (excl infections and neoplasms) | 4 (1.4%) | 7 (1.2%) |
| Sinus congestion | 3 (1.1%) | 7 (1.2%) |
| | | |
| GASTROINTESTINAL DISORDERS | 84 (29.5%) | 281 (49.0%) |
| HLGT: Dental and gingival conditions | 16 (5.6%) | 25 (4.4%) |
| HLT: Dental and periodontal infections and inflammations | 7 (2.5%) | 12 (2.1%) |
| Dental caries | 2 (0.7%) | 7 (1.2%) |
| Periodontitis | 5 (1.8%) | 6 (1.0%) |
| HLT: Dental pain and sensation disorders | 6 (2.1%) | 11 (1.9%) |
| Toothache | 6 (2.1%) | 11 (1.9%) |
| HLGT: Gastrointestinal inflammatory conditions | 4 (1.4%) | 28 (4.9%) |
| HLT: Gastritis (excl infective) | 2 (0.7%) | 16 (2.8%) |
| Gastritis | 2 (0.7%) | 16 (2.8%) |
| HLT: Oesophagitis (excl infective) | 1 (0.4%) | 6 (1.0%) |
| Reflux oesophagitis | 1 (0.4%) | 6 (1.0%) |
| HLGT: Gastrointestinal motility and defaecation conditions | 39 (13.7%) | 99 (17.2%) |
| HLT: Diarrhoea (excl infective) | 27 (9.5%) | 71 (12.4%) |
| Diarrhoea | 27 (9.5%) | 71 (12.4%) |
| HLT: Gastrointestinal atonic and hypomotility disorders NEC | 14 (4.9%) | 36 (6.3%) |
| Constipation | 11 (3.9%) | 30 (5.2%) |
| Gastrooesophageal reflux disease | 3 (1.1%) | 6 (1.0%) |
| HLGT: Gastrointestinal signs and symptoms | 52 (18.2%) | 217 (37.8%) |
| HLT: Dyspeptic signs and symptoms | 4 (1.4%) | 34 (5.9%) |
| Dyspepsia | 4 (1.4%) | 34 (5.9%) |
| HLT: Flatulence, bloating and distension | 4 (1.4%) | 23 (4.0%) |
| Abdominal distension | 3 (1.1%) | 21 (3.7%) |
| HLT: Gastrointestinal and abdominal pains (excl oral and throat) | 17 (6.0%) | 38 (6.6%) |
| Abdominal pain | 8 (2.8%) | 22 (3.8%) |
| Abdominal pain upper | 7 (2.5%) | 17 (3.0%) |
| HLT: Gastrointestinal signs and symptoms NEC | 8 (2.8%) | 7 (1.2%) |
| Abdominal discomfort | 8 (2.8%) | 6 (1.0%) |
| HLT: Nausea and vomiting symptoms | 33 (11.6%) | 176 (30.7%) |
| Nausea | 25 (8.8%) | 161 (28.0%) |
| Vomiting | 15 (5.3%) | 61 (10.6%) |
| | | |
| HEPATOBILIARY DISORDERS | 8 (2.8%) | 19 (3.3%) |
| HLGT: Hepatic and hepatobiliary disorders | 8 (2.8%) | 14 (2.4%) |
| HLT: Hepatocellular damage and hepatitis NEC | 8 (2.8%) | 11 (1.9%) |
| Hepatic steatosis | 7 (2.5%) | 9 (1.6%) |
| | | |
| SKIN AND SUBCUTANEOUS TISSUE DISORDERS | 31 (10.9%) | 49 (8.5%) |
| HLGT: Epidermal and dermal conditions | 19 (6.7%) | 26 (4.5%) |
| HLT: Bullous conditions | 3 (1.1%) | 2 (0.3%) |
| Blister | 3 (1.1%) | 2 (0.3%) |
| HLT: Dermatitis and eczema | 5 (1.8%) | 7 (1.2%) |
| Eczema | 4 (1.4%) | 2 (0.3%) |
| HLT: Pruritus NEC | 4 (1.4%) | 9 (1.6%) |
| Pruritus | 3 (1.1%) | 7 (1.2%) |
| HLT: Rashes, eruptions and exanthems NEC | 4 (1.4%) | 5 (0.9%) |
| Rash | 4 (1.4%) | 3 (0.5%) |
| HLGT: Skin appendage conditions | 11 (3.9%) | 17 (3.0%) |
| HLT: Apocrine and eccrine gland disorders | 7 (2.5%) | 15 (2.6%) |
| Hyperhidrosis | 4 (1.4%) | 12(2.1%) |
| HLT: Nail and nail bed conditions (excl infections and infestations) | 3 (1.1%) | 1 (0.2%) |
| Ingrowing nail | 3 (1.1%) | 1 (0.2%) |
| | | |
| MUSCULOSKELETAL AND CONNECTIVE TISSUE DISORDERS | 55 (19.3%) | 125 (21.8%) |
| HLGT: Joint disorders | 25 (8.8%) | 47 (8.2%) |
| HLT: Arthropathies NEC | 5 (1.8%) | 5 (0.9%) |
| Arthritis | 5 (1.8%) | 5 (0.9%) |
| HLT: Joint related disorders NEC | 2 (0.7%) | 12 (2.1%) |
| Periarthritis | 2 (0.7%) | 8 (1.4%) |
| HLT: Joint related signs and symptoms | 11 (3.9%) | 22 (3.8%) |
| Arthralgia | 11 (3.9%) | 20 (3.5%) |
| HLT: Osteoarthropathies | 6 (2.1%) | 11 (1.9%) |
| Osteoarthritis | 4 (1.4%) | 10 (1.7%) |
| HLGT: Muscle disorders | 7 (2.5%) | 24 (4.2%) |
| HLT: Muscle pains | 2(0.7%) | 12 (2.1%) |
| Myalgia | 2 (0.7%) | 10 (1.7%) |
| HLT: Muscle related signs and symptoms NEC | 5 (1.8%) | 9 (1.6%) |
| Muscle spasms | 4 (1.4%) | 9 (1.6%) |
| HLGT: Musculoskeletal and connective tissue deformities (incl intervertebral disc disorders) | 5 (1.8%) | 8 (1.4%) |
| HLT: Intervertebral disc disorders NEC | 4 (1.4%) | 3 (0.5%) |
| Intervertebral disc protrusion | 3 (1.1%) | 1 (0.2%) |
| HLGT: Musculoskeletal and connective tissue disorders NEC | 27 (9.5%) | 63 (11.0%) |
| HLT: Musculoskeletal and connective tissue pain and discomfort | 27 (9.5%) | 61 (10.6%) |
| Back pain | 12 (4.2%) | 36 (6.3%) |
| Flank pain | 3(1.1%) | 3 (0.5%) |
| Musculoskeletal chest pain | 3 (1.1%) | 3 (0.5%) |
| Musculoskeletal pain | 7 (2.5%) | 13 (2.3%) |
| Pain in extremity | 6 (2.1%) | 17 (3.0%) |
| HLGT: Tendon, ligament and cartilage disorders | 7 (2.5%) | 8 (1.4%) |
| HLT: Tendon disorders | 7 (2.5%) | 7 (1.2%) |
| Trigger finger | 5 (1.8%) | 0 |
| | | |
| REPRODUCTIVE SYSTEM AND BREAST DISORDERS | 7 (2.5%) | 14 (2.4%) |
| HLGT: Prostatic disorders (excl infections and inflammations) | | 3 (0.5%) |
| HLT: Prostatic neoplasms and hypertrophy | 5 (1.8%) | 3 (0.5%) |
| Benign prostatic hyperplasia | 5 (1.8%) | 3 (0.5%) |
| | | |
| GENERAL DISORDERS AND ADMINISTRATION SITE CONDITIONS | 36 (12.6%) | 115 (20.0%) |
| HLGT: Administration site reactions | 7 (2.5%) | 27 (4.7%) |
| HLT: Injection site reactions | 7 (2.5%) | 27 (4.7%) |
| Injection site pain | 5 (1.8%) | 6 (1.0%) |
| Injection site pruritus | 0 | 9 (1.6%) |
| HLGT: Body temperature conditions | 6 (2.1%) | 8 (1.4%) |
| HLT: Febrile disorders | 5 (1.8%) | 8 (1.4%) |
| Pyrexia | 5 (1.8%) | 8 (1.4%) |
| HLGT: General system disorders NEC | 29 (10.2%) | 90 (15.7%) |
| HLT: Asthenic conditions | 14 (4.9%) | 48 (8.4%) |
| Asthenia | 7 (2.5%) | 24 (4.2%) |
| Fatigue | 6 (2.1%) | 25 (4.4%) |
| HLT: Feelings and sensations NEC | 4 (1.4%) | 16 (2.8%) |
| Hunger | 3 (1.1%) | 6 (1.0%) |
| HLT: General signs and symptoms NEC | 4 (1.4%) | 9 (1.6%) |
| Influenza like illness | 4 (1.4%) | 8 (1.4%) |
| HLT: Oedema NEC | 8 (2.8%) | 17 (3.0%) |
| Oedema peripheral | 6 (2.1%) | 13 (2.3%) |
| HLT: Pain and discomfort NEC | 4 (1.4%) | 16 (2.8%) |
| Non-cardiac chest pain | 1 (0.4%) | 6 (1.0%) |
| | | |
| INVESTIGATIONS | 33 (11.6%) | 69 (12.0%) |
| HLGT: Endocrine investigations (incl sex hormones) | 5 (1.8%) | 7 (1.2%) |
| HLT: Gastrointestinal, pancreatic and APUD hormone analyses | 5 (1.8%) | 7 (1.2%) |
| Blood calcitonin increased | 5 (1.8%) | 7 (1.2%) |
| HLGT: Gastrointestinal investigations | 9 (3.2%) | 18 (3.1%) |
| HLT: Digestive enzymes | 9 (3.2%) | 18 (3.1%) |
| Blood amylase increased | 2 (0.7%) | 6 (1.0%) |
| Lipase increased | 4 (1.4%) | 14 (2.4%) |
| Pancreatic enzymes increased | 4 (1.4%) | 1 (0.2%) |
| HLGT: Metabolic, nutritional and blood gas investigations | 11 (3.9%) | 31 (5.4%) |
| HLT: Carbohydrate tolerance analyses (incl diabetes) | 11 (3.9%) | 31 (5.4%) |
| Blood glucose decreased | 11 (3.9%) | 30 (5.2%) |
| | | |
| INJURY, POISONING AND PROCEDURAL COMPLICATIONS | 37 (13.0%) | 67 (11.7%) |
| HLGT: Injuries NEC | 30 (10.5%) | 44 (7.7%) |
| HLT: Muscle, tendon and ligament injuries | 4 (1.4%) | 8 (1.4%) |
| Muscle strain | 3 (1.1%) | 4(0.7%) |
| HLT: Non-site specific injuries NEC | 22 (7.7%) | 23 (4.0%) |
| Arthropod bite | 3 (1.1%) | 1 (0.2%) |
| Fall | 12 (4.2%) | 10 (1.7%) |
| Road traffic accident | 2 (0.7%) | 7 (1.2%) |
| HLT: Skin injuries NEC | 6 (2.1%) | 19 (3.3%) |
| Contusion | 5 (1.8%) | 9 (1.6%) |
| Skin laceration | 2 (0.7%) | 6 (1.0%) |
| | | |
| SURGICAL AND MEDICAL PROCEDURES | 5 (1.8%) | 11 (1.9%) |
| HLGT: Vascular therapeutic procedures | 4 (1.4%) | 7 (1.2%) |
| HLT: Arterial therapeutic procedures (excl aortic) | 4 (1.4%) | 7 (1.2%) |
| Percutaneous coronary intervention | 4 (1.4%) | 4 (0.7%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 13.1. n (%) = number and percentage of patients with at least one TEAE. Table sorted by SOC internationally agreed order and HLGT, HLT, PT by alphabetic order. Only SOC with at least one PT ≥ 1% in at least one group are presented. | | |

## Claims

1. A pharmaceutical combination for the use in the prevention of hypoglycaemia in diabetes mellitus type 2 comprising
(a) desPro³⁶Exendin-4(1-39)-Lys6-NH₂ or/and a pharmaceutically acceptable salt thereof, and
(b) a sulfonyl urea or/and a pharmaceutically acceptable salt thereof.

2. The pharmaceutical combination for the use of claim 1, wherein (i) desPro³⁶Exendin-4(1-39)-Lys6-NH₂ or/and a pharmaceutically acceptable salt thereof is to be administered subcutaneously, or/and (ii) the sulfonyl urea or/and the pharmaceutically acceptable salt thereof is to be administered orally.

3. The pharmaceutical combination for the use of any one of the claims 1 to 2, wherein desPro^{3®}Exendin-4(1-39)-Lys₆-NH₂ or/and a pharmaceutically acceptable salt is to be administered in an add-on therapy to administration of the sulfonyl urea.

4. The pharmaceutical combination for the use of any one of the preceding claims, wherein the diabetes mellitus type 2 is not adequately controlled with the sulfonyl urea alone.

5. The pharmaceutical combination for the use of any one of the claims 1 to 4, further comprising
(c) metformin or/and a pharmaceutically acceptable salt thereof, wherein the metformin or/and the pharmaceutically acceptable salt thereof is particularly to be administered orally.

6. The pharmaceutical combination for the use of claim 5, wherein desPro³⁶Exendin-4(1-39)-Lys6-NH₂ or/and a pharmaceutically acceptable salt is to be administered in an add-on therapy to the administration of metformin and the sulfonyl urea.

7. The pharmaceutical combination for the use of any one of the claims 5 to 6, wherein diabetes mellitus type 2 is not adequately controlled with the sulfonyl urea and metformin alone.

8. The pharmaceutical combination for the use of any one of the claims 1 to 7, wherein the sulfonyl urea is suitable for the treatment of diabetes type 2 and is particularly selected from Glibenclamide, Glibenclamide MR, Gliclazide, Gliclazide LM, Glimepiride, Glipizide, Glipizide XL, Gliquidone, and Tolbutamide.

9. The pharmaceutical combination for the use of any one of the preceding claims, wherein the subject to be treated is obese, and wherein the subject particularly has a body mass index of at least 30.

10. The pharmaceutical combination for the use of any one of the preceding claims, wherein the subject to be treated is an adult subject.

11. The pharmaceutical combination for the use of any one of the preceding claims, wherein the subject to be treated has a HbA1 c value in the range of 8% to 10%.

12. The pharmaceutical combination for the use of any one of the preceding claims, wherein the hypoglycaemia is associated with a plasma glucose concentration of below 60 mg/dL, below 50 mg/dL, below 40 mg/dL, or below 36 mg/dL.

13. The pharmaceutical combination for the use of any one of the preceding claims, wherein
(i) the hypoglycaemia is a symptomatic hypoglycaemia, which is particularly associated with at least one symptom selected from sweating, palpitations, hunger, restlessness, anxiety, fatigue, irritability, headache, loss of concentration, somnolence, psychiatric disorders, visual disorders, transient sensory defects, transient motor defects, confusion, convulsions, and coma, or/and
(ii) the hypoglycaemia is a severe symptomatic hypoglycaemia, which is particularly associated with a plasma glucose concentration below 36 mg/dL, and wherein the severe symptomatic hypoglycaemia is particularly associated with acute neurological impairment, which is particularly at least one selected from somnolence, psychiatric disorders, visual disorders, transient sensory defects, transient motor defects, confusion, convulsions, and coma.

14. A pharmaceutical combination comprising
(a) desPro36Exendin-4(1-39)-Lys₆-NH₂ or/and a pharmaceutically acceptable salt thereof, particularly for subcutaneous administration, and
(b) a sulfonyl urea or/and a pharmaceutically acceptable salt thereof, particularly for oral administration.

15. The pharmaceutical combination of claim 14, further comprising
(c) metformin or/and a pharmaceutically acceptable salt thereof, particularly for oral administration.

16. The pharmaceutical combination of any one of the claims 14 to 15, for the use in the glycemic control in diabetes mellitus type 2 patients, particularly for use in the reduction of post-prandial plasma glucose concentration or/and reduction of fasting plasma glucose concentration.

17. The pharmaceutical combination of any one of the claims 14 to 15, for the use in the induction of weight loss in diabetes mellitus type 2 patients or/and in the prevention of weight gain in diabetes mellitus type 2 patients.

## Patentansprüche

1. Pharmazeutische Kombination zur Verwendung bei der Prävention von Hypoglykämie bei Diabetes mellitus vom Typ 2, umfassend
(a) desPro³⁶Exendin-4(1-39)-Lys₆-NH₂ und/oder ein pharmazeutisch unbedenkliches Salz davon und
(b) einen Sulfonylharnstoff und/oder ein pharmazeutisch unbedenkliches Salz davon.

2. Pharmazeutische Kombination zur Verwendung nach Anspruch 1, wobei (i) desPro³⁶Exendin-4(1-39)-Lys₆-NH₂ und/oder ein pharmazeutisch unbedenkliches Salz davon subkutan zu verabreichen ist und/oder (ii) der Sulfonylharnstoff und/oder das pharmazeutisch unbedenkliche Salz davon oral zu verabreichen ist.

3. Pharmazeutische Kombination zur Verwendung nach Anspruch 1 oder 2, wobei desPro³⁶Exendin-4(1-39)-Lys₆-NH₂ und/oder ein pharmazeutisch unbedenkliches Salz davon als eine Zusatztherapie zur Verabreichung des Sulfonylharnstoffs zu verabreichen ist.

4. Pharmazeutische Kombination zur Verwendung nach einem der vorhergehenden Ansprüche, wobei der Diabetes mellitus vom Typ 2 durch den Sulfonylharnstoff alleine nicht angemessen behandelt wird.

5. Pharmazeutische Kombination zur Verwendung nach einem der Ansprüche 1 bis 4, weiterhin umfassend
(c) Metformin und/oder ein pharmazeutisch unbedenkliches Salz davon,
wobei das Metformin und/oder das pharmazeutisch unbedenkliche Salz davon insbesondere oral zu verabreichen ist.

6. Pharmazeutische Kombination zur Verwendung nach Anspruch 5, wobei desPro³⁶Exendin-4 (1-39)-Lys₆-NH₂ und/oder ein pharmazeutisch unbedenkliches Salz davon als eine Zusatztherapie zur Verabreichung von Metformin und dem Sulfonylharnstoff zu verabreichen ist.

7. Pharmazeutische Kombination zur Verwendung nach Anspruch 5 oder 6, wobei der Diabetes mellitus vom Typ 2 durch den Sulfonylharnstoff und Metformin alleine nicht angemessen behandelt wird.

8. Pharmazeutische Kombination zur Verwendung nach einem der Ansprüche 1 bis 7, wobei der Sulfonylharnstoff für die Behandlung von Diabetes vom Typ 2 geeignet und insbesondere aus Glibenclamid, Glibenclamid MR, Gliclazid, Gliclazid LM, Glimepirid, Glipizid, Glipizid XL, Gliquidon und Tolbutamid ausgewählt ist.

9. Pharmazeutische Kombination zur Verwendung nach einem der vorhergehenden Ansprüche, wobei der zu behandelnde Patient adipös ist und wobei der Patient insbesondere einen Körpermassenindex von mindestens 30 aufweist.

10. Pharmazeutische Kombination zur Verwendung nach einem der vorhergehenden Ansprüche, wobei es sich bei dem zu behandelnden Patienten um einen erwachsenen Patienten handelt.

11. Pharmazeutische Kombination zur Verwendung nach einem der vorhergehenden Ansprüche, wobei der zu behandelnde Patient einen HbA1c-Wert im Bereich von 8% bis 10% aufweist.

12. Pharmazeutische Kombination zur Verwendung nach einem der vorhergehenden Ansprüche, wobei die Hypoglykämie mit einer Plasmaglucosekonzentration von unter 60 mg/dl, unter 50 mg/dl, unter 40 mg/dl oder unter 36 mg/dl assoziiert ist.

13. Pharmazeutische Kombination zur Verwendung nach einem der vorhergehenden Ansprüche, wobei
(i) es sich bei der Hypoglykämie um eine symptomatische Hypoglykämie handelt, die insbesondere mit mindestens einem Symptom ausgewählt aus Schwitzen, Palpitationen, Hunger, Ruhelosigkeit, Angst, Müdigkeit, Reizbarkeit, Kopfschmerzen, Konzentrationsverlust, Schläfrigkeit, psychiatrischen Störungen, Sehstörungen, vorübergehenden sensorischen Defekten, vorübergehenden motorischen Defekten, Verwirrung, Krämpfen und Koma assoziiert ist, oder/und
(ii) es sich bei der Hypoglykämie um eine schwere symptomatische Hypoglykämie handelt, die insbesondere mit einer Plasmaglucosekonzentration unter 36 mg/dl assoziiert ist, und wobei die schwere symptomatische Hypoglykämie insbesondere mit einer akuten neurologischen Störung assoziiert ist, bei der es sich insbesondere um mindestens eine ausgewählt aus Schläfrigkeit, psychiatrischen Störungen, Sehstörungen, vorrübergehenden sensorischen Defekten, vorrübergehenden motorischen Defekten, Verwirrung, Krämpfen und Koma handelt.

14. Pharmazeutische Kombination, umfassend
(a) desPro³⁶Exendin-4(1-39)-Lys₆-NH₂ und/oder ein pharmazeutisch unbedenkliches Salz davon, insbesondere zur subkutanen Verabreichung, und
(b) einen Sulfonylharnstoff und/oder ein pharmazeutisch unbedenkliches Salz davon, insbesondere zur oralen Verabreichung.

15. Pharmazeutische Kombination nach Anspruch 14, weiterhin umfassend
(c) Metformin und/oder ein pharmazeutisch unbedenkliches Salz davon, insbesondere zur oralen Verabreichung.

16. Pharmazeutische Kombination nach Anspruch 14 oder 15 zur Verwendung bei der glykämischen Einstellung von Patienten mit Diabetes mellitus vom Typ 2, insbesondere zur Verwendung bei der Absenkung der postprandialen Plasmaglucosekonzentration und/oder der Absenkung der nüchtern-Plasmaglucosekonzentration.

17. Pharmazeutische Kombination nach Anspruch 14 oder 15 zur Verwendung bei der Induktion einer Gewichtsabnahme bei Patienten mit Diabetes mellitus vom Typ 2 und/oder bei der Prävention einer Gewichtszunahme bei Patienten mit Diabetes mellitus vom Typ 2.

## Revendications

1. Association pharmaceutique destinée à être utilisée dans la prévention de l'hypoglycémie dans les cas de diabète sucré de type 2 comprenant
(a) de la desPro³⁶Exendine-4(1-39)-Lys₆-NH₂ et/ou un sel pharmaceutiquement acceptable de celle-ci et
(b) une sulfonylurée et/ou un sel pharmaceutiquement acceptable de celle-ci.

2. Association pharmaceutique destinée à être utilisée selon la revendication 1, dans laquelle
(i) de la desPro³⁶Exendine-4(1-39)-Lys₆-NH₂ et/ou un sel pharmaceutiquement acceptable de celle-ci sont à administrer par voie sous-cutanée et/ou
(ii) la sulfonylurée et/ou le sel pharmaceutiquement acceptable de celle-ci sont à administrer par voie orale.

3. Association pharmaceutique destinée à être utilisée selon l'une quelconque des revendications 1 à 2, dans laquelle de la desPro³⁶Exendine-4(1-39)-Lys₆-NH₂ et/ou un sel pharmaceutiquement acceptable sont à administrer dans une thérapie d'appoint à l'administration de la sulfonylurée.

4. Association pharmaceutique destinée à être utilisée selon l'une quelconque des revendications précédentes, le diabète sucré de type 2 n'étant pas géré de façon adéquate avec la sulfonylurée seule.

5. Association pharmaceutique destinée à être utilisée selon l'une quelconque des revendications 1 à 4, comprenant en outre
(c) de la metformine et/ou un sel pharmaceutiquement acceptable de celle-ci, la metformine et/ou le sel pharmaceutiquement acceptable de celle-ci étant en particulier à administrer par voie orale.

6. Association pharmaceutique destinée à être utilisée selon la revendication 5, dans laquelle de la desPro³⁶Exendine-4 (1-39)-Lys₆-NH₂ et/ou un sel pharmaceutiquement acceptable de celle-ci sont à administrer dans une thérapie d'appoint à l'administration de metformine et de la sulfonylurée.

7. Association pharmaceutique destinée à être utilisée selon l'une quelconque des revendications 5 à 6, le diabète sucré de type 2 n'étant pas géré de façon adéquate avec la sulfonylurée et la metformine seules.

8. Association pharmaceutique destinée à être utilisée selon l'une quelconque des revendications 1 à 7, dans laquelle la sulfonylurée est appropriée pour le traitement du diabète de type 2 et est en particulier choisie parmi le glibenclamide, le glibenclamide MR, le gliclazide, le gliclazide LM, le glimépiride, le glipizide, le glipizide XL, la gliquidone et le tolbutamide.

9. Association pharmaceutique destinée à être utilisée selon l'une quelconque des revendications précédentes, le sujet à traiter étant obèse et le sujet en particulier ayant un indice de masse corporelle d'au moins 30.

10. Association pharmaceutique destinée à être utilisée selon l'une quelconque des revendications précédentes, le sujet à traiter étant un sujet adulte.

11. Association pharmaceutique destinée à être utilisée selon l'une quelconque des revendications précédentes, le sujet à traiter ayant une valeur d'HbA1c dans la plage de 8 % à 10 %.

12. Association pharmaceutique destinée à être utilisée selon l'une quelconque des revendications précédentes, l'hypoglycémie étant associée à une concentration plasmatique de glucose au-dessous de 60 mg/dl, au-dessous de 50 mg/dl, au-dessous de 40 mg/dl ou au-dessous de 36 mg/dl.

13. Association pharmaceutique destinée à être utilisée selon l'une quelconque des revendications précédentes,
(i) l'hypoglycémie étant une hypoglycémie symptomatique, qui est en particulier associée à au moins un symptôme choisi parmi la sudation, les palpitations, la faim, l'agitation, l'anxiété, la fatigue, l'irritabilité, la céphalée, la perte de concentration, la somnolence, les troubles psychiatriques, les troubles visuels, les anomalies sensorielles transitoires, les anomalies motrices transitoires, la confusion, les convulsions et le coma et/ou
(ii) l'hypoglycémie étant une hypoglycémie symptomatique sévère, qui est en particulier associée à une concentration plasmatique de glucose au-dessous de 36 mg/dl, et l'hypoglycémie symptomatique sévère étant en particulier associée à une déficience neurologique aiguë, qui en est en particulier au moins une choisie parmi la somnolence, les troubles psychiatriques, les troubles visuels, les anomalies sensorielles transitoires, les anomalies motrices transitoires, la confusion, les convulsions et le coma.

14. Association pharmaceutique comprenant
(a) de la desPro³⁶Exendine-4(1-39)-Lys₆-NH₂ et/ou un sel pharmaceutiquement acceptable de celle-ci, en particulier pour administration par voie sous-cutanée, et
(b) une sulfonylurée et/ou un sel pharmaceutiquement acceptable de celle-ci, en particulier pour administration par voie orale.

15. Association pharmaceutique selon la revendication 14, comprenant en outre
(c) de la metformine et/ou un sel pharmaceutiquement acceptable de celle-ci, en particulier pour administration par voie orale.

16. Association pharmaceutique selon l'une quelconque des revendications 14 à 15, destinée à être utilisée dans la régulation glycémique chez des patients atteints de diabète sucré de type 2, en particulier destinée à être utilisée dans la réduction de la concentration plasmatique postprandiale de glucose et/ou la réduction de la concentration plasmatique de glucose à jeun.

17. Association pharmaceutique selon l'une quelconque des revendications 14 à 15, destinée à être utilisée dans l'induction de la perte de poids chez des patients atteints de diabète sucré de type 2 et/ou dans la prévention de la prise de poids chez des patients atteints de diabète sucré de type 2.


### SEQUENCE LISTING

<110> Sanofi-Aventis Deutschland GmbH
<120> Prevention of hypoglycemia in diabetes mellitus type 2 patients
<130> 50539P WO
<150> EP 11160270.2
   <151> 2011-03-29
<160> 2
<170> PatentIn version 3.5
<210> 1
   <211> 44
   <212> PRT
   <213> Artificial
<220>
   <223> desPro36-Exendin-4(1-39)-Lys6-NH2
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 39
   <212> PRT
   <213> Heloderma suspectum
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35